# EUROPEAN PATENT SPECIFICATION

(11) **EP 0 466 408 B1**
(45) Date of publication and mention of the grant of the patent: **12.01.2000**
(21) Application number: 91306113.1
(22) Date of filing: 04.07.1991
(51) Int. Cl.: C07D 231/06, A01N 43/56, C07D 213/46, C07D 307/58, C07D 275/02, C07D 277/34, C07C 49/225

(54) **N-aryl-3-aryl-4-substituted-4,5-dihydro-1H-pyrazole-1-carboxamides and processes for their production**
N-Aryl-3-aryl-4-substituierte-4,5-dihydro-1H-pyrazol-1-carboxamide und Verfahren zu deren Herstellung
Carboxamides de N-aryl-3-aryl-4,5-dihydro-1H-pyrazoles-1 substitués en position 4 et leurs procédés de préparation

(30) Priority: 13.07.1990 US 553220; 17.06.1991 US 713692
(43) Date of publication of application: 15.01.1992
(73) Proprietor: ROHM AND HAAS COMPANY, Philadelphia Pennsylvania 19105 (US)
(72) Inventor: Jacobson, Richard Martin, Chalfont, Pennsylvania (US)
(74) Representative: Buckley, Guy Julian

(56) References cited:
- EP-A- 0 113 213
- DE-A- 3 545 786
- US-A- 4 464 386
- CHEMICAL ABSTRACTS, vol. 108, no. 9, February 29, 1988 Columbus, Ohio, USA MITSUI TOATSU CHEMICALS "Preparation of pyrazoline carboxamides as insecticides" page 682, column 1, abstract no. 75 392m & JP-A-62 056476

## Description

This invention relates to novel N-aryl-3-aryl-4-substituted-4,5-dihydro-1H-pyrazole-1-carboxamides which are useful as pesticides, compositions containing those compounds, methods of controlling pests and processes for preparing the compounds of the present invention.

The search for pesticides which have a combination of excellent pesticidal activity and essentially no other toxicity is a continuing one due to recognition of the possible toxicity to animals and humans of many known pesticides.

Presently known dihydropyrazole insecticides, such as those disclosed in US-A-4,863,947, 4,070,365, 4,174,393, 4,439,440, 4,407,813, 4,464,386 and 4,156,007, are believed to be subject to problems with photostability and/or biodegradability. These compounds tend to degrade faster than is desirable when applied to the external parts of plants due to the action of sunlight on these compounds. Moreover, when known compounds are applied to the soil, they exhibit poor biodegradability causing an undesirable residue to remain in the soil. Although US-A-4,464,386, EP-A2/A3 - 0 113 213, DE-A1 - 3,545,786 and Chemical Abstracts, vol. 108, no. 9, February 29, 1998 Abstract No. 75392m (Jpn. Kokai Tokkyo Koho JP 62 56,476) disclose dihydropyrazole compounds, none of these compounds possess the substituent pattern at the 4-position of the dihydropyrazole ring which provides the compounds of the present invention with their attendant advantages.

This invention concerns particularly N-aryl-3-aryl-4-substituted-4,5-dihydro-1H-pyrazole-1-carboxamides substituted at the 4 position by a substituent which is attached to the pyrazoline ring by a heteroatom.

It is believed that this substitution may sufficiently alter metabolic pathway transformations in plants and insects to provide the necessary differentiation which allows for high insect toxicity and low mammalian toxicity. It is further believed to permit appropriate biodegradation.

In accordance with the present invention, there is provided a compound of the formula 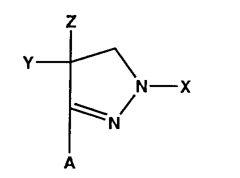 wherein
X is 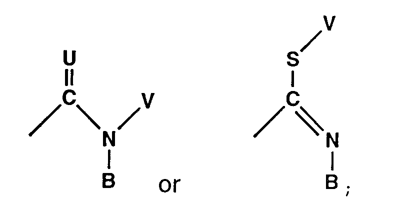
A and B are ; pyridyl, furyl, thiazolyl or naphthyl, each of which is optionally substituted by one or two independently chosen substituents selected from nitro, (C₁-C₆)alkyl, halo(C₁-C₆)alkyl and halo;
or phenyl optionally substituted by one to three substituents independently selected from (C₁-C₆)alkyl; halo(C₁-C₆)alkyl; halo;(C₁-C₆)alkoxy; halo(C₁-C₆)alkoxy; (C₃-C₆)alkenyloxy; (C₃-C₆ )alkynyloxy; (C₁-C₆)alkoxy(C₁-C₆)alkoxy; phenyl(C₁-C₆)alkoxy; phenyloxy; pyridyloxy; mono(C₁-C₆)alkylaminocarbonyloxy; di(C₁-C₆)alkylaminocarbonyloxy; (C₁-C₆)alkanoyloxy; (C₁-C₆)alkoxycarbonyloxy; (C₁-C₆)alkylsulfonyloxy; (C₁-C₆)alkylthio; halo(C₁-C₆)alkylthio; (C₁-C₆)alkoxy(C₁-C₆)alkyl; (C₁-C₆)alkanoyl; (C₁-C₆)alkoxycarbonyl; nitro; (C₁-C₆)alkylsulfonyl; halo(C₁-C₆)alkylsulfonyl; phenyl; hydroxy; cyano; *iso*cyano; amino; mono(C₁-C₆)alkylamino; di(C₁-C₆)alkylamino; formylamino; (C₁-C₆)alkanoylamino; halo(C₁-C₆)alkanoylamino; phenylcarbonylamino; mono(C₁-C₆)alkylaminocarbonylamino; and di(C₁-C₆)alkylaminocarbonylamino;
Y is -NR¹R²;
wherein R¹ and R² are each independently hydrogen, cyano, (C₁-C₆)alkyl, halo(C₁-C₆)alkyl, cyano(C₁-C₆)alkyl, phenyl(C₁-C₆)alkyl, (C₃-C₆)alkenyl, halo(C₃-C₆)alkenyl, (C₃-C₆)alkynyl, phenyl, halophenyl, formyl, (C₁-C₆)alkylcarbonyl, halo(C₁-C₆)alkylcarbonyl, (C₂-C₆)alkenylcarbonyl, halo(C₂-C₆)alkenylcarbonyl, (C₁-C₆)alkoxy(C₁-C₆)alkylcarbonyl, phenylcarbonyl, phenyl(C₂-C₆)alkenylcarbonyl, carboxy, (C₁-C₆)alkoxycarbonyl, halo(C₁-C₆)alkoxycarbonyl, cyano(C₁-C₆)alkoxycarbonyl, (C₂-C₆)alkenyloxycarbonyl, (C₃-C₆)alkynyloxycarbonyl, (C₁-C₆)alkanoyl(C₁-C₆)alkoxycarbonyl, (C₁-C₆)alkoxycarbonyl(C₁-C₆)alkoxycarbonyl, carboxy(C₁-C₆)alkoxycarbonyl, phenyloxycarbonyl, phenyl(C₁-C₆)alkoxycarbonyl, ((C₁-C₆)alkylthio)carbonyl, N-(C₁-C₆)alkylaminocarbonyl, N,N-di(C₁-C₆)alkylaminocarbonyl, N-phenyl-N-(C₁-C₆)alkylaminocarbonyl, N-(phenylcarbonyl)aminocarbonyl, di(C₁-C₆)alkylphosphoryl, (C₁-C₆)alkylsulfonyl, (C₂-C₆)alkenylsulfonyl, N,N-di(C₁-C₆)alkylaminosulfonyl, phenylsulfonyl, pyridyl or pyrazinyl; or
R¹ and R² together with the nitrogen to which they are attached form a 5- or 6-membered ring selected from 2-oxazolidonyl, pyrrolidinonyl, piperidonyl and succinimidyl;
Z is hydrogen;
U is oxygen or sulfur; and
V is hydrogen, (C₁-C₆)alkyl, (C₁-C₆)alkoxy(C₁-C₆)alkyl, formyl, (C₁-C₆)alkylcarbonyl, (C₁-C₆)alkylaminocarbonyl, (C₁-C₆)alkoxycarbonyl, (C₃-C₆)alkenyloxycarbonyl, phenyloxycarbonyl, (C₁-C₆)alkoxycarbonylcarbonyl, cyano(C₁-C₆)alkylthio, (C₁-C₆)alkylthio, phenylthio, (C₁-C₆)alkoxycarbonyl(C₁-C₆)alkylthio or (C₁-C₆)alkoxycarbonylthio, with the proviso that V is not hydrogen if attached to S; and
agronomically acceptable salts thereof.

For the avoidance of doubt, all Y substituents are attached to the pyrazoline ring by the heteroatom present in the substituent.

Alkyl means straight and branched alkyl groups, for example (C₁-C₆)alkyl such as methyl, ethyl, *n*-propyl, *iso*propyl, *n*-butyl, *iso*butyl, *s*-butyl, *t*-butyl or *n*-pentyl. An alkyl portion of any one of the substituents listed above for V, Y and Z or of the substituents on the aryl rings listed below is optionally substituted by one to eight halogens to form groups such as trifluoromethyl, bromodifluoromethyl, 1,1,2,2,2-pentafluoroethyl, 1,1,2,2-tetrafluoroethyl, chloromethyl, dichloromethyl, trichloromethyl, difluoromethyl, 2-bromoethyl, 2-chloroethyl, 3-bromopropyl, 2-chloro-1,1,2-trifluoroethyl, 2-bromo-1,1,2,2-tetrafluoroethyl, or 1,1,2,3,3,3-hexafluoropropyl; or optionally substituted by cyano to form groups such as 3-cyanopropyl.

Alkenyl is, for example, (C₂-C₆)alkenyl such as vinyl and allyl.

Alkynyl is, for example, (C₃-C₆)alkynyl such as propargyl.

Alkoxyalkyl is, for example (C₁-C₆)alkoxy(C₁-C₆)alkyl such as methoxymethyl and methoxyethyl.

Alkylthioalkyl is, for example, (C₁-C₆)alkylthio(C₁-C₆)alkyl.

Phenylalkyl is, for example, phenyl(C₁-C₆)alkyl such as benzyl and 2-phenylethyl.

Alkylcarbonyl is, for example, (C₁-C₆)alkylcarbonyl such as methylcarbonyl (acetyl), ethylcarbonyl, *n*-propylcarbonyl, *iso*propylcarbonyl, *n*-butylcarbonyl, *iso*butylcarbonyl, *t*-butylcarbonyl, *n*-pentylcarbonyl, chloromethylcarbonyl, trichloromethylcarbonyl, trifluoromethylcarbonyl, 3-chloropropylcarbonyl, 4-chlorobutylcarbonyl, pentafluoroethylcarbonyl and heptafluoropropylcarbonyl.

Alkenylcarbonyl is, for example, (C₂-C₆)alkenylcarbonyl such as vinylcarbonyl, 1-methylvinylcarbonyl, 2-methylvinylcarbonyl, 2,2-dimethylvinylcarbonyl and 1,2,2-trichlorovinylcarbonyl.

Alkynylcarbonyl is, for example, (C₂-C₆)alkynylcarbonyl.

Alkoxyalkylcarbonyl is, for example, (C₁-C₆)alkoxy(C₁-C₆)alkylcarbonyl such as methoxymethylcarbonyl.

Phenylalkylcarbonyl is, for example, phenyl(C₁-C₆)alkylcarbonyl.

Phenylalkenylcarbonyl is, for example, phenyl(C₂-C₆)alkenylcarbonyl such as phenylvinylcarbonyl (cinnamoyl).

Phenylalkynylcarbonyl is, for example, phenyl(C₂-C₆)alkynyl.

Alkylaminocarbonyl is, for example, mono(C₁-C₆)alklyaminocarbonyl, such as methylaminocarbonyl, or di(C₁-C₆)alkylaminocarbonyl such as dimethylaminocarbonyl.

Alkoxycarbonyl is for example, (C₁-C₆)alkoxycarbonyl such as methoxycarbonyl (carbomethoxy), ethoxycarbonyl (carboethoxy), *n*-propyloxycarbonyl, *iso*propyloxycarbonyl, *n*-butyloxycarbonyl, *iso*butyloxycarbonyl, *t*-butyloxycarbonyl, *n*-pentyloxycarbonyl, cyanomethoxycarbonyl, 2-cyanoethoxycarbonyl, 2-bromoethoxycarbonyl, 2-chloroethoxycarbonyl, 2,2,2-trifluoroethoxycarbonyl, 2,2,2-trichloroethoxycarbonyl, 3-bromopropyloxycarbonyl, 3-chloropropyloxycarbonyl and 4-chlorobutyloxycarbonyl.

Alkenyloxycarbonyl is, for example, (C₂-C₆)alkenyloxycarbonyl such as vinyloxycarbonyl and allyloxycarbonyl.

Alkynyloxycarbonyl is, for example, (C₃-C₆)alkynyloxycarbonyl such as propargyloxycarbonyl.

Alkoxyalkoxycarbonyl is, for example,
(C₁-C₆)alkoxy(C₁-C₆)alkoxycarbonyl such as methoxyethoxycarbonyl.

Alkanoylalkoxycarbonyl is, for example,
(C₁-C₆)alkanoyl(C₁-C₆)alkoxycarbonyl such as methylcarbonylmethoxycarbonyl.

Alkoxycarbonylalkoxycarbonyl is, for example,
(C₁-C₆)alkoxycarbonyl, (C₁-C₆)alkoxycarbonyl such as ethoxycarbonylmethoxycarbonyl and ethoxycarbonylethoxycarbonyl.

Carboxyalkoxycarbonyl is, for example,
carboxy(C₁-C₆)alkoxycarbonyl such as carboxyethoxycarbonyl and carboxypropoxycarbonyl.

Phenylalkoxycarbonyl is, for example,
phenyl(C₁-C₆)alkoxycarbonyl such as benzyloxycarbonyl and 2-phenylethoxycarbonyl.

(Alkylthio)carbonyl is, for example, ((C₁-C₆)alkylthio)carbonyl such as (methylthio)carbonyl, (ethylthio)carbonyl, (*n*-propylthio)carbonyl, and (*n*-butylthio)carbonyl.

(Alkenylthio)carbonyl is, for example, ((C₃-C₆)alkylthio)carbonyl.

(Alkynylthio)carbonyl is, for example,
((C₃-C₆)alkynylthio)carbonyl.

Alkylcarbonyl(alkylthio)carbonyl is, for example,
(C₁-C₆)alkyl carbonyl(C₁-C₆)alkylthio)carbonyl.

Alkoxycarbonyl(alkylthio)carbonyl is, for example,
(C₁-C₆)alkoxycarbonyl(C₁-C₆)alkylthio)carbonyl.

(Alkylthio)carbonylalkoxycarbonyl is, for example,
((C₁-C₆)alkylthio)carbonyl(C₁-C₆)alkoxycarbonyl.

(Alkylthio)carbonyl(alkylthio)carbonyl is, for example,
((C₁-C₆)alkylthio)carbonyl((C₁-C₆)alkylthio)carbonyl.

Carboxy(alkylthio)carbonyl is, for example,
carboxy((C₁-C₆)-alkylthio)carbonyl.

(Phenylalkylthio)carbonyl is, for example,
(phenyl(C₁-C₆)alkylthio)carbonyl.

N-alkylaminocarbonyl is, for example,
N-(C₁-C₆)alkylaminocarbonyl such as methylaminocarbonyl.

N,N-dialkylaminocarbonyl is, for example,
N,N-di(C₁-C₆)alkylaminocarbonyl such as dimethylaminocarbonyl.

N-phenyl-N-alkylaminocarbonyl is, for example,
N-phenyl-N-(C₁-C₆)alkylaminocarbonyl such as N-methyl-N-(phenyl)aminocarbonyl.

N-(phenylcarbonyl)aminocarbonyl is, for example,
N-(2,6-difluorophenylcarbonyl)aminocarbonyl.

Dialkylphosphoryl is, for example,
di(C₁-C₆)alkylphosphoryl such as diethylphosphoryl.

Dialkylthiophosphoryl is, for example,
di(C₁-C₆)alkylthiophosphoryl such as diethylthiophosphoryl.

Alkylsulfonyl is, for example, (C₁-C₆)alkylsulfonyl such as methylsulfonyl, *n*-butylsulfonyl, chloromethylsulfonyl,
trifluoromethylsulfonyl and 2,2,2-trifluoroethylsulfonyl.

Alkenylsulfonyl is, for example, (C₂-C₆)alkenylsulfonyl such as vinylsulfonyl.

Alkynylsulfonyl is, for example, (C₃-C₆)alkynylsulfonyl.

N,N-dialkylaminosulfonyl is, for example,
N,N-di(C₁-C₆)alkylaminosulfonyl such as dimethylaminosulfonyl.

Alkoxy is, for example, (C₁-C₆)alkoxy such as methoxy, ethoxy, *n*-propyloxy, *n*-butyloxy, *iso*butyloxy, *n*-pentyloxy, trifluoromethoxy, difluoromethoxy, 1,1,2,2-tetrafluoroethoxy and
1,1,2,3,3,3-hexafluoropropyloxy.

Alkylthio is, for example, (C₁-C₆)alkylthio such as methylthio, *n*-propylthio, *n*-butylthio and 3-cyanopropylthio.

Alkoxycarbonylthio is, for example (C₁-C₆)alkoxycarbonylthio such as methoxycarbonylthio.

Phenylthio includes, for example, phenylthio and 2-nitrophenylthio.

Alkoxycarbonylalkylthio is, for example,
(C₁-C₆)alkoxycarbonyl(C₁-C₆)alkylthio such as 1-(methoxycarbonyl)prop-2-ylthio.

Heterocyclyl means five or six membered heterocyclic ring containing one, two or three heteroatoms such as oxygen, nitrogen or sulfur and includes saturated and aromatic rings, for example tetrahydrofuryl, furyl, pyridyl, pyrazinyl, oxazolyl, piperidyl, triazolyl, thienyl, thiazolyl or piperazyl. The heterocyclyl ring is optionally substituted by one or two independently choses substituents, for example, nitro, (C₁-C₆)alkyl such as methyl and trifluoromethyl and halo such as chloro.

Aryl is an aromatic carbocyclic structure, for example, phenyl or naphthyl.

Naphthyl is optionally substituted by one or two independently chosen substituents, for example, nitro, (C₁-C₆)alkyl such as methyl and trifluoromethyl and halo such as chloro.

Phenyl is optionally substituted by one to three independently chosen substituents, for example, (C₁-C₆)alkyl, halo, hydroxy, (C₁-C₆)alkoxy, (C₃-C₆)alkenyloxy, (C₃-C₆)alkynyloxy, (C₁-C₆)alkoxy(C₁-C₆)alkoxy, phenyl(C₁-C₆)alkoxy, phenyloxy, pyridyloxy, mono(C₁-C₆)alkylaminocarbonyloxy, di(C₁-C₆)alkylaminocarbonyloxy, (C₁-C₆)alkanoyloxy, (C₁-C₆)alkoxycarbonyloxy, (C₁-C₆)alkylsulfonyloxy, (C₁-C₆)alkylthio, (C₁-C₆)alkoxy(C₁-C₆)alkyl, (C₁-C₆)alkanoyl, (C₁-C₆)alkoxycarbonyl, nitro, (C₁-C₆)alkylsulfonyl, phenyl, cyano, *iso*cyano (-NC), amino, mono(C₁-C₆)alkylamino, di(C₁-C₆)alkylamino, formylamino (-NHCHO), (C₁-C₆)alkanoylamino, phenylcarbonylamino, mono(C₁-C₆)-alkylaminocarbonylamino, and di(C₁-C₆)alkylaminocarbonylamino, such as 4-methyl, 4-ethyl, 4-propyl, 4-*t*-butyl, 4-trifluoromethyl, 4-dichloromethyl, 4-trichloromethyl, 4-fluoro, 4-bromo, 4-chloro, 4-iodo, 4-hydroxy, 4-methoxy, 4-ethoxy, 4-*n*-propyloxy, 4-*iso*propyloxy, 4-*sec*-butyloxy, 4-*n*-butyloxy, 4-*iso*butyloxy, 4-*n*-pentyloxy, 4-difluoromethoxy, 4-trifluoromethoxy, 4-(1,1,2,2-tetrafluoroethoxy), 4-bromodifluoromethoxy, 4-(1,1,2,3,3,3-hexafluoropropyloxy), 4-allyloxy, 4-propargyloxy, 4-methoxymethoxy, 4-benzyloxy, 4-(2-phenylethoxy), 4-phenyloxy, 4-(2-chloro-4-trifluoromethylphenyloxy), 4-(5-chloro-2-pyridyloxy), 4-(5-(trifluoromethyl)-2-pyridyloxy), 4-(3-chloro-5-(trifluoromethyl)-2-pyridyloxy), 4-methylaminocarbonyloxy, 4-(N,N-dimethylaminocarbonyloxy), 4-acetoxy, 4-methoxycarbonyloxy, 4-methylsulfonyloxy, 4-trifluoromethylsulfonyloxy, 4-methylthio, 4-(1,1,2,2,tetrafluoroethylthio), 4-(2-ethoxyethyl), 4-acetyl, (i.e., methylcarbonyl), 4-ethylcarbonyl, 4-*iso*propylcarbonyl, 4-methoxycarbonyl, 4-ethoxycarbonyl, 4-*iso*propyloxycarbonyl, 4-nitro, 4-methylsulfonyl, 4-(1,1,2,2-tetrafluoroethylsulfonyl), 4-phenyl, 4-cyano, 4-*iso*cyano, 4-amino, 4-methylamino, 4-dimethylamino, 4-formylamino, 4-acetamido, 4-trifluoroacetamido, 4-phenylcarbonylamino, 4-(4-chlorophenylcarbonylamino), 4-methylaminocarbonylamino, and 4-(di-*n*-propylaminocarbonylamino).

Halo means fluoro, chloro, bromo and iodo.

Agronomically acceptable salts include those known in the art, for example, metal salts such as sodium, potassium, calcium and magnesium; ammonium salts such as *iso*propylammonium; and trialkylsulfonium salts such as trimethylsulfonium.

Preferably, when X is 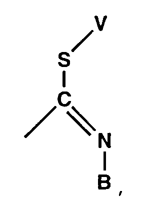 A is 4-chlorophenyl or 4-*n*-propyloxyphenyl; B is 4-trifluoromethylphenyl; Y is N-methyl-N-methoxycarbonylamino or N-propyl-N-formylamino; Z is hydrogen; and V is methyl, ethyl, *n*-propyl, *iso*propyl, propenyl, methoxyethyl or benzyl.

Preferably, when X is 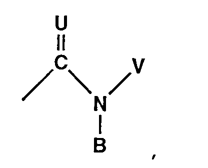 and U is sulfur, A is 4-chlorophenyl or 4-n-propyloxyphenyl, B is 4-chlorophenyl or 4-trifluoromethylphenyl, Y is N-methyl-N-methoxycarbonylamino, V is hydrogen and Z is hydrogen.

In another aspect of the invention, there are provided insecticidal compositions containing compounds of the present invention, and in a further aspect there are provided processes for preparing 1-substituted-4-substituted-4,5-dihydro-1H-pyrazoles of the present invention.

Further, in accordance with the invention there are provided intermediates useful in making the compounds of the invention, having the structure 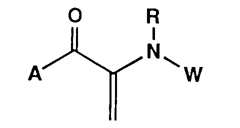 wherein A is as defined above, R is hydrogen or (C₁-C₆)alkyl and W is (C₁-C₆)alkylcarbonyl, halo(C₁-C₆)alkylcarbonyl, (C₁-C₆)alkoxycarbonyl or (C₁-C₆)alkylsulfonyl.

These intermediate compounds also have fungicidal and biocidal activity.

In one class of the preferred embodiment of the invention are compounds of Formula I wherein X is 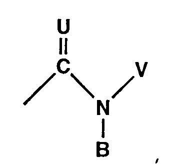 Y is -NR¹R²; V is hydrogen and the remaining substituents are as defined above.

In an embodiment of this class are compounds of the structure 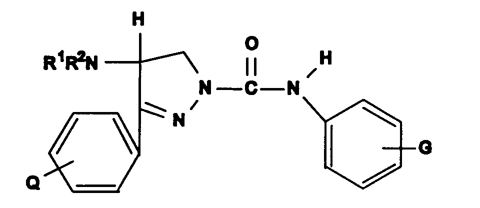 wherein
Q is hydrogen, halo, (C₁-C₆)alkyl, (C₁-C₆)alkoxy(C₁-C₆)alkyl, halo(C₁-C₆)alkyloxy or (C₁-C₆)alkoxy;
G is halo, cyano, halo(C₁-C₆)alkyl, (C₁-C₆)alkoxycarbonyl or halo(C₁-C₆)alkoxy;
R¹ is (C₁-C₆)alkyl, (C₃-C₆)alkenyl, (C₃-C₆)alkynyl, (C₁-C₆)alkoxy(C₁-C₆)alkyl, cyano(C₁-C₆)alkyl, (C₁-C₆)alkoxycarbonyl(C₁-C₆)alkyl, phenyl(C₁-C₆)alkyl, phenyl or halophenyl;
R² is cyano, (C₁-C₆)alkyl, (C₁-C₆)alkylsulfonyl, (C₁-C₆)alkoxycarbonyl, ((C₁-C₆)alkylthio)carbonyl, halo(C₁-C₆)alkoxycarbonyl, formyl, (C₁-C₆)alkylcarbonyl, halo(C₁-C₆)alkylcarbonyl, (C₁-C₆)alkoxy(C₁-C₆)alkylcarbonyl, (C₂-C₆)alkenylcarbonyl, halo(C₂-C₆)alkenylcarbonyl, di(C₁-C₆)alkylaminocarbonyl, phenylcarbonyl, di(C₁-C₆)alkylphosphoryl or di(C₁-C₆)alkylthiophosphoryl; or
R¹ and R² together with the nitrogen to which they are attached form pyrid-2-one-1-yl, pyrid-4-one-1-yl, triazolyl, 2-oxazolidonyl, *iso*morpholin-2-onyl, pyrrolidinonyl, piperidonyl or succinimidyl.

More preferred are compounds wherein
Q is 4-chloro, G is 4-trifluoromethyl, R¹ is methyl and R² is formyl, methylcarbonyl, ethylcarbonyl, dimethylaminocarbonyl, methoxycarbonyl, ethoxycarbonyl or *iso*propyloxycarbonyl;
Q is 4-chloro, G is 4-trifluoromethyl, R² is methoxycarbonyl and R¹ is ethyl, propyl or propargyl;
Q is 4-chloro, G is 4-trifluoromethoxy, R² is methoxycarbonyl and R¹ is methyl, ethyl, propyl, ally or propargyl;
Q is 4-chloro, G is 4-difluoromethoxy, R¹ is methyl and R² is methoxycarbonyl;
Q is 4-chloro, G is 4-(1,1,2,2-tetrafluoroethoxy), R² is methoxycarbonyl and R¹ is methyl, ethyl or propargyl;
Q is 4-chloro, G is 4-(1,1,2,3,3,3-hexafluoropropyloxy), R¹ is methyl and R² is methoxycarbonyl;
Q is 4-ethoxy, G is 4-trifluoromethoxy, R¹ is methyl and R² is methoxycarbonyl;
Q is 4-*n*-propyloxy, G is 4-trifluoromethoxy, R¹ is methyl and R² is methoxycarbonyl;
Q is 4-*n*-butyloxy, G is 4-trifluoromethoxy, R¹ is methyl and R² is methoxycarbonyl;
Q is 4-difluoromethoxy, R¹ is methyl, R² is methoxycarbonyl and G is 4-chloro, 4-trifluoromethyl, 4-difluoromethoxy, 4-trifluoromethoxy, 4-(1,1,2,2-tetrafluoroethoxy), 4-(1,1,2,3,3,3-hexafluoropropyloxy), or 4-*iso*propyloxycarbonyl; or
Q is hydrogen, R¹ is methyl, R² is methoxycarbonyl and G is 4-trifluoromethyl or 4-trifluoromethoxy.

In another embodiment of this class are compounds of the formula 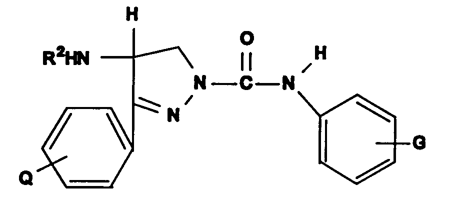 wherein
Q is hydrogen, halo, halo(C₁-C₆)alkoxy or (C₁-C₆)alkoxy;
G is halo, halo(C₁-C₆)alkyl or halo(C₁-C₆)alkoxy; and
R² is hydrogen, (C₁-C₆)alkyl, phenyl(C₁-C₆)alkyl, cyano, (C₁-C₆)alkoxycarbonyl, halo(C₁-C₆)alkoxycarbonyl, ((C₁-C₆)alkylthio)carbonyl, halo(C₁-C₆)alkoxycarbonyl, cyano(C₁-C₆)alkoxycarbonyl, (C₁-C₆)alkoxy(C₁-C₆)alkoxycarbonyl, (C₁-C₆)alkoxycarbonyl(C₁-C₆)alkoxycarbonyl, phenoxycarbonyl, phenyl(C₁-C₆)alkoxycarbonyl, (C₃-C₆)alkenyloxycarbonyl, (C₃-C₆)alkynyloxycarbonyl, formyl, (C₁-C₆)alkylcarbonyl, halo(C₁-C₆)alkylcarbonyl, carboxy(C₁-C₆)alkylcarbonyl, phenylcarbonyl, phenyl(C₁-C₆)alkylcarbonyl, phenyl(C₂-C₆)alkenylcarbonyl, mono(C₁-C₆)alkylaminocarbonyl, di(C₁-C₆)alkylaminocarbonyl, phenylaminocarbonyl, (C₁-C₆)alkoxycarbonyl(C₁-C₆)alkylaminocarbonyl, phenyl((C₁-C₆)alkyl)aminocarbonyl, di(C₁-C₆)alkylphosphoryl, di(C₁-C₆)alkylthiophosphoryl, (C₁-C₆)alkylsulfonyl, (C₂-C₆)alkenylsulfonyl, halo(C₁-C₆)alkylsulfonyl, phenylsulfonyl, di(C₁-C₆)alkylaminosulfonyl, 2-pyridyl or 2-pyrazinyl.

More preferred are compounds wherein Q is hydrogen, G is 4-trifluoromethyl and R² is methoxycarbonyl; Q is 4-chloro, G is 4-trifluoromethyl and R² is methoxycarbonyl; Q is 4-chloro, G is 4-trifluoromethoxy and R² is methoxycarbonyl; and Q is 4-chloro, 4-n-butyloxy, 4-n-propyloxy or 4-difluoromethoxy, G is 4-trifluoromethyl, 4-trifluoromethoxy, or 4-(1,1,2,2-tetrafluoroethoxy) and R² is n-propyl.

In another class of the preferred embodiment of the invention is the compound of Formula I wherein
V is (C₁-C₆)alkyl, (C₁-C₆)alkoxy(C₁-C₆)alkyl, formyl, (C₁-C₆)alkylcarbonyl, (C₁-C₆)alkylaminocarbonyl, (C₁-C₆)alkoxycarbonyl, (C₃-C₆)alkenyloxycarbonyl, phenyloxycarbonyl, (C₁C₆)alkoxycarbonylcarbonyl, cyano(C₁-C₆)alkylthio, (C₁-C₆)alkylthio, phenylthio, (C₁-C₆)alkoxycarbonyl(C₁-C₆)alkylthio or (C₁-C₆)alkoxycarbonylthio;
Y is -NR¹R²; and the remaining substituents are as defined above.

Preferred are compounds of the structure wherein 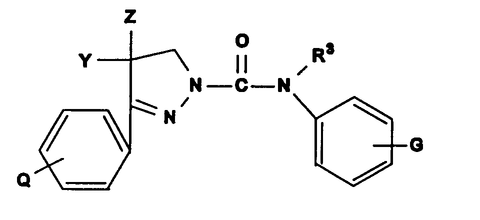
Q is halo;
G is halo or halo(C₁-C₆)alkyl;
Y is NR¹R²;
R¹ is hydrogen or (C₁-C₆)alkyl;
R² is (C₁-C₆)alkoxycarbonyl;
R³ is (C₁-C₆)alkyl; and
Z is hydrogen.

More preferred are compounds wherein Q is 4-halo, G is 4-halo or 4-halo(C₁-C₆)alkyl; R¹ is hydrogen or (C₁-C₆)alkyl R² is (C₁-C₆)alkoxycarbonyl; R³ is (C₁-C₆)alkyl and Z is hydrogen.

Most preferred are compounds wherein Q is 4-chloro, G is 4-chloro, R¹ is methyl, R² is methoxycarbonyl, R³ is methyl and Z is hydrogen; Q is 4-chloro, G is 4-trifluoromethyl, R¹ is hydrogen, R² is methoxycarbonyl, R³ is methyl and Z is hydrogen; and Q is 4-chloro, G is 4-trifluoromethyl, R¹ is methyl, R² is methoxycarbonyl, R³ is methyl and Z is hydrogen.

A process for preparing many of the compounds of the invention starts from compounds disclosed in U.S.-A-4,863,947, incorporated herein by reference, according to the following general synthesis shown in Scheme 1.

More particularly, in the case where Y is attached to the pyrazoline ring by a nitrogen, the starting pyrazoline (III)
(wherein R is alkyl and A, B, U, V and Z are as defined above) contains a carboxylic acid ester at the Y-position. The ester is saponified to yield the corresponding carboxylic acid (IV) under normal saponification conditions. Preferred solvents are protic solvents such as methanol or solvent mixtures such as methanol and tetrahydrofuran at temperatures between about 0°C and about 100°C, more preferably between about 25° and about 75°.

The acid is then converted to the acid chloride (V) by known means, for example, treatment with thionyl chloride. Preferred solvents are toluene and chloroform.

The acid chloride is reacted with azide anion, for example, sodium azide, to yield the azidocarbonyl compound (VI). Preferred solvents are acetonitrile and dimethylformamide.

The azidocarbonyl compound is then converted to the corresponding *iso*cyanate (VII) by heating in an appropriate solvent until gas evolution ceases. Preferred solvents are toluene, benzene, and chlorobenzene.

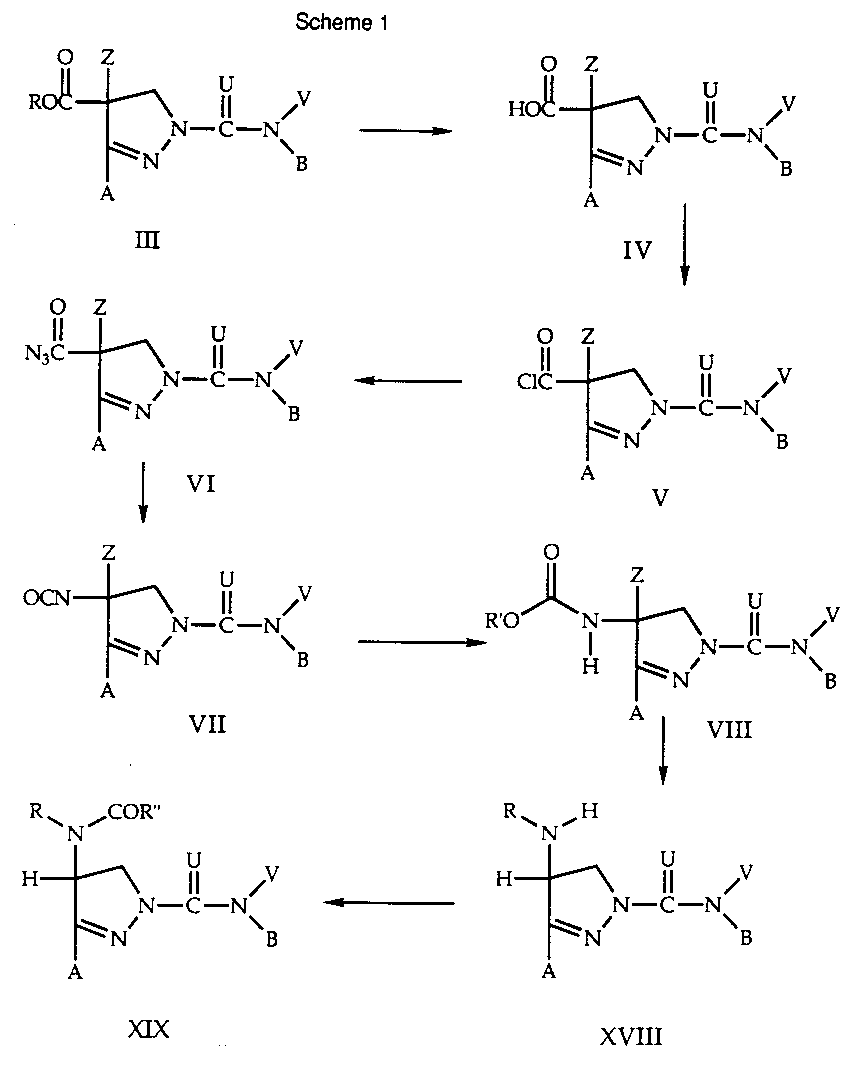

The carboalkoxyamino compounds of the invention (VIII) are obtained by reacting the *iso*cyanato compounds with the appropriate alcohol. The alcohol can be used as the solvent, for example methanol or ethanol, or alternatively a slight excess of the alcohol along with a base is used in a inert solvent. Preferred solvents are benzene and toluene.

In the case where the compound (VIII) is a *t*-butyloxycarbonylamino compound and the like, the carboalkoxyamino compound can then be decarbalkoxylated to yield the corresponding amino compound (XVII) by heating in an inert solvent in the presence of acid. Preferred solvents include halogenated solvents such as chloroform and methylene chloride. Preferred acids include, for example, trifluoroacetic acid and toluene sulfonic acid.

The corresponding carboxamide compounds (XIX), and the like, are prepared from the amino compound by treatment with the appropriate acid chloride in the presence of base. Preferred solvents are methylene chloride and tetrahydrofuran. Prefered bases include pyridine and triethylamine.

Alternatively, the amino-substituted compounds are prepared using the synthesis shown in Scheme 2. chloride.

The alkylaminomethyl keton is acylated with an acylating agent, for example, with an alkyl chloroformate such as methyl chloroformate, ethyl chloroformate, propyl chloroformate, or *iso*propyl chloroformate or an alkanoyl chloride or anhydride such as propionyl chloride, propionic anhydride, acetic anhydride, acetyl chloride, butyryl chloride, *iso*butyryl chloride, trifluoracetic anhydride, methane sulfonyl chloride, valeryl chloride, 2-methylbutyryl chloride, 3-methylbutyryl chloride or hexanoyl chloride, to produce the substituted compound (XIV) where W is acyl. Preferred solvents are aprotic solvents such as methylene chloride at a temperature between about -25°C and about 50°C, more preferably between about 0°C and about 20°C.

The acyl compound is then treated with formaldehyde to obtain the corresponding prop-2-enone (XV). Preferred solvents are protic solvents such as propanol or 2-methoxyethanol at temperatures between about 0°C and about 140°C, preferably at about the reflux temperature of the solvent used. Preferably catalytic amounts of a base such as piperidine and an acid such as acetic acid are also present in the reaction mixture.

The resulting prop-2-enone is then converted to the corresponding dihydropyrazole (XVI) by treatment with hydrazine. Preferred solvents are protic solvents such as methanol at temperatures between about 0 °C and about 100 °C, preferably between about 25 °C and about 70 °C. The resulting dihydropyrazole is generally reacted with an appropriate isocyanate as described in US-A-4,863,947 to obtain the corresponding carboxamide (XVII).

When W is carboalkoxy, the carboxamide (XVII) can be decarboxylated by known means to obtain the disubstituted amino compound (XVIII). Preferred, when R' is 2,2,2-trichloroethoxycarbonyl, are reagents such as acetic acid and zinc dust in protic solvents such as methanol at temperatures from about 0°C to about 100°C, more preferably from about 20°C to about 70°C .

The amino dihydropyrazole is then acylated under standard conditions to yield the corresponding acylated amino compound XIX. Preferred solvents are aprotic solvents such as ethyl acetate at temperatures between about -25°C and about 50°C , more preferably between about 0°C and about 20°C.

Alternatively, the amino dihydropyrazole (XVIII) can be alkylated with either an alkyl halide or an aldehyde/sodium cyanoborohydride as described above yielding Compound XXXVI.

When R is hydrogen, hexamethylenetetramine is used as the amine to yield, after hydroloysis, the amino compound (XVIII) wherein R and R² are both hydrogen. The amino compound is subsequently acylated and treated with formaldehyde to give the corresponding prop-2-enone (XV) wherein R is hydrogen.

The reaction with hexamethylenetramine is generally carried out in a solvent such as acetonitrile at temperatures between 0°C and 100°C and preferably at temperatures between 20°C and 70°C. The hydrolysis of the resulting quaternary salt is generally carried out in a solvent such as methanol or ethanol with aqueous acids such as hydrochloric acid at temperatures between 0°C and 100°C and preferably at temperatures between 20°C and 50°C.

The S-alkyl compounds of the formula 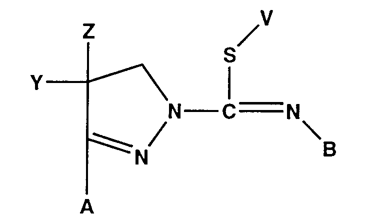 are prepared by alkylating the corresponding thiocarboxamide with the appropriate alkyl halide according to known procedures.

The following examples will further illustrate this invention. In Tables I, II and III, typical 1-substituted-4-substituted-4,5-dihydro-1H-pyrazoles of the present invention are listed. Structures were confirmed by NMR and in some cases by IR and/or elemental analysis. Table IV contains NMR data for those examples of Tables I, II and III which were oils. Specific illustrative preparations of compounds of the invention are described. It will be appreciated by those skilled in the art that the Y and Z substituents can be interchanged within the scope of the present invention.

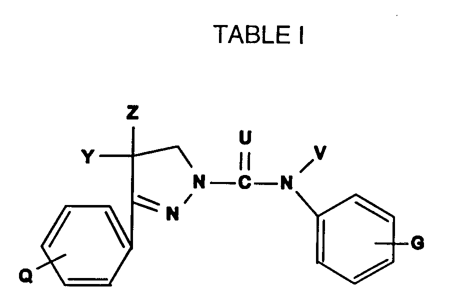

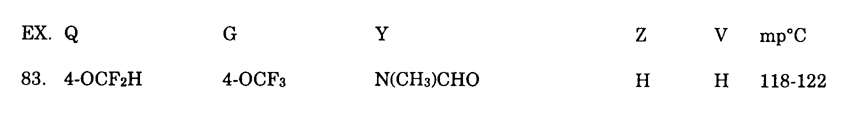

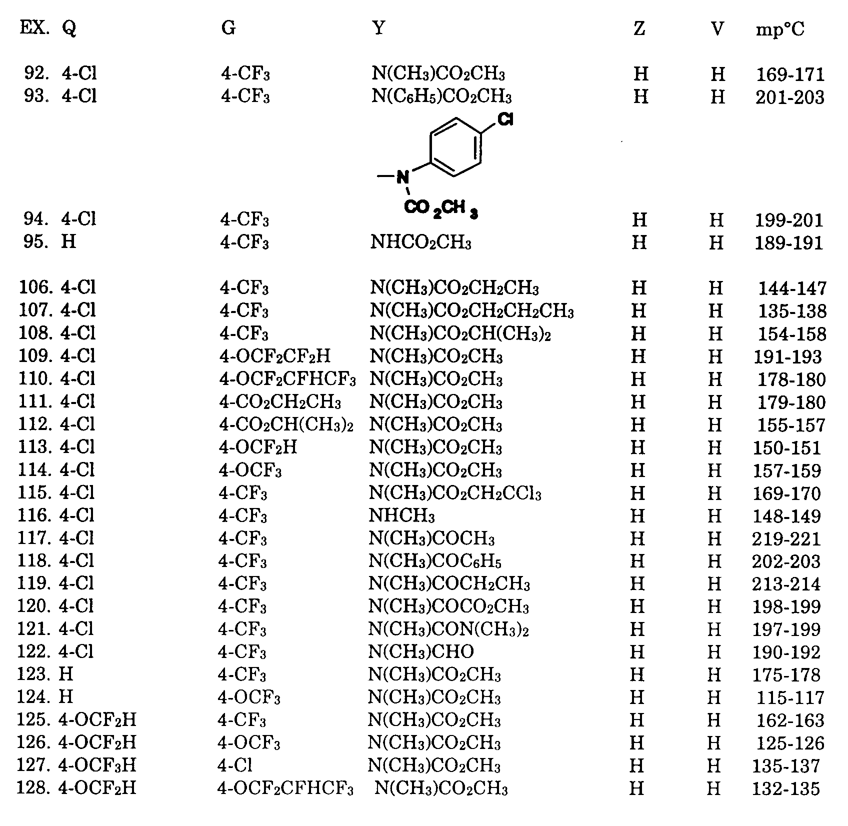

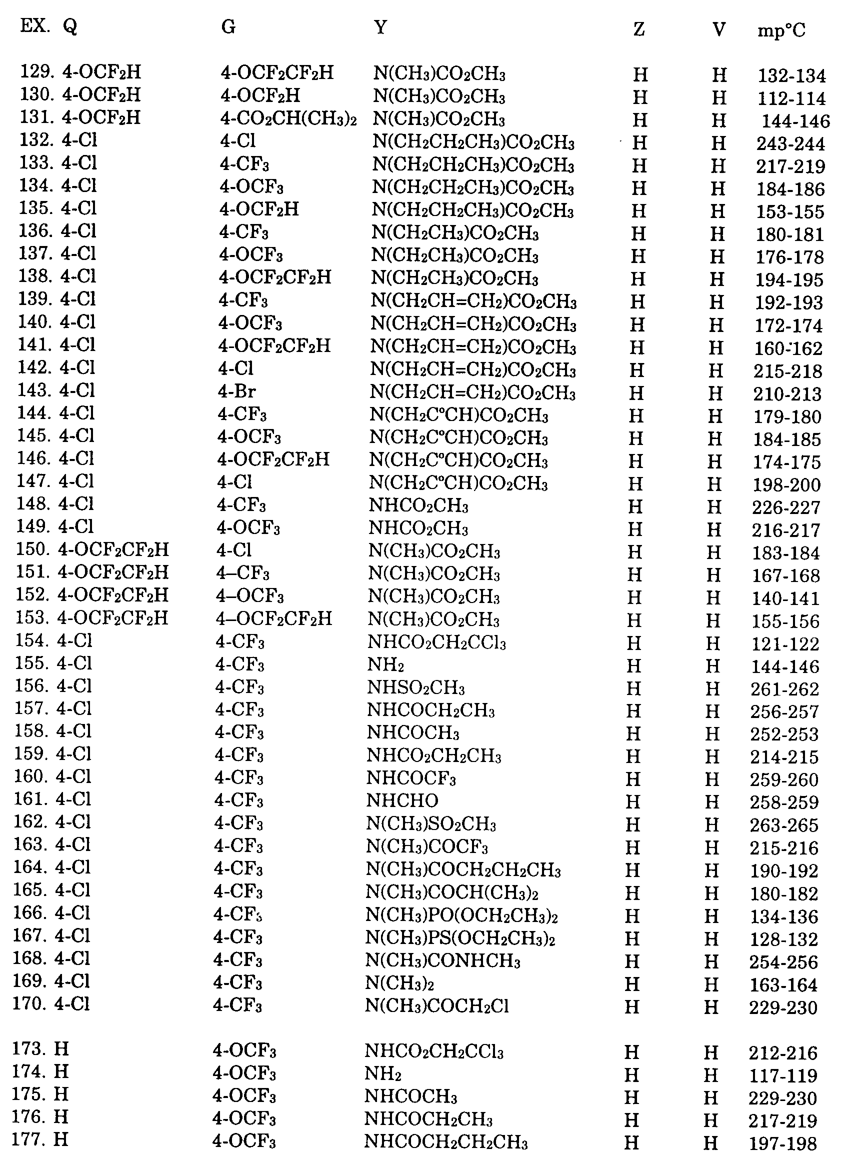

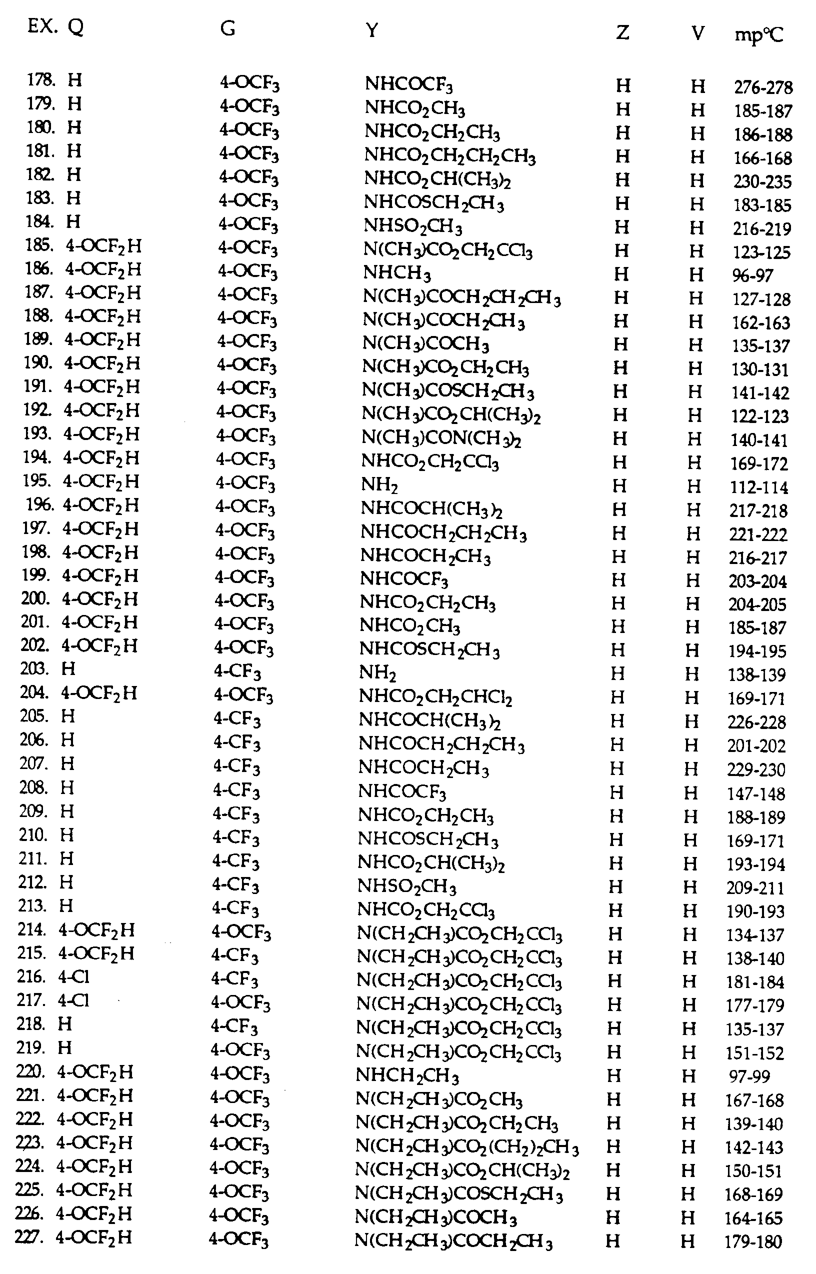

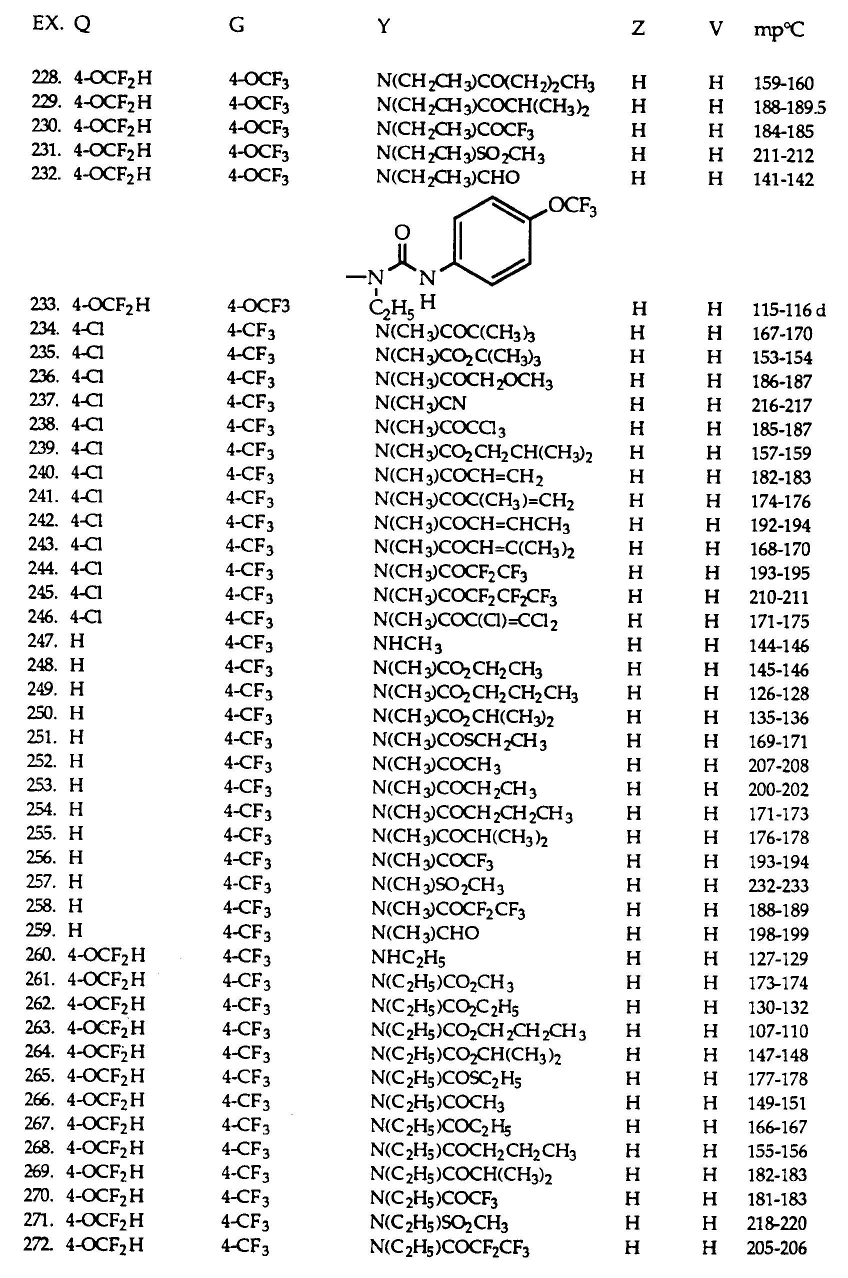

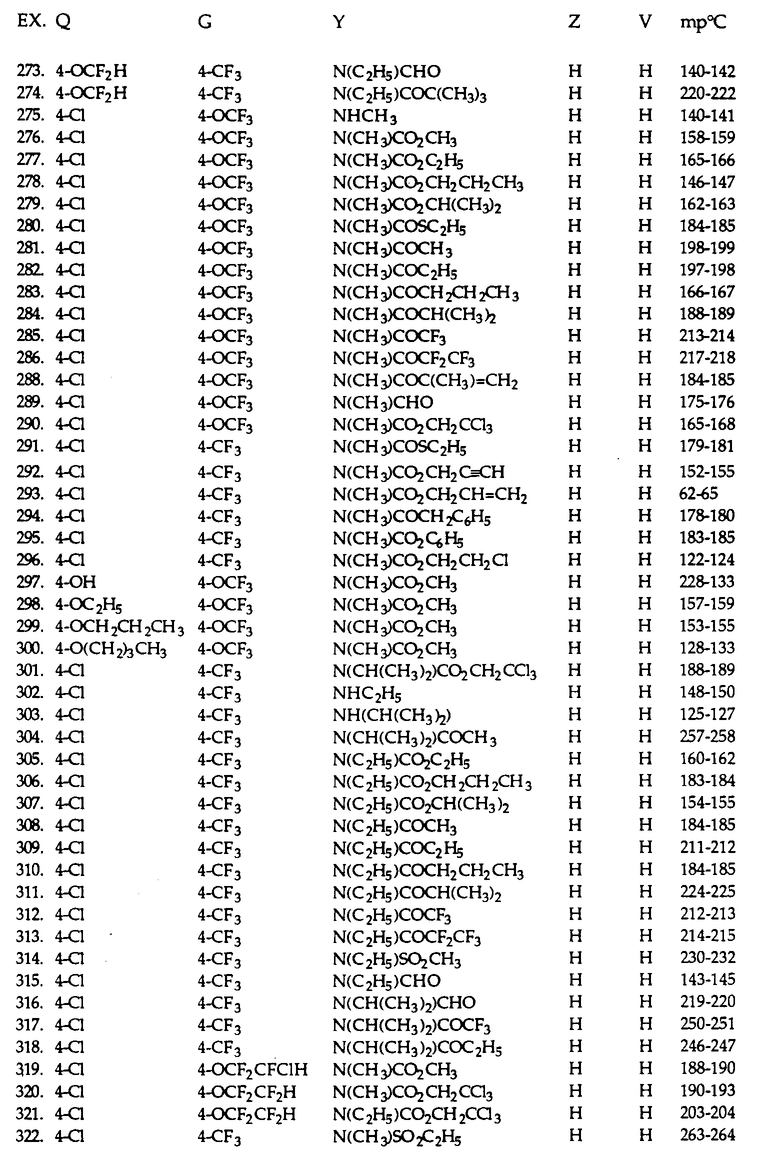

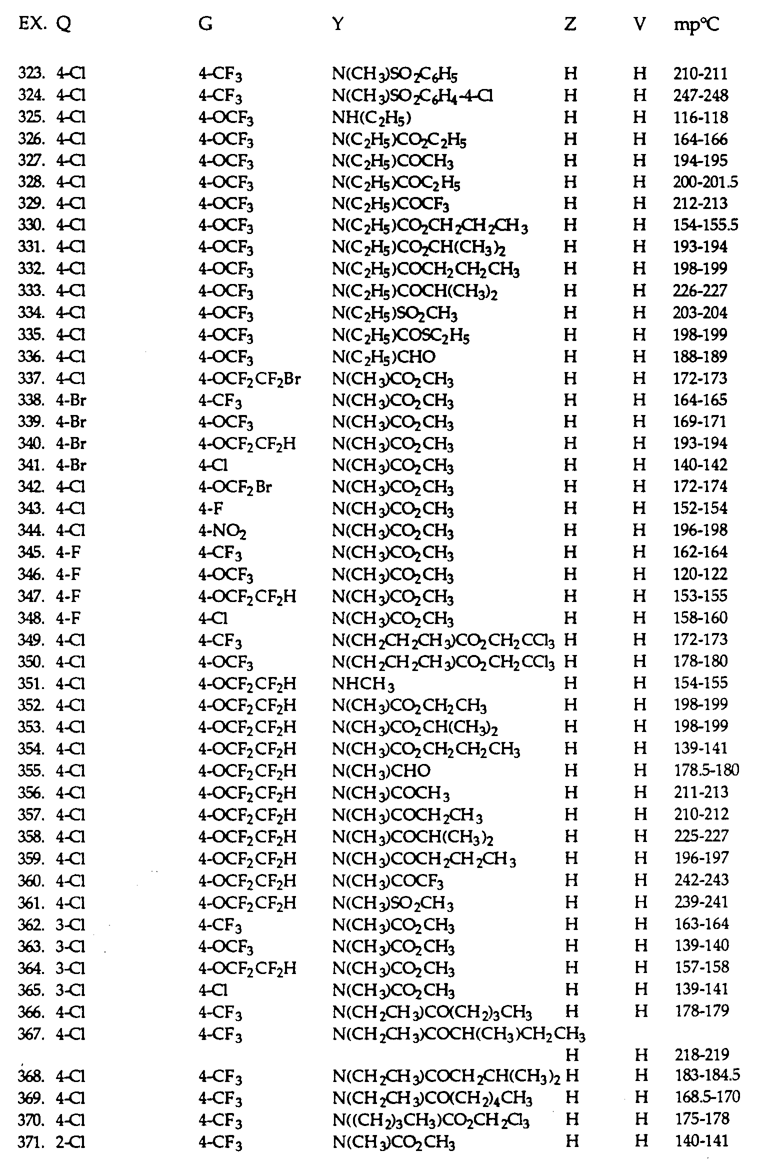

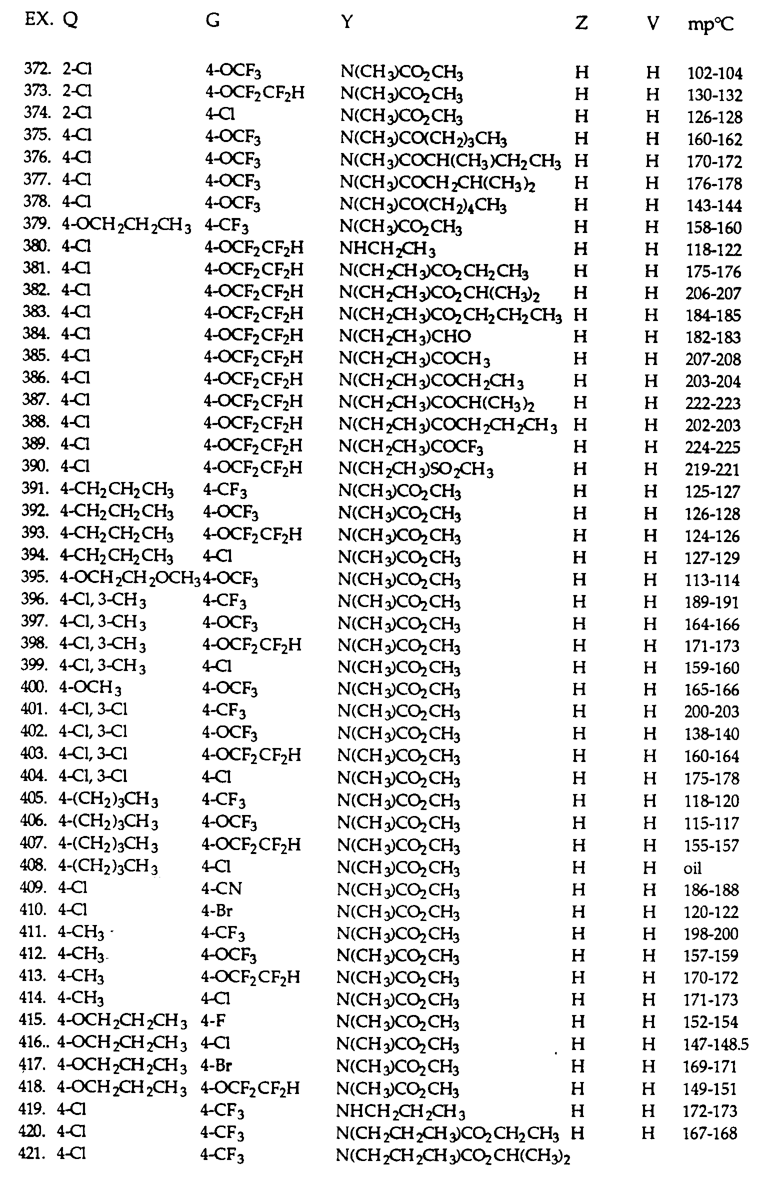

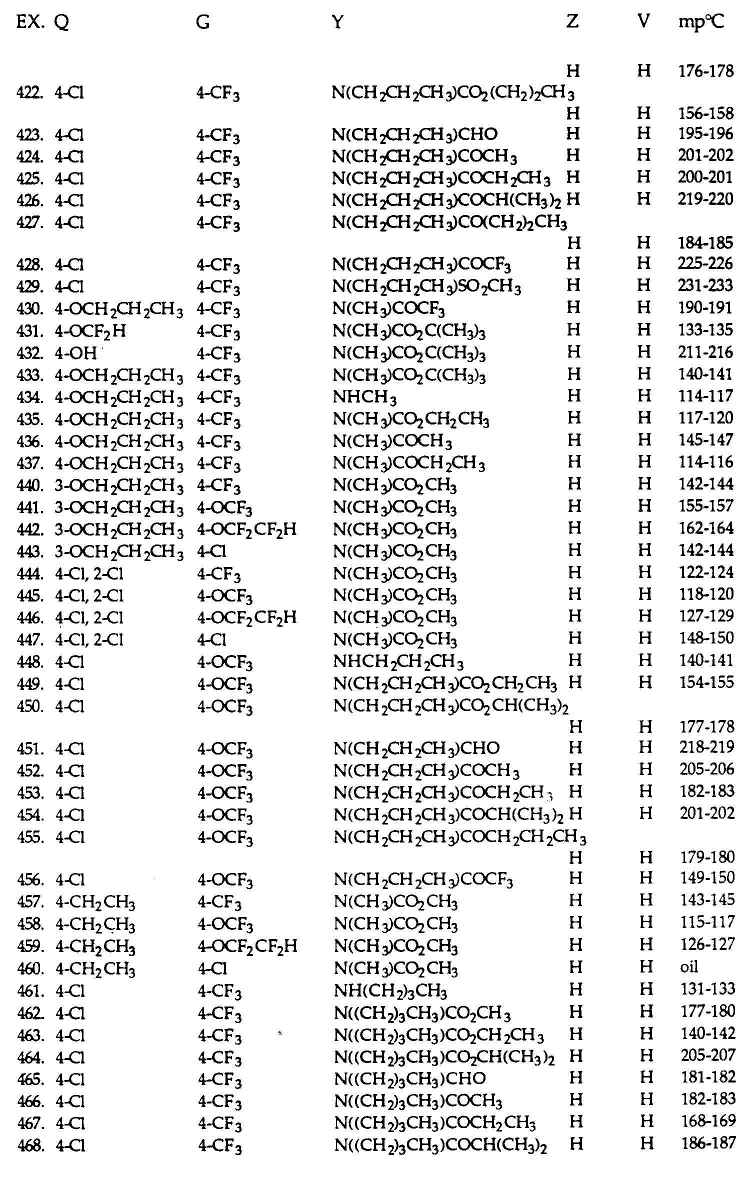

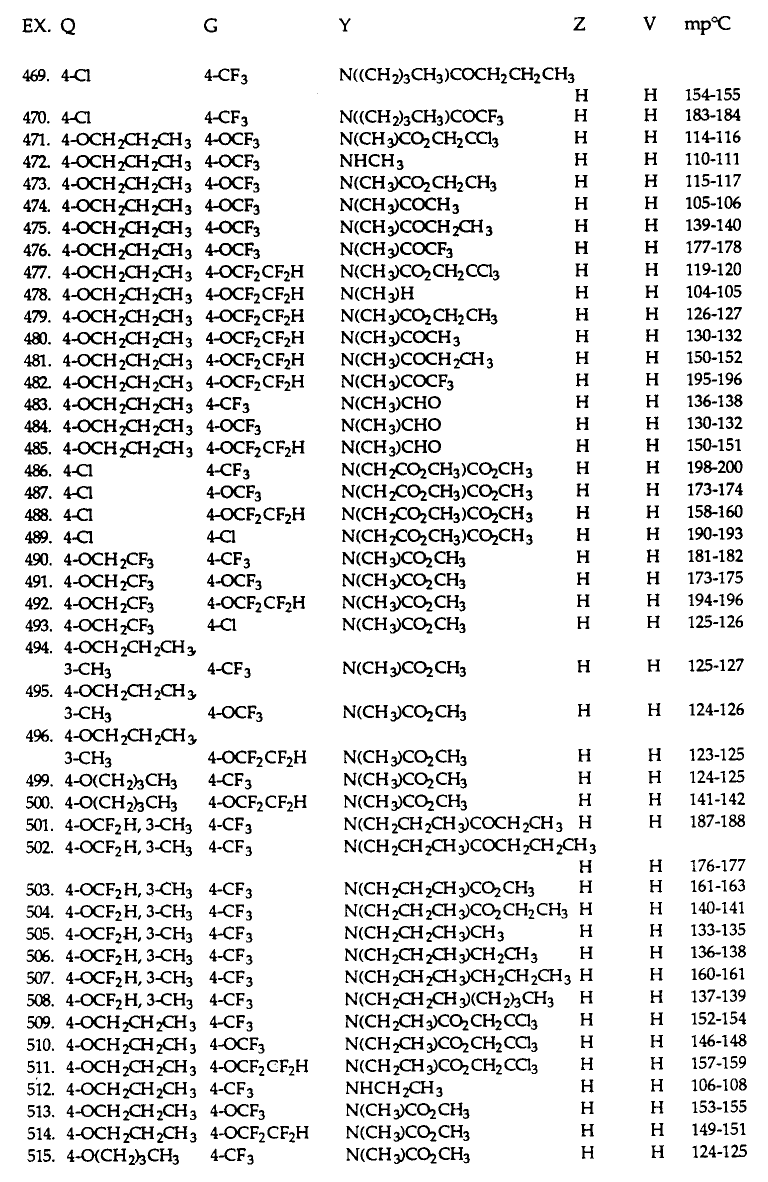

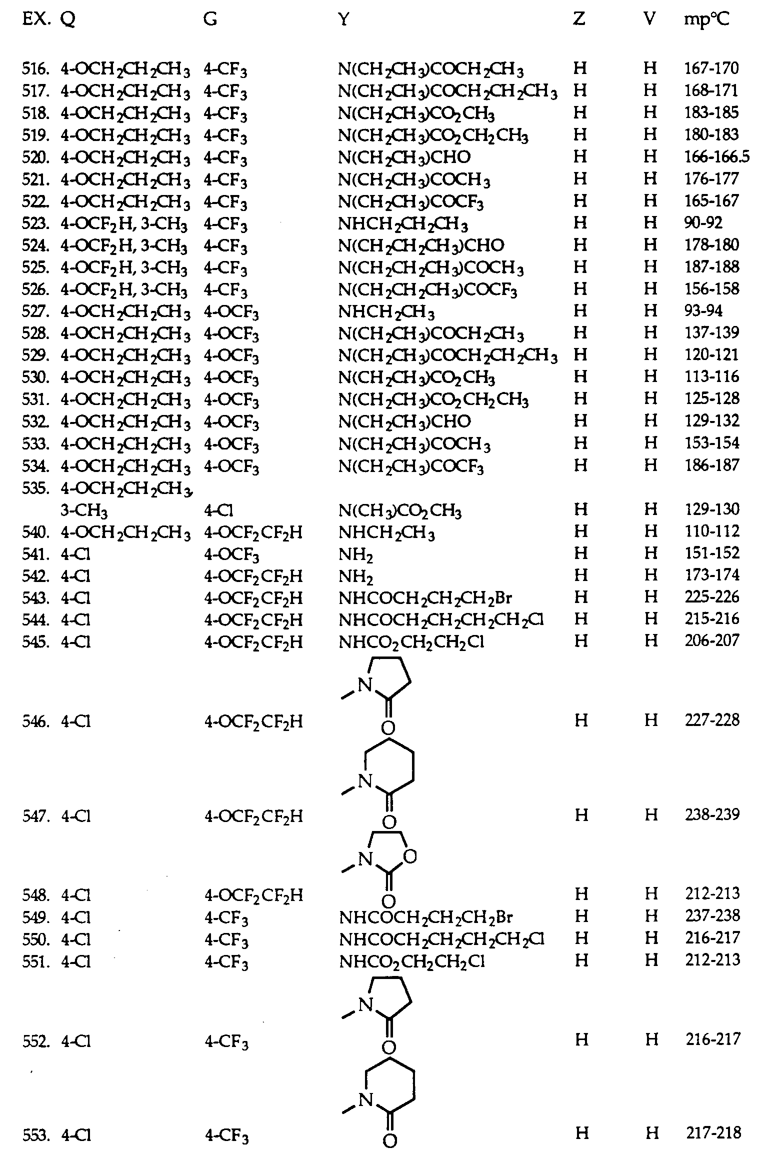

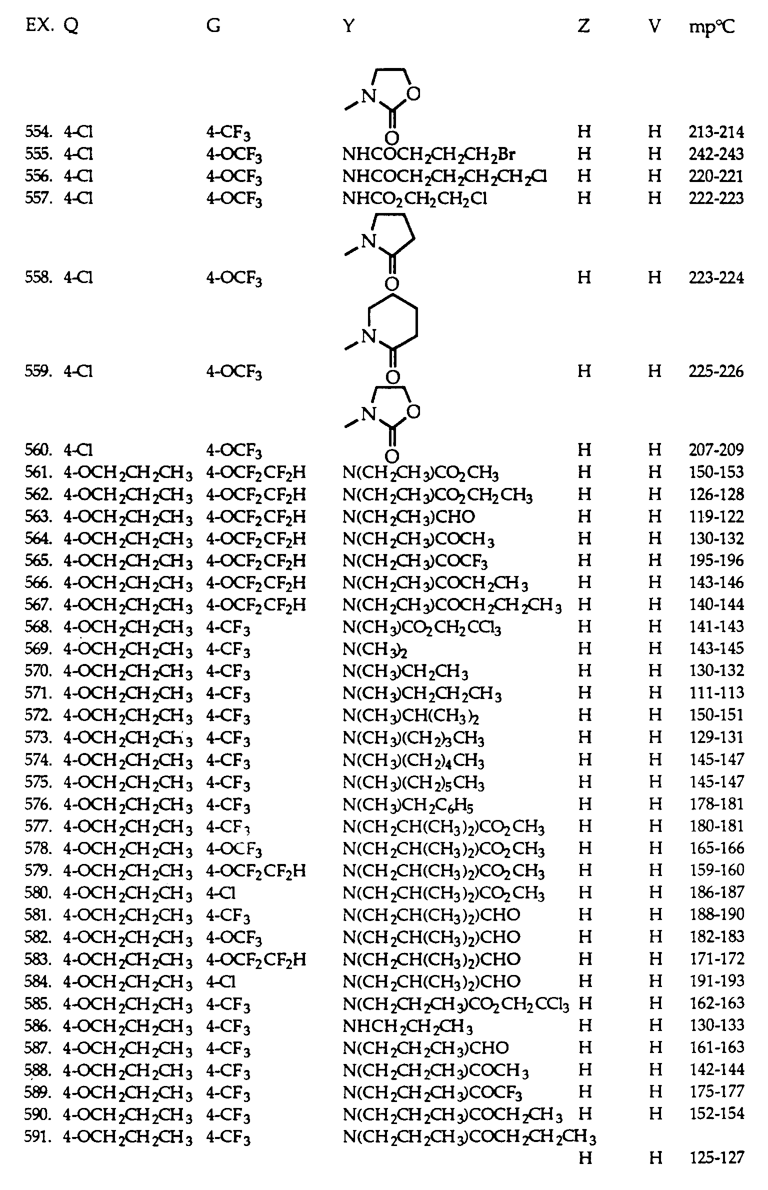

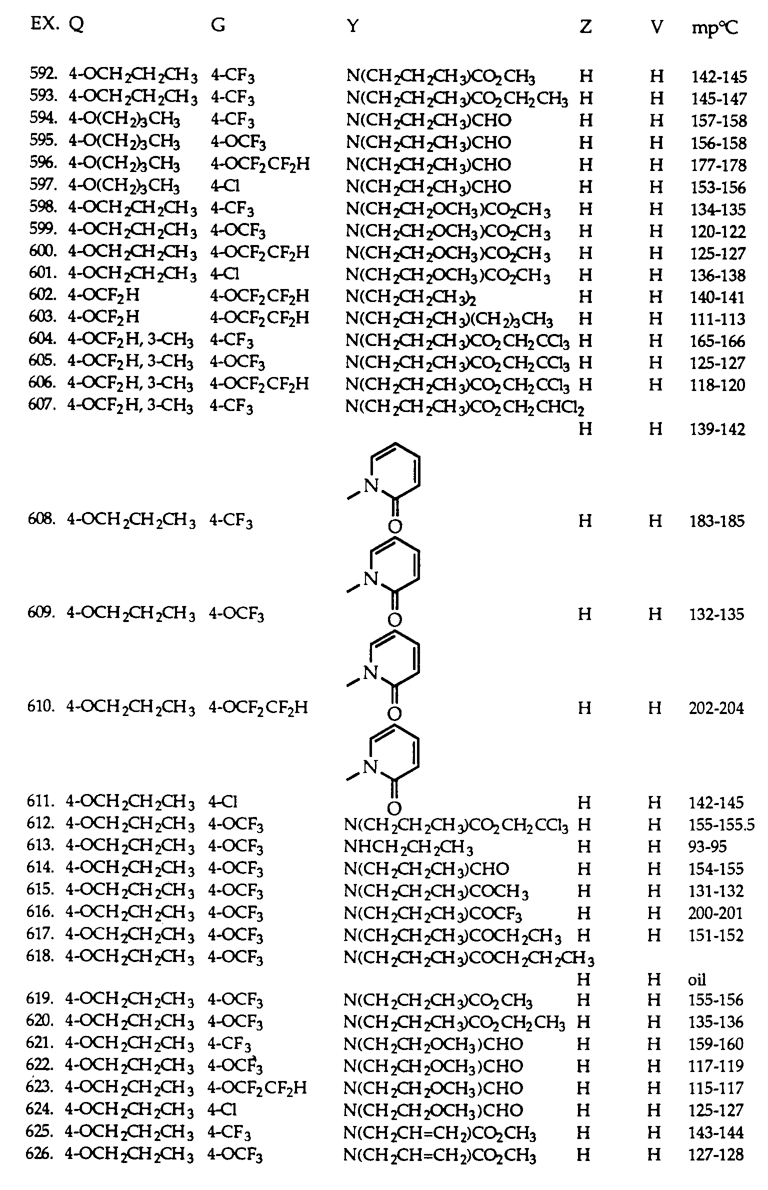

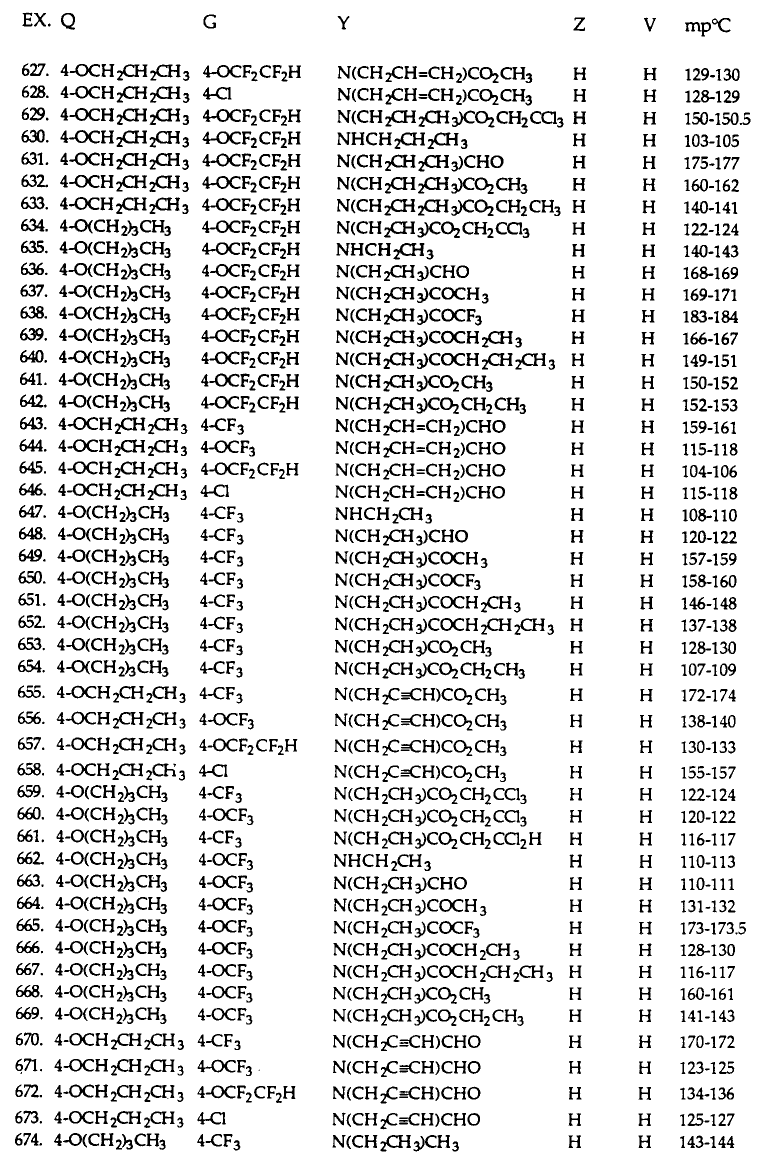

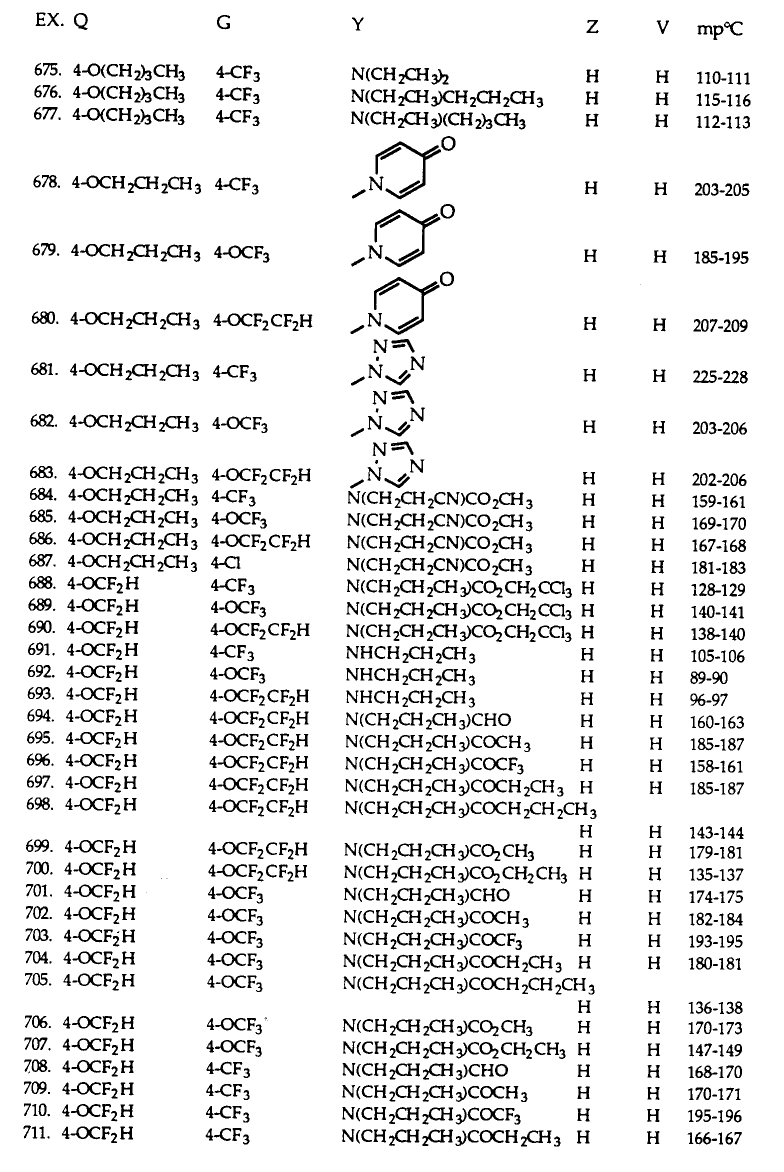

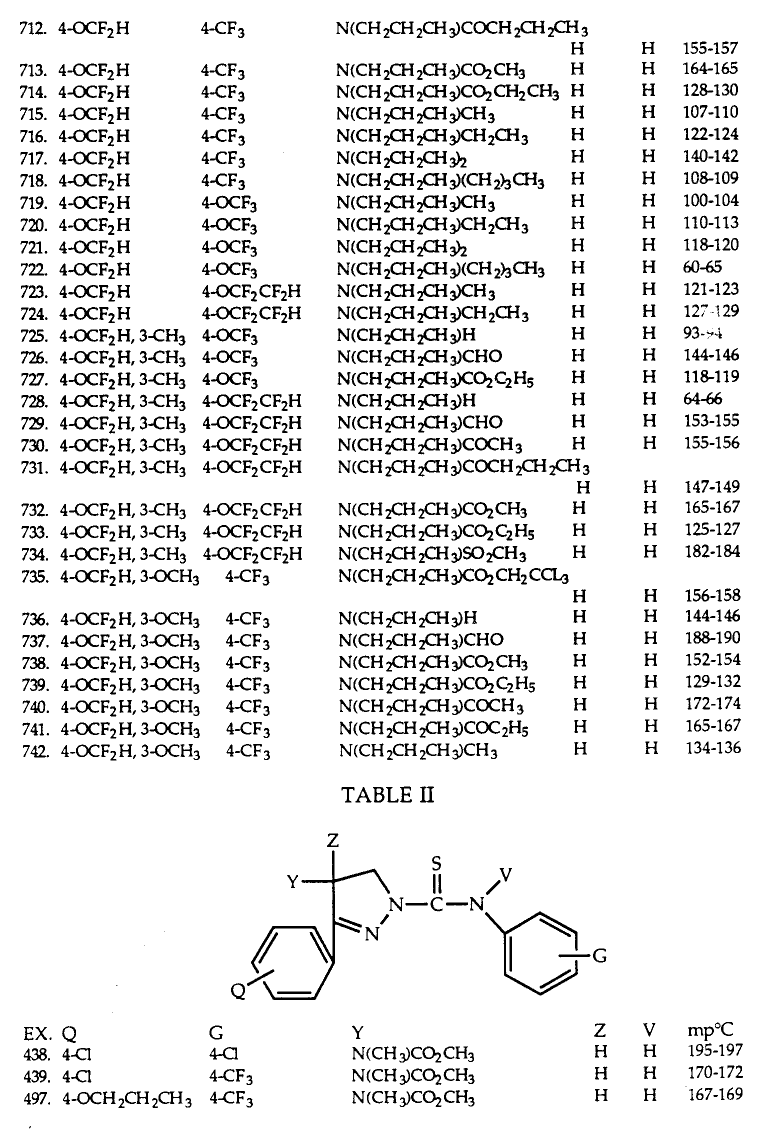

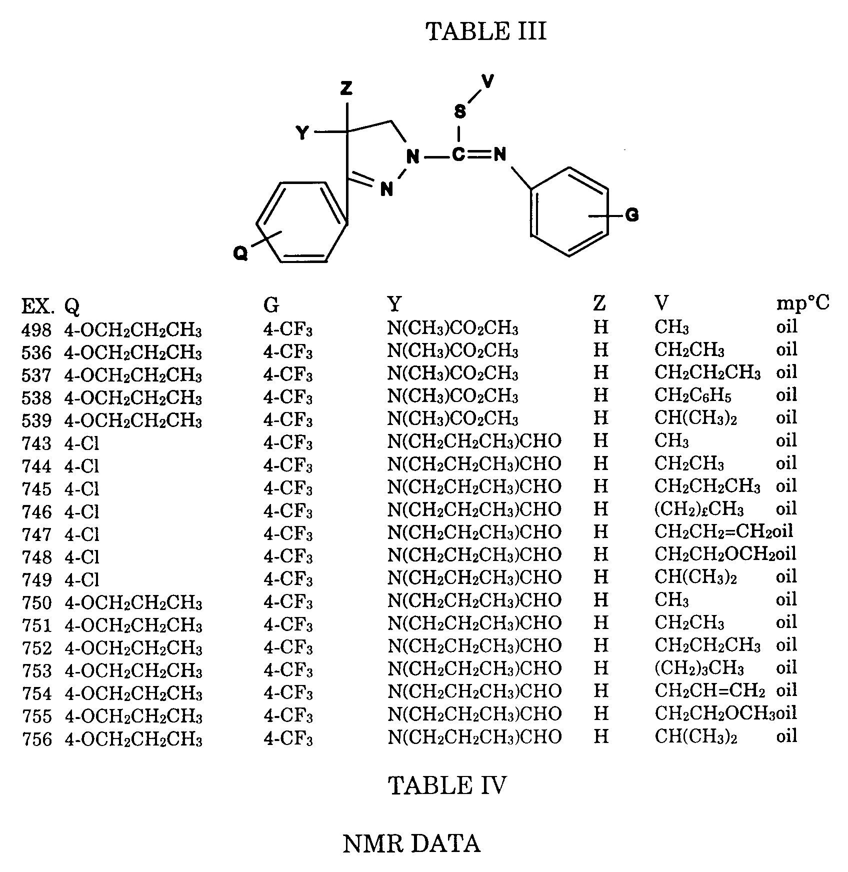

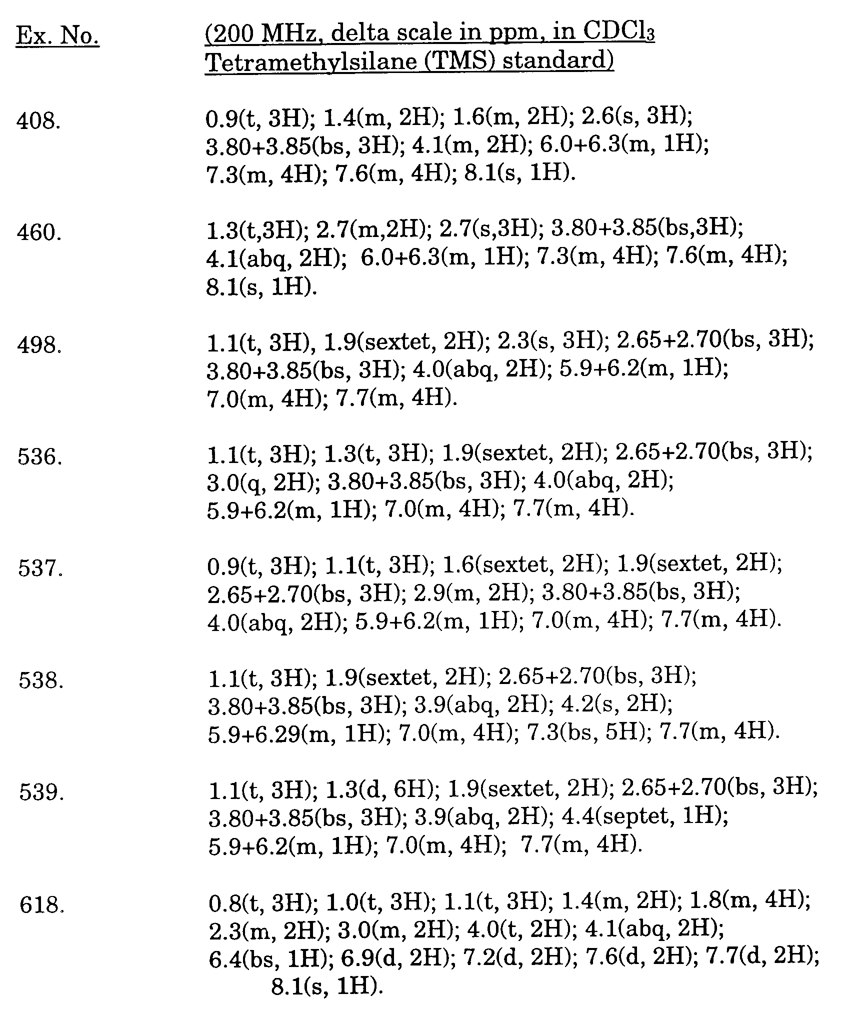

### EXPERIMENTAL

### Comparative Example 1: N-(4-trifluoromethylphenyl)-3-(4-chlorophenyl)-4-carbomethoxyamino-4-methyl-4,5,-dihydro-1H-pyrazole-1-carboxamide

### a: N-(4-trifluoromethylphenyl)-3-(4-chlorophenyl)-4-carboxy-4-methyl-4,5-dihydro-1H-pyrazole-1-carboxamide

To 250 grams (g) (569 mmole) of N-(4-trifluoromethylphenyl)-3(4 chlorophenyl)-4-carbomethoxy-4-methyl-4,5-dihydro-1H-pyrazole-1-carboxamide (USP 4,663,341, compound 149) was added 250 ml of methanol and 250 milliliters (ml) of tetrahydrofuran. The mixture was warmed to 60°C to achieve solution. Separately, 51 g (637 mmole) of 50% aqueous sodium hydroxide was dissolved in 100 ml of methanol. The two solutions were mixed while hot and stirred for 30 minutes whereon no starting material was present by TLC analysis. The mixture was acidified with 55 ml of 37% aqueous hydrochloric acid and 700 ml of tetrahydrofuran and 200 ml of water were then added to dissolve the products. The aqueous layer was separated, dried over magnesium sulfate, filtered, and concentrated in vacuo. The resulting solid was triturated with ethyl ether and dried in a vacuum oven to yield 234 g (97%) of carboxylic acid, mp 142-3°C.

### b: N-(4-trifluoromethylphenyl)-3-(4-chlorophenyl)-4-chlorocarbonyl-4-methyl-4,5-dihydro-1H-pyrazole-1-carboxamide

To a suspension of 100 g (235 mmole) of N-(4-trifluoromethylphenyl)-3-(4-chlorophenyl)-4-carboxy-4-methyl-4,5-dihydro-1H-pyrazole-1-carboxamide in 300 ml of chloroform was added 38 g (319 mmole) of thionyl chloride and 1 g of dimethylformamide. The mixture was refluxed until solution was achieved and gas evolution ceased, about 2 hours. Then 200 ml of toluene was added and the solvents were evaporated in vacuo quantitatively yielding the solid acid chloride which was used unpurified in the next reaction, mp 164-172°C.

### c: N-(4-trifluoromethylphenyl)-3-(4-chlorophenyl)-4-azidocarbonyl-4-methyl-4,5-dihydro-1H-pyrazole-1-carboxamide

To all of the acid chloride prepared in Example 1b (235 mmole) was added 500 ml of acetonitrile. The mixture was warmed to achieve solution and then cooled to 20°C, 20 g (308 mmole) of sodium aside was added and the mixture was stirred for 1 hour. Infared spectroscopy of the crude reaction mixture showed the reaction was complete. Most of the acetonitrile was removed in vacuo with the water bath temperature kept below 40°C. Then 500 ml of toluene was added and the mixture was filtered through Celite® to remove salts. This solution of the acyl azide was used as is in the next reaction.

### d: N-(4-trifluoromethylphenyl)-3-(4-chlorophenyl)-4-isocyanato-4-methyl-4,5-dihydro-1H-pyrazole-1-carboxamide

All the solution of acyl azide prepared in Example 1c was slowly warmed to reflux. When the internal temperature reached about 70°C a gas was vigorously evolved. When reflux was achieved, gas evolution had ceased. After refluxing for 15 minutes the solvent was removed in vacuo and the resulting solid was triturated with about 250 ml of 50/50 ethyl ether/hexanes, yielding 99 g (99%) of isocyanate, mp 146-149°C.

### e: N-(4-trifluoromethylphenyl)-3-(4-chlorophenyl)-4-carbomethoxyamino-4-methyl-4,5-dihydro-1H-pyrazole-1-carboxamide

A solution of 20 g (47 mmole) of N-(4-trifluoromethylphenyl)-3-(4-chlorophenyl)-4-*iso*cyanato-4-methyl-4,5-dihydro-1H-pyrazole-1-carboxamide (Example 1d) in 200 ml of methanol was refluxed for 2 hours, concentrated in vacuo and recrystalized from ethyl acetate/hexanes yielding 19.8 g (92%) of a white solid, mp 179-181°C.

### Comparative Example 2: N-(4-trifluoromethylphenyl)-3-(4-chlorophenyl)-4-carbo-t-butoxyamino-4-methyl-4,5-dihydro-1H-pyrazole-1-carboxamide

To 100 g (236 mmole) of N-(4-trifluoromethylphenyl)-3-(4-chlorophenyl)-4-*iso*cyanato-4-methyl-4,5-dihydro-1H-pyrazole-1-carboxamide (Example 1d) was added 100 g of *t*-butanol, 10 g of pyridine, 10 g of triethylamine, and 100 ml of toluene. The mixture was refluxed for 2 hours, concentrated in vacuo, and triturated with about 250 ml of 1:1 ethyl ether/hexanes yielding 96 g (82%) of a white solid, mp 205-206°C.

### Comparative Example 3: N-(4-trifluoromethylphenyl)-3-(4-chlorophenyl)-4-amino-4-methyl-4,5-dihydro-1H-pyrazole-1-carboxamide

To 74 g (149 mmole) of N-(4-trifluoromethylphenyl)-3-(4-chlorophenyl)-4-carbo-*t*-butoxyamino-4-methyl-4,5-dihydro-1H-pyrazole-1-carboxamide (Example 2) was added 74 g of trifluoroacetic acid and 74 g of chloroform. The mixture was refluxed for 40 minutes while gas was evolved and then an additional 20 minutes. The mixture was then concentrated in vacuo and then partitioned between ethyl ether and dilute aqueous sodium hydroxide. The organic layer was washed with brine, dried over magnesium sulfate, concentrated in vacuo, and the resulting solid was triturated with hexanes and filtered yielding 50 g (85%) of a white solid, mp 165-167°C.

### Comparative Example 43: N-(4-trifluoromethylphenyl)-3-(4-chlorophenyl)-4-methyl-4-(N-ethylamino)-4,5-dihydro-1H-pyrazole-1-carboxamide

To 25 g (63 mmole) of N-(4-trifluoromethylphenyl)-3-(4-chlorophenyl)-4-methyl-4-amino-4,5-dihydro-1H-pyrazole-1-carboxamide (Example 3) dissolved in 100 ml of acetonitrile and 100 ml of tetrahydrofuran was added 3.05 g (69 mmole) of acetaldehyde and 2.02 g (32 mmole) of sodium cyanoborohydride. To this solution was dropwise added 4.0 g (67 mmole) of acetic acid. After 45 minutes, some starting material was still present on tlc, an additional 1.1 g of acetaldehyde, 1.03 g of sodium cyanoborohydride, and 1.5 g of acetic acid were added and the reaction was allowed to stir for an additional 30 minutes. The reaction mixture was concentrated in vacuo, partitioned between diethyl ether and water, washed with dilute aqueous sodium hydroxide, washed with brine, dried over anhydrous magnesium sulfate, and concentrated in vacuo yielding 26 g of Example 43, an oil.

Example 169 was prepared following substantially the same procedure.

### Example 115: N-(4-trifluoromethylphenyl)-3-(4-chlorophenyl)-4-N-methyl-N-(2,2,2,-trichloroethoxycarbonyl)-amino)-4,5,dihydro-1H-pyrazole-1-carboxamide

### a: 2-dimethylamino-4'-chloroacetophenone

To 66 g (1460 mmole) of anhydrous dimethylamine in 200 ml of methylene chloride and cooled to -30°C was added a solution of 155 g (664 mmole) of 2-bromo-4'-chloroacetophenone in 200 ml of methylene chloride. After the addition was complete the reaction was allowed to warm to 20°C. Concentration in vacuo, partitioning between diethyl ether and 1 **M** aqueous sodium hydroxide, washing with brine, drying over anhydrous magnesium sulfate, and reconcentration in vacuo gives 130 g of the title compound, an oil.

### b: 2-(N-methyl-N-(2,2,2-trichloroethoxycarbonyl)-amino)-4'-chloroacetophenone

### Method i

To 130 g (660 mmole) of 2-dimethylamino-4'-chloroacetophenone (Example 115a) in 500 ml of methylene chloride cooled to 0°C was added 153 g (720 mmole) of 2,2,2-trichloroethylchloroformate. After the addition was complete the reaction mixture was allowed to warm to 20°C. Concentration in vacuo, partitioning between diethyl ether and 1 **M** aqueous hydrochloric acid, washing with brine, drying over anhydrous magnesium sulfate, and reconcentration in vacuo gave a crude oil. Chromatography of this oil on silica gel using 15% diethyl ether in hexanes gave 84 g of the title compound, an oil.

### Method ii

By substantially following the procedure of Example 126d(Method ii) and using 117 g (500 mmole) of 2-bromo-4'-chloroacetophenone and 100 g of 2,2,2-trichloroethylchloroformate in place of methyl chloroformate one obtains 158 g of the title compound, an oil.

### c: 2-(N-methyl-N-(2,2,2-trichloroethoxycarbonyl)-amino)-1-(4-chlorophenyl)-prop-2-enone

To 21.5 g (60 mmole) of 2-(N-methyl-N-(2,2,2-trichloroethoxycarbonyl)-amino)-4'-chloroacetophenone (Example 115b) in 100 ml of 1-propanol was added 9.7 g (120 mmole) of 37% formalin, 1 g of piperidine and 0.7 g of acetic acid. The mixture was refluxed for four hours, concentrated in vacuo, partitioned between diethyl ether and water, washed with brine, dried over anhydrous magnesium sulfate, and reconcentrated under vacuo, yielding 20 g of the title compound, an oil.

### d: N-(4-trifluoromethylphenyl)-3-(4-chlorophenyl)-4-(N-methyl-N-(2,2,2-trichloroethoxycarbonyl) -amino)-4,5-dihydro-1H-pyrazole-1-carboxamide

To 35.7 g (96 mmole) of 2-(N-methyl-N-(2,2,2-trichloroethoxycarbonyl)-amino)-1-(4-chlorophenyl)-prop-2-enone (Example 115c) in 60 ml of methanol was added 5.8 g (115 mmole) of hydrazine monohydrate. The mixture was refluxed for ten minutes, concentrated in vacuo, partitioned between diethyl ether and water, washed with brine, dried over anhydrous magnesium sulfate, and filtered. This yielded a diethyl ether solution of 3-(4-chlorophenyl)-4-(N-methyl-N-(2,2,2-trichloroethoxycarbonyl)-amino)-4,5-dihydro-1H-pyrazole which was not isolated. To this solution was added 18 g (96 mmole) of 4-trifluoromethylphenyl *iso*cyanate. After refluxing for 1 hour the mixture was concentrated in vacuo and chromatographed over silica gel using diethyl ether and hexanes to yield 17.3 g of the title compound, a white solid, mp 169-170°C.

Examples 185, 214-219, 290, 301, 320 and 321 were prepared following substantially the same procedure and substituting where appropriate 2-bromoacetophenone or 2-bromo-4'-difluoromethoxyacetophenone for 2-bromo-4'-chloroacetophenone; 4-trifluoromethoxyphenyl *iso*cyanate or 4-tetrafluoroethoxyphenyl *iso*cyanate for 4-trifluoromethyl *iso*cyanate; and *iso*propylamine or ethylamine for methylamine.

### Example 116: N-(4-trifluoromethylphenyl)-3-(4-chlorophenyl)-4-(N-methylamino)-4,5-dihydro-1H-pyrazole-1-carboxamide

To 17.3 g (30 mmole) of N-(4-trifluoromethylphenyl)-3-(4-chlorophenyl)-4-(N-methyl-N-(2,2,2-trichloroethoxycarbonyl)-amino)-4,5-dihydro-1H-pyrazole-1-carboxamide (Example 115) dissolved in 50 ml of methanol and 50 ml of tetrahydrofuran was added 14 g (233 mmole) of acetic acid and 3.95 g (60 mmole) of zinc dust. After stirring for 1 hour the reaction mixture was filtered, concentrated in vacuo, dissolved in diethyl ether, washed with water, 5% aqueous sodium bicarbonate, and brine and dried over anhydrous magnesium sulfate. Concentration in vacuo and chromatography over silica gel using diethyl ether, hexanes, and ethyl acetate yielded 7.3 g of the compound, of Example 116, a white solid, mp 148-149°C.

Examples 186, 220, 247, 260, 275, 302, 303 and 325 were prepared following substantially the same procedure.

### Example 126: N-(4-trifluoromethoxyphenyl)-3-(4-difluoromethoxy-phenyl)-4-(N-methyl-N-(methoxycarbonyl)-amino)-4,5-dihydro-1H-pyrazole-1-carboxamide

### a: 4-difluoromethoxyacetophenone

To 200 g (1470 mmole) of 4-hydroxyacetophenone dissolved in 1000 ml of dimethylformamide and cooled to 10°C was added gaseous chlorodifluoromethane until the mixture was saturated. While vigorously mechanically stirring the mixture, 330 g (2600 mmole) of 45% aqueous potassium hydroxide was added while cofeeding chlorodifluoromethane to maintain an excess. The internal temperature was maintained at 10°C during the addition. After standing overnight, the reaction mixture was carefully poured onto 5000 ml of water, gas is released! Extraction with a mixture of diethyl ether and hexanes, drying over anhydrous magnesium sulfate, and vacuum distillation yields 173 g of 4-difluoromethoxyacetophenone, an oil, bp 65-70°C at 0.2 torr.

### b: 2-bromo-4'-difluoromethoxyacetophenone

### Method i

To 173 g (930 mmole) of 4-difluoromethoxyacetophenone (Example 126a) dissolved in 150 ml of methylene chloride was added a few drops of bromine. The mixture was heated until the bromine color dissipated and then 25 ml of dioxane was added followed by 45 g (872 mmole) of bromine over the course of 30 minutes. Hydrogen bromide evolved. After the addition was complete the solvents are removed in vacuo and the product is taken up in diethyl ether and washed with water and brine. After drying over anhydrous magnesium sulfate the mixture was concentrated in vacuo and crystalized from 200 ml of 1:1 diethyl ether/hexanes yielding 164 g of the title compound, a white solid, mp 64-66°C.

### Method ii

By substantially following the proceedures of Example 585b using 4'-difluoromethoxyacetophenone (Example 126a) one obtains the desired compound, mp 64-66°C. The crude mixture of unbrominated, monobrominated, and dibrominated compounds could also be used as is in the next reaction.

### c: 2-dimethylamino-4'-difluoromethoxyacetophenone

By substantially following the procedure of Example 115a using 20 g (75 mmole) of 2-bromo-4'-difluoromethoxyacetophenone (Example 126b) 16.8 g of 2-dimethylamino-4'-difluoromethoxyacetophenone, an oil, was obtained.

### d: 2-(N-methyl-N-(methoxycarbonyl)-amino)-4'-difluoromethoxyacetophenone

### Method i

By substantially following the procedure of Example 115b using 16.8 g (73 mmole) of 2-dimethylamino-4'-difluoromethoxy-acetophenone Example 126c and 7.6 g (81 mmole) of methyl chloroformate one obtains 12 g of 2-(N-methyl-N-(methoxycarbonyl)-amino)-4'-difluoromethoxyacetophenone, an oil, bp 150-190°C at 0.7 torr.

### Method ii

To 47 g (1510 mmole) of monomethylamine dissolved in 300 ml of methylene chloride and cooled to -50°C was added a solution of 136 g (512 mmole) of 2-bromo-4'-difluoromethoxyacetophenone (Example 126b) in 200 ml of methylene chloride. The internal temperature rose to 15°C. This mixture was allowed to stir for 15 minutes and then a solution of 40 g (500 mmole) of 50% aqueous sodium hydroxide in 100 ml of water was added. The resulting mixture was rapidly washed with two 500 ml portions of ice water. The resulting organic layer was cooled to 5°C and 52 g (550 mmole) of methyl chloroformate and a mixture of 40 g (500 mmole) of 50% aqueous sodium hydroxide in 150 ml of water were simultaneously added with rapid stirring. The internal temperature was maintained between 0°C and 10°C. After 10 minutes the organic layer was separated and washed with water and dilute aqueous hydrochloric acid. After drying over anhydrous magnesium sulfate, the methylene chloride was removed in vacuo and the diethyl ether soluble portion was filtered through silica gel. Concentration in vacuo yielded 90 g of the title compound as a tan solid, mp 50-52°C.

### e: 2-(N-methyl-N-(methoxycarbonyl)-amino)-1-(4-difluoromethoxyphenyl)-prop-2-enone

By substantially following the procedure of Example 115c, using 9.1 g (33 mmole) of 2-(N-methyl-N-(methoxycarbonyl)-amino)-4'-difluoromethoxyacetophenone (Example 126d) was used to obtain 5.1 g of the title compound, an oil.

### f: N-(4-trifluoromethoxyphenyl)-3-(4-difluoromethoxyphenyl)-4-(N-methyl-N-(methoxycarbonyl)-amino)-4,5-dihydro-1H-pyrazole-1-carboxamide

By substantially following the procedure of Example 115d, using 3.1 g (10 mmole) of 2-(N-methyl-N-(methoxycarbonyl)-amino)-1-(4-difluoromethoxyphenyl)-prop-2-enone (Example 126e) and 2.0 g (10 mmole) of 4-trifluoromethoxyphenyl *iso*cyanate yields 2.1 g of the title compound, a white solid, mp 125-126°C.

Examples 92-94, 109-114, 123-125, 127-131, 150-153, 319, 337-342, 343-346, 348, 362-365, 371-374, 391-394, 396-399, 401-414, 431, 440-447, 457-460, 490-496, and 535 were prepared following substantially the same procedure, substituting, where appropriate, for
4′-difluoromethoxyacetophenone a compound selected from
4′-chloroacetophenone, 4′-fluoroacetophenone,
3′-chloroacetophenone, 2′-chloroacetophenone,
4′-propylacetophenone, 4′-chloro-3′-methylacetophenone,
3′,4′-dichloroacetophenone, 4′-butylacetophenone,
4′-methylacetophenone, 2′,4′-dichloroacetophenone,
4′-propoxyacetophenone, 3′-propoxyacetophenone,
4′-ethylacetophenone, 4′-(2,2,2-trifluoroethoxy)-acetophenone,
4′-difluoromethoxy-3′-methylacetophenone,
4′-difluoromethoxy-3′-methoxyacetophenone, or
4′-propoxy-3′-methylacetophenone, and substituting where appropriate for 4-trifluoromethoxyphenyl *iso*cyanate a compound selected from
4-trifluoromethylphenyl *iso*cyanate, 4-tetrafluoroethoxyphenyl *iso*cyanate, 4-chlorophenyl *iso*cyanate, 4-fluorophenyl *iso*cyanate,
4-bromophenyl *iso*cyanate, 4-nitrophenyl *iso*cyanate, or 4-cyanophenyl *iso*cyanate

### Example 145: N-(4-trifluoromethoxyphenyl)-3-(4-chlorophenyl)-4-(N-propargyl-N-carbomethoxyamino)-4,5-dihydro-1H-pyrazole-1-carboxamide

### a: 2-(N-propargyl-N-carbomethoxyamino)-4′-chloroacetophenone

To 25 g (454 mmole) of propargylamine in 200 ml of methylene chloride cooled to -40°C was rapidly added 46 g (200 mmole) of 2-bromo-4′-chloroacetophenone dissolved in 200 ml of methylene chloride. The temperature rose to 0°C and was maintained there for 15 minutes. The reaction mixture was washed with 250 ml of 1 **M** sodium hydroxide and recooled to -20°C whereon 22 g (233 mmole) of methyl chloroformate was added while maintaining an internal temperature of -20°C, then 20 g (253 mmole) of pyridine was added. After stirring for 1 hour the mixture was concentrated in vacuo, partitioned between diethyl ether and water, washed with dilute hydrochloric acid, washed with brine, dried over anhydrous magnesium sulfate, reconcentrated in vacuo, and chromatographed over silica gel using diethyl ether and hexanes to yield 34 g of the expected compound, an oil.

### b: 2-(N-propargyl-N-carbomethoxyamino)-1-(4-chlorophenyl)-prop-2-enone

By substantially following the procedure given in Example 115c using 9.9 g of 2-(N-propargyl-N-carbomethoxyamino)-4′-chloroacetophenone 9.7 g of the desired compound, an oil, was obtained.

### c: N-(4-trifluoromethoxyphenyl)-3-(4-chlorophenyl)-4-(N-propargyl-N-carbomethoxyamino)-4,5-dihydro-1H-pyrazole-1-carboxamide

By substantially following the procedure given in Example 115d using 2.3 g (8.3 mmole) of 2-(N-propargyl-N-carbomethoxyamino)-1-(4-chlorophenyl)-prop-2-enone (Example 145b) and 1.7 g (8.4 mmole) of 4-trifluoromethoxyphenyl *iso*cyanate, 1.4 g of the desired compound, mp 184-185°C, were obtained.

Examples 132-144 and 146 were prepared following substantially the same procedure and substituting where appropriate, propylamine, ethylamine or allylamine for propargylamine; and 4-chlorophenyl *iso*cyanate, 4-trifluoromethylphenyl *iso*cyanate, 4-difluoromethoxyphenyl *iso*cyanate, 4-tetrafluoroethoxyphenyl *iso*cyanate or 4-bromophenyl *iso*cyanate for 4-trifluoromethoxyphenyl *iso*cyanate.

### Example 154: N-(4-trifluoromethylphenyl)-3-(4-chlorophenyl)-4-(N-(2,2,2-trichloroethoxycarbonyl) amino)-4,5-dihydro-1H-pyrazole-1-carboxamide

### a: 2-(N-(2,2,2-trichloroethoxycarbonyl)-amino)-4′-chloroacetophenone

To a slurry of 30 g (214 mmole) of hexamethylenetetramine in 400 ml of acetonitrile was added 50 g (214 mmole) of 2-bromo-4′-chloroacetophenone in 100 ml of warm acetonitrile, the mixture self warms to 45°C. After stirring for 1 hour the mixture was diluted with diethyl ether and filtered giving a crude quaternary salt which was used directly in the next step.

To a slurry of this crude quaternary salt in 400 ml of ethanol was added 80 ml of 37% aqueous hydrochloric acid. After stirring for 1 hour all was in solution. The resulting mixture was concentrated in vacuo, basified with dilute aqueous sodium hydroxide and extracted with methylene chloride giving a solution of crude 2-amino-4′-chloroacetophenone which was used as is in the next step.

The methylene chloride solution of 2-amino-4′-chloroacetophenone was cooled to 0°C and 80 g (377 mmole) of 2,2,2-trichloroethylchloroformate was added along with 60 g of 25% aqueous sodium hydroxide. Separation of the organic layer, drying over anhydrous magnesium sulfate, concentration in vacuo and trituration with diethyl ether and hexanes gave 63 g of the 2-(N-(2,2,2-trichloroethoxycarbonyl)-amino)-4′-chloroacetophenone, a white solid. mp 104-105°C.

### b: 2-(N-(2,2,2-trichloroethoxycarbonyl)-amino)-1-(4-chlorophenyl)-prop-2-enone

To 37 g (107 mmole) of 2-(N-(2,2,2-trichloroethoxycarbonyl)-amino)-4′chloroacetophenone (Example 154a) in 100 ml of methanol was added 17.4 g (214 mmole) of 37% formalin, 1.7 g of piperidine and 1.2 g of acetic acid. The mixture was refluxed for 1 hour, concentrated in vacuo, partitioned between diethyl ether and water, washed with brine, dried over anhydrous magnesium sulfate, and concentrated in vacuo to yield 36.6 g of the title compound, an oil.

### c: N-(4-trifluoromethylphenyl)-3-(4-chlorophenyl)-4-(N-(2,2,2-trichloroethoxycarbonyl)-amino)-4,5-dihydro-1H-pyrazole-1-carboxamide

To 36.6 g (102 mmole) of 2-(N-(2,2,2-trichloroethoxycarbonyl)-amino)-1-(4-chlorophenyl)-prop-2-enone (Example 154b) in 50 ml of methanol was added 6 g (120 mmole) of hydrazine monohydrate. The reaction mixture was refluxed for 15 minutes, cooled, concentrated in vacuo, partitioned between diethyl ether and water, washed with brine, and dried over anhydrous mangesium sulfate. The resulting solution of 3-(4-chlorophenyl)-4-(N-(2,2,2-trichloroethoxycarbonyl)-amino)-4,5-dihydro-1H-pyrazole was treated, at reflux, with 19 g (102 mmole) of 4-trifluoromethyphenyl *iso*cyanate. The precipitate was filtered and dried yielding 17 g of the compound of Example 154, a white solid, mp 121-122°C.

Examples 95, 148, 149, 173 and 194 were prepared following substantially the same procedure and where appropriate substituting methyl chloroformate for 2,2,2-trichloroethylchloroformate;
2-bromoacetophenone or 2-bromo-4′-difluoromethoxyacetophenone for 2-bromo-4′-chloroacetophenone; and 4-trifluoromethoxyphenyl *iso*cyanate for 4-trifluoromethylphenyl *iso*cyanate.

### Example 155: N-(4-trifluoromethylphenyl)-3-(4-chlorophenyl)-4-amino-4,5-dihydro-1H-pyrazole-1-carboxamide

To 20 g (36 mmole) of N-(4-trifluoromethylphenyl)-3-(4-chlorophenyl)-4-(N-(2,2,2-trichloroethoxycarbonyl)-amino)-4,5-dihydro-1H-pyrazole-1-carboxamide (Example 154) in 50 ml of methanol and 50 ml of tetrahydrofuran was added 4.69 g (72 mmole) of zinc dust and 16 g (267 mmole) of acetic acid. After stirring for 1 hour the reaction was filtered, concentrated in vacuo, partitioned between diethyl ether and water, washed with 5% aqueous sodium bicarbonate, washed with brine, dried over anhydrous magnesium sulfate, concentrated in vacuo, and chromatographed on silica gel using methylene chloride and ethyl acetate to yield 6 g of the title compound, a white solid, mp 144-146°C.

Examples 174, 195, 203, and 541-542 were prepared following substantially the same procedure starting from, for example, Examples 173 and 194.

Example 204 was isolated as a side product of the preparation of Example 195.

### Example 160: N-(4-trifluoromethylphenyl)-3-(4-chlorophenyl)-4-trifluoroacetylamino-4,5-dihydro-1H-pyrazole-1-carboxamide

To 1.9 g (5.0 mmole) of N-(4-trifluoromethylphenyl)-3-(4-chlorophenyl)-4-amino-4,5-dihydro-1H-pyrazole-1-carboxamide (Example 155) in 10 ml of methylene chloride was added 1.1 g (5.2 mmole) of trifluoroacetic anhydride followed by 0.40 g (5.1 mmole) of pyridine. After stirring for 1 hour the reaction mixture was concentrated in vacuo, partitioned between ethyl acetate and water, washed with brine, dried over anhydrous magnesium sulfate, concentrated in vacuo, and triturated with diethyl ether and hexanes to yield 1.8 g of the title compound, a white solid, mp 259-260°C.

Examples 156-159, 161, 175-184, 197-202, 205-213, 543-545, 549-551 and 555-557 were prepared following substantially the same procedure starting from Examples 155, 174, 195, 203, 541 and 542 where appropriate substituting for trifluoroacetic anhydride a compound selected from: methanesulfonyl chloride, propionyl chloride, formic acetic anhydride, acetic anhydride, propionyl chloride, butyryl chloride, methyl chloroformate, ethyl chloroformate, *n*-propyl chloroformate, *iso*propyl chloroformate, S-ethyl thiochloroformate, 4-bromobutyryl chloride, 5-chlorovaleryl chloride or 2-chloroethyl chloroformate.

### Example 165: N-(4-trifluoromethylphenyl)-3-(4-chlorophenyl)-4-(N-methyl-N-(isobutyryl)-amino)-4,5-dihydro-1H-pyrazole-1-carboxamide

To 1.8 g (4.5 mmole) of N-(4-trifluoromethylphenyl)-3-(4-chlorophenyl)-4-(N-methylamino)-4,5-dihydro-1H-pyrazole-1-carboxamide (Example 116) in 10 ml of ethyl acetate cooled to 0°C was added 0.5 g (4.7 mmole) of *iso*butyryl chloride and 0.4 g of pyridine. After stirring overnight the mixture was diluted with ethyl acetate, washed with water and brine and dried over anhydrous magnesium sulfate. Concentration in vacuo and trituration with diethyl ether gave 1.7 g of the title compound, a white solid, mp 180-182°C.

Examples 106-108, 117-122, 162-164, 166-168, 170, 187-193, 222-236, 238-246, 248-259, 261-274, 276-289, 291-296, 304-318, 322-324 and 326-336 were prepared following substantially the same procedure starting from Examples 115, 186, 220, 247, 260, 275, 302, 303 or 326 and where appropriate substituting for *iso*butytyl chloride a compound selected from: propionyl chloride, *n*-butyryl chloride, methyl chloroformate, ethyl chloroformate, *n*-propyl chloroformate, *iso*propyl chloroformate, S-ethyl thiochloroformate, *iso*butyl chloroformate, allyl chloroformate, propargyl chloroformate, benzyl chloroformate, phenyl chloroformate, 2-chloroethyl chloroformate, acetic anhydride, benzoyl chloride, ethyl oxalyl chloride, methyl *iso*cyanate, dimethylcarbamyl chloride, formic acetic anhydride, methanesulfonyl chloride, ethanesulfonyl chloride, phenylsulfonyl chloride, 4-chlorophenylsulfonyl chloride, trifluoroacetic anhydride, diethyl chlorophosphate, diethyl chlorothiophosphate, 4-trifluoromethoxyphenyl *iso*cyanate, di-*t*-butyl dicarbonate, pivaloyl chloride, methoxyacetyl chloride, trichloroacetyl chloride, acryloyl chloride, methacrylic anhydride, crotonyl chloride, 3,3-dimethylacryloyl chloride, pentafluoropropionic anhydride, heptafluorobutyryl chloride or trichloroacryloyl chloride.

### Example 237 : N-(4-trifluoromethylphenyl)-3-(4-chlorophenyl)-4-(N-methyl-N-cyanoamino)-4,5-dihydro-1H-pyrazole-1-carboxamide

To 4.0 g (10 mmole) of N-(4-trifluoromethylphenyl)-3-(4-chlorophenyl)-4-(N-methylamino)-4,5-dihydro-1H-pyrazole-1-carboxamide (Example 116) in 100 ml of methylene chloride was added 10 ml of 1.0 **M** aqueous NaOH and 1.2 g (11.3 mmole) of cyanogen bromide dissolved in 5 ml of methylene chloride. After stirring overnight, the organic layer was separated, concentrated in vaccuo, and triturated with diethyl ether to yield the title compound, a white solid, mp 216-217°C.

### Example 297 : N-(4-trifluoromethoxyphenyl)-3-(4-hydroxyphenyl)-4-(N-methyl-N-(methoxycarbonyl)-amino)-4,5-dihydro-1H-pyrazole-1-carboxamide

To 25.5 g (50.8 mmole) of N-(4-trifluoromethoxyphenyl)-3-(4-difluoromethoxyphenyl)-4-(N-methyl-N-(methoxycarbonyl)-amino)-4,5-dihydro-1H-pyrazole-1-carboxamide (Example 126) dissolved in 25 g of tetrahydrofuran and 25 g of *t*-butanol was added 10 g of potassium *t*-butoxide. The mixture was refluxed for 1 hour and an additional 3.5 g of potassium *t*-butoxide was added. After refluxing an additional hour the mixture was acidified with acetic acid, concentrated in vacuo, and partitioned between methylene chloride and water. The organic layer was dried over anhydrous magnesium sulfate, filtered, concentrated in vacuo, and chromatographed over silica gel using hexane and diethyl ether to yield the title compound, a white solid, mp 228-233°C.

Example 432 was prepared by substantially the same method using Example 125 as starting material.

### Example 299 : N-(4-trifluoromethoxyphenyl)-3-(4-propoxyphenyl)-4-(N-methyl-N-(methoxycarbonyl)-amino)-4,5-dihydro-1H-pyrazole-1-carboxamide

To 1.0 g (2.2 m mole) of N-(4-trifluoromethoxyphenyl)-3-(4-hydroxyphenyl)-4-(N-methyl-N-(methoxycarbonyl)-amino)-4,5-dihydro-1H-pyrazole-1-carboxamide (Example 297) dissolved in 7.5 ml of dimethylsulfoxide was added 0.35 g (2.8 mmole) of 45% aqueous potassium hydroxide and 1.02 g (6.0 mmole) of 1-iodopropane. The mixture was warmed to 50°C for 30 minutes and diluted with diethyl ether and water. The organic layer was washed with brine, dried over anhydrous magnesium sulfate, filtered, concentrated in vacuo, and chromatographed over silica gel using hexane and diethyl ether to yield the title compound, a white solid, mp 153-155°C.

Examples 298, 300, 379, 395, 400, and 433 were prepared following substantially the same procedure and substituting iodoethane, iodomethane, 2-bromo-1-methoxyethane, or 1-iodobutane for 1-iodopropane on the analogous starting materials.

### Example 349: N-(4-trifluoromethylphenyl)-3-(4-chlorophenyl)-4-(N-propyl-N-(2,2,2-trichloroethycarbonyl)-amino)-4,5,-dihydro-1H-pyrazole-1-carboxamide

### a. 2-(N-propyl-N-(2,2,2-trichloroethoxycarbonyl)-amino)-4′-chloroacetophenone

To 250 ml of methylene chloride was added 180 g (3050 mmole) of 1-aminopropane. The resulting mechanically stirred mixture was cooled to +5°C (internal) and a solution of 117 g (500 mmole) 4′-chloro-2-bromoacetophenone in 250 ml of methylene chloride was added over 6 minutes while strictly maintaining the internal temperature between +5°C and +15°C as a moderate exotherm occured. Toward the end of the addition, the internal temperature was allowed to rise to +20°C. After the addition was complete, the mixture was stirred at +20°C for 9 minutes and then rapidly cooled to -5°C whereon 40 g (500 mmole) of 50% aqueous sodium hydroxide dissolved in 300 ml of water was added. The mixture was transfered to a separatory funnel and the organic layer was separated and thrice washed with 500 ml portions of ice cold water. The organic layer was immediately recooled to -5°C and a solution of 40 g (500 mmole) of 50% aqueous sodium hydroxide and a solution of 100 g (450 mmole) of 2,2,2-trichloroethyl chloroformate in 100 ml of methylene chloride were simultaneously added with rapid stirring while maintaining the internal temperature between -5°C and +5°C. After stirring an additional 15 minutes, the organic layer was separated, washed with brine, dried over anhydrous magnesium sulfate, and concentrated in vacuo at 20 torr. The lower boiling impurities were then removed by distillation at 0.2 torr with the bath temperature rising to 200°C and a head temperature of up to 130°C.
The undistilled pot residue was dissolved in 300 ml of diethyl ether and 300 ml of hexane, filtered through silica gel and concentrated in vacuo to yield 120 g of the desired compound, an oil. An analytical sample was isolated by column chromatography on silica gel eluting with diethyl ether and hexanes.

### b. 2-(N-propyl-N-(2,2,2-trichloroethoxycarbonyl)-amino)-1-(4-chlorophenyl)-prop-2-enone

To 118 g (297 mmole) of 2-(N-propyl-N-(2,2,2-trichloroethoxycarbonyl)-amino)-4′-chloroacetophenone (Example 349a) was added 45 g (555 mmole) of 37% formalin, 150 ml of 1-propanol, 8 g (133 mmole) of acetic acid, and 7 g (83 mmole) of piperidine. The resulting mixture was refluxed and followed by TLC. After 90 minutes it was complete. The resulting mixture was cooled, concentrated in vacuo, poured into 500 ml of diethyl ether, washed twice with 500 ml of water and once with 200 ml of brine. The organic layer was dried over anhydrous magnesium sulfate and concentrated in vacuo yielding 120 g of the desired compound, an amber oil.

### c. 3-(4-chlorophenyl)-4-(N-propyl-N-(2,2,2-trichloroethoxycarbonyl)-amino)-4,5-dihydro-1H-pyrazole

To 120 g (≤ 297 mmole) of 2-(N-propyl-N-(2,2,2-trichloroethoxycarbonyl)-amino)-1-(4-chlorophenyl)-prop-2-enone (Example 349b) was added 7 g (116 mmole) of acetic acid and 200 ml of methanol. To this mixture was added 22 g (440 mmole) of hydrazine monohydrate. An exotherm occured. After refluxing for 10 minutes, the solvent was removed in vacuo and the resulting mixture was dissolved in 200 ml of diethyl ether. The organic layer was washed twice with 100 ml portions of water, washed once with 100 ml of brine, and dried over anhydrous magnesium sulfate. The resulting ether solution contained the desired compound and was used as is in the next reaction.

### d. N-(4-trifluoromethylphenyl)-3-(4-chlorophenyl)-4-(N-propyl-N-(2,2,2-trichloroethoxycarbonyl)-amino)-4,5-dihydro-1H-pyrazole-1-carboxamide

To half of the ether solution of 3-(4-chlorophenyl)-4-(N-propyl-N-(2,2,2-trichloroethoxycarbonyl)-amino)-4,5-dihydro-1H-pyrazole (Example 349c) (≤ 149 mmole) was added 18.7 g (100 mmole) of 4-trifluoromethylphenyl *iso*cyanate. After stirring for 30 minutes, the mixture was concentrated in vacuo and dissolved in 200 ml of 50/50 diethyl ether/hexanes and cooled to -20°C. Crystals of the desired compound formed and were filtered yielding 30 g of the desired compound, mp 172-173°C.

Example 350 was prepared following substantially the same procedure and substituting 4-trifluoromethoxyphenyl *iso*cyanate for 4-trifluoromethyphenyl *iso*cyanate.

### Example 487: N-(4-trifluoromethoxyphenyl)-3-(4-chlorophenyl)-4-(N-methoxycarbonyl-N-(carbomethoxymethyl)-amino)-4,5,-dihydro-1H-pyrazole-1-carboxamide

### a. 2-(N-methoxycarbonyl-N-(carbomethoxymethyl)-amino)-4′-chloroacetophenone

By substantially following the procedure given in Example 585c using 35 g (150 mmole) of 2-bromo-4′-chloroacetophenone, 20.7 g (160 mmole) of glycine methyl ester hydrochloride, 40.6 g (310 mmole) of di*iso*propylethyl amine, and 14.2 g (150 mmole) of methyl chloroformate, 12.5 g of the desired compound, an oil, was obtained.

### b. 2-(N-methoxycarbonyl-N-(carbomethoxymethyl)-amino)-1-(4-chlorophenyl)-prop-2-enone

By substantially following the procedure given in Example 585d using 12 g (40 mmole) of 2-(N-methoxycarbonyl-N-(carbomethoxymethyl)-amino)-4′-chloroacetophenone (Example 487a), 12 g of the desired compound, an oil, was obtained.

### c. 3-(4-chlorophenyl)-4-(N-methoxycarbonyl-N-(carbomethoxymethyl)-amino)-4,5-dihydro-1H-pyrazole

By substantially following the procedure given in Example 585e using 12 g (39 mmole) of 2-(N-methoxycarbonyl-N-(carbomethoxymethyl)-amino)-1-(4-chlorophenyl)-prop-2-enone (Example 487b) a solution of the desired compound, which was not further characterized, was obtained.

### d. N-(4-trifluoromethylphenyl)-3-(4-chlorophenyl)-4-(N-methoxycarbonyl-N-(carbomethoxymethyl)-amino)-4,5-dihydro-1H-pyrazole-1-carboxamide

By substantially following the procedure given in Example 585f using one third (≤ 13 mmole) of the solution of 3-(4-chlorophenyl)-4-(N-methoxycarbonyl-N-(carbomethoxymethyl)-amino)-4,5-dihydro-1H-pyrazole (Example 487c) and 1.87 g (10 mmole) of 4-trifluoromethylphenyl *iso*cyanate, 2.5 g of the desired compound, mp 198-200°C was obtained.

Examples 486, 488 and 489 were prepared following substantially the same procedure and substituting where appropriate for 4-trifluoromethoxyphenyl *iso*cyanate a compound selected from 4-trifluoromethylphenyl *iso*cyanate or 4-tetrafluoroethoxyphenyl *iso*cyanate.

### Example 497: N-(4-trifluoromethylphenyl)-3-(4-propoxyphenyl)-4-carbomethoxymethylamino-4,5-dihydro-1H-pyrazole-1-thiocarboxamide

By substantially following the procedure of Example 568 using methyl chloroformate in Step 568a and 4-trifluoromethylphenyl *iso*thiocyanate in Step 568b, the desired compound, mp 167-169°C, was obtained.

Examples 438 and 439 were prepared following substantially the same procedure, substituting 4-chlorophenyl *iso*thiocyanate for
4-trifluoromethylphenyl *iso*thiocyanate, and substituting
4′-chloroacetophenone for 4′-propoxyacetophenone where appropriate.

### Example 498: S-methyl-N-(4-trifluoromethylphenyl)-3-(4-propoxyphenyl)-4-carbomethoxymethylamino-4,5-dihydro-1H-pyrazole-1-isothiocarboxamide

A solution of 1.0 g (2.0 mmole) of N-(4-trifluoromethylphenyl)-3-(4-propoxyphenyl)-4-carbomethoxymethylamino-4,5-dihydro-1H-pyrazole-1-thiocarboxamide (Example 497), 3.0 ml (50 mmole) of iodomethane, and 5 ml of methylene chloride was allowed to stand at room temperature for 4 days. The mixture was concentrated in vacuo, dissolved in methylene chloride, washed with dilute aqueous sodium hydroxide, dried over anhydrous magnesium sulfate, concentrated in vacuo, and triturated with diethyl ether and hexanes to give the desired compound, an oil.

Examples 536-539 were prepared following substantially the same procedure and substituting where appropriate for iodomethane a compound selected from iodoethane, 1-iodopropane, 2-iodopropane, or benzyl bromide.

### Example 552: N-(4-trifluoromethylphenyl)-3-(4-chlorophenyl)-4-(pyrrolid-2-on-1-yl)--4,5,-dihydro-1H-pyrazole-1-carboxamide

To a slurry of 0.14 g (3.5 mmole) of 60% sodium hydride (hexane washed) in 5 ml of dimethylformamide was added 1.8 g (3.0 mmole) of N-(4-trifluoromethylphenyl)-3-(4-chlorophenyl)-4-((4-bromobutyryl)-amino)-4,5,-dihydro-1H-pyrazole-1-carboxamide (Example 549) dissolved in 10 ml of dimethylformamide. Gas was evolved. After stirring at room temperature for 1 hour, the solvent was removed in vacuo, the resulting mixture is dissolved in methylene chloride, washed with water, dried over anhydrous magnesium sulfate, concentrated in vacuo, and triturated with diethyl ether and hexane to yield 1.3 g of the desired compound, mp 216-217°C.

Examples 546 and 558 were prepared following substantially the same procedure and starting with Examples 543 and 555 respectively.

Examples 553, 547 and 559 were prepared following substantially the same procedure and starting with Examples 550, 544 and 556 respectively.

Examples 554, 548 and 560 were prepared following substantially the same procedure and starting with Examples 551, 545 and 557 respectively.

### Example 568: N-(4-trifluoromethylphenyl)-3-(4-propoxyphenyl)-4-(N-methyl-N-(2,2,2-trichloroethoxycarbonyl)-amino-4,5,-dihydro-1H-pyrazole-1-carboxamide

### a. 2-(N-methyl-N-(2,2,2-trichloroethoxycarbonyl)-amino)-4′-propoxyacetophenone

By substantially following the proceedure of Example 349a using 128 g (500 mmole) of 2-bromo-4′-propoxyacetophenone (Example 585b) 150 g of the desired compound, an oil, was obtained.

### b. N-(4-trifluoromethylphenyl)-3-(4-propoxyphenyl)-4-(N-methyl-N-(2,2,2-trichloroethoxycarbonyl)-amino--4,5,-dihydro-1H-pyrazole-1-carboxamide

By substantially following the proceedures of Example 585d using 150 g (390 mmole) of 2-(N-methyl-N-(2,2,2-trichloroethoxycarbonyl)-amino)-4′-propoxyacetophenone and following with the proceedures of Example 585e and Example 585f, 50 g of the desired compound, mp 141-143°C, was obtained.

Examples 415-418, 471-472, 477, 499-500, 509-511, 513-515, 634, and 659-660 were prepared following substantially the same procedure, substituting where appropriate for 4-trifluoromethylphenyl *iso*cyanate a compound selected from 4-trifluoromethoxyphenyl *iso*cyanate,4-tetrafluoroethoxyphenyl *iso*cyanate, 4-fluorophenyl *iso*cyanate, 4-chlorophenyl *iso*cyanate, or 4-bromophenyl *iso*cyanate, substituting 4′-butoxyacetophenone for 4′-propoxyacetophenone where appropriate, substituting ethylamine for methylamine where appropriate, and substituting methyl chloroformate for 2,2,2-trichloroethyl chloroformate where appropriate.

### Example 570: N-(4-trifluoromethylphenyl)-3-(4-propoxyphenyl)-4-(N-ethyl-N-methylamino)-4,5,-dihydro-1H-pyrazole-1-carboxamide

To 1.5 g (3.6 mmole) of N-(4-trifluoromethylphenyl)-3-(4-propoxyphenyl)-4-methylamino-4,5,-dihydro-1H-pyrazole-1-carboxamide (Example 434) dissolved in 10 ml of methanol and 5 ml of tetrahydrofuran was added 0.15 g (2.4 mmole) of sodium cyanoborohydride and 0.2 g (4.3 mmole) of acetaldehyde. To this stirred solution was dropwise added 0.6 g (9.5 mmole) of acetic acid dissolved in 3 ml of methanol. After 30 minutes, the reaction was concentrated in vacuo, dissolved in 50 ml of diethyl ether, washed with water, washed with 1 **M** aqueous sodium hydroxide, washed with brine, dried over anhydrous magnesium sulfate, and concentrated in vacuo. Trituration with diethyl ether and hexanes yielded 1.4 g of the desired compound, mp 130-132°C.

Examples 569, 571-576, 674-677, 715-726, and 739-742 were prepared following substantially the same procedure, substituting where appropriate for 4-trifluoromethylphenyl *iso*cyanate a compound selected from 4-trifluoromethoxyphenyl *iso*cyanate or
4-tetrafluoroethoxyphenyl *iso*cyanate, substituting where appropriate for 4′-propoxyacetophenone a compound selected from
4′-butoxyacetophenone, 4′-difluoromethoxyacetophenone,
4′-difluoromethoxy-3′-methoxyacetophenone, or
4′-difluoromethoxy-3′-methylacetophenone, substituting ethylamine or propylamine for methylamine where appropriate, substituting where appropriate for acetaldehyde a compound selected from formalin, propionaldehyde, butryaldehyde, *iso*butyraldehyde, pentanal, hexanal, or benzaldehyde.

### Example 585: N-(4-trifluoromethylphenyl)-3-(4-propoxyphenyl)-4-(N-propyl-N-(2,2,2-trichloroethoxycarbonyl)-amino)-4,5,-dihydro-1H-pyrazole-1-carboxamide

### a: 4′-propoxyacetophenone

To 500 g (3670 mmole) of 4′-hydroxyacetophenone dissolved in 1000 ml of ethanol was added 450 g (3620 mmole) of 45% aqueous potassium hydroxide. To this mixture was added 650 g (3820 mmole) of 1-iodopropane. The resulting mixture was refluxed for 2 hours, cooled, concentrated in vacuo, and the resulting mixture was dissolved in 1000 ml of diethyl ether and 1000 ml of water. The organic layer was separated, washed with brine adjusted to pH 13 with aqueous sodium hydroxide, dried over anhydrous magnesium sulfate, concentrated in vacuo, and distilled yielding 640 g of the desired compound, bp 110°C at 0.2 torr.

### b. 2-bromo-4′-propoxyacetophenone

To 133 g (750 mmole) of 4′-propoxyacetophenone dissolved in 500 ml of diethyl ether and heated to reflux was dropwise added 120 g (750 mmole) of bromine at a rate to maintain reflux and yet allow decolorization of the red color. The resulting organic solution was twice washed with 500 ml of water, washed once with 200 ml of brine, dried over anhydrous magnesium sulfate, and concentrated in vacuo. This mixture, upon analysis via gas chromatography was approximately 10% unbrominated starting material, 80% the desired monobrominated product, and 10% dibrominated compound. It was used as is in the next reaction. It could be crystallized from diethyl ether and hexane to yield pure monobrominated compound, a solid, mp 36-38°C.

### c. 2-(N-propyl-N-(2,2,2-trichloroethoxycarbonyl)-amino)-4′-propoxyacetophenone

To 250 ml of methylene chloride was added 44 g (750 mmole) of 1-aminopropane and 107 g (830 mmole) of N,N-di*iso*propylethylamine. The resulting mechanically stirred mixture was cooled to +5°C (internal) and a solution of 2-bromo-4′-propoxyacetophenone (Example 585b) (≤750 mmole) in 250 ml of methylene chloride was added over 6 minutes while strictly maintaining the internal temperature between +5°C and +15°C as a moderate exotherm occured. Toward the end of the addition, the internal temperature was allowed to rise to +20°C. After the addition was complete, the mixture was stirred at +20°C for 9 minutes and then rapidly cooled to -5°C whereon a total of 120 g (1500 mmole) of 50% aqueous sodium hydroxide dissolved in 500 ml of water and 159 g (750 mmole) of 2,2,2-trichloroethyl chloroformate were added in alternate aliquots, roughly one eighth of the total for each aliquot, to the stirred reaction mixture. The internal temperature was maintained at between -5°C and +5°C during the additions. After stirring an additional 15 minutes, the organic layer was separated, washed with brine, dried over anhydrous magnesium sulfate, and concentrated in vacuo at 20 torr. The lower boiling impurities were then removed by distillation at 0.2 torr with the bath temperature rising to 200°C and head temperature up to 130°C. The undistilled pot residue was dissolved in 300 ml of diethyl ether and 300 ml of hexane, filtered through silica gel and concentrated in vacuo to yield 123 g of the desired compound, an oil. An analytical sample was isolated by column chromatography on silica gel eluting with diethyl ether and hexanes.

### d. 2-(N-propyl-N-(2,2,2-trichloroethoxycarbonyl)-amino)-1-(4-propoxyphenyl)-prop-2-enone

To 50 g (120 mmole) of 2-(N-propyl-N-(2,2,2-trichloroethoxycarbonyl)-amino)-4′-propoxyacetophenone (Example 585c) was added 20 g (240 mmole) of 37% formalin, 20 g of 2-methoxyethanol, 9 g (150 mmole) of acetic acid, and 8.5 g (100 mmole) of piperidine. The resulting mixture was refluxed and followed by TLC. After 1 hour it was complete. The resulting mixture was cooled, poured into 500 ml of diethyl ether, washed twice with 500 ml of water and once with 200 ml of brine. The organic layer was dried over anhydrous magnesium sulfate and concentrated in vacuo yielding 45 g of the desired compound, an amber oil.

### e. 3-(4-propoxyphenyl)-4-(N-propyl-N-(2,2,2-trichloroethoxycarbonyl)-amino)-4,5-dihydro-1H-pyrazole

To 36 g (85 mmole) of 2-(N-propyl-N-(2,2,2-trichloroethoxycarbonyl)-amino)-1-(4-propoxyphenyl)-prop-2-enone (Example 585d) was added 1.5 g (25 mmole) of acetic acid and 100 ml of methanol. To this mixture was added 8.5 g (170 mmole) of hydrazine monohydrate. A mild exotherm occured. After refluxing for 10 minutes, the solvent was removed in vacuo and the resulting mixture was dissolved in 200 ml of diethyl ether. The organic layer was washed twice with 100 ml portions of water, washed once with 100 ml of brine, and dried over anhydrous magnesium sulfate. The resulting ether solution contained the desired compound and was used as is in the next reaction.

### f. N-(4-trifluoromethylphenyl)-3-(4-propoxyphenyl)-4-(N-propyl-N-(2,2,2-trichloroethoxycarbonyl)-amino)-4,5-dihydro-1H-pyrazole-1-carboxamide

To an ether solution of 3-(4-propoxyphenyl)-4-(N-propyl-N-(2,2,2-trichloroethoxycarbonyl)-amino)-4,5-dihydro-1H-pyrazole (Example 585e) (≤ 85 mmole) was added 12.7 g (68 mmole) of 4-trifluoromethylphenyl isocyanate. After stirring for 30 minutes, the mixture was concentrated in vacuo and dissolved in 200 ml of 50/50 diethyl ether/hexanes and cooled to -20°C. Crystals of the desired compound formed and were filtered yielding 23 g of the desired compound, mp 162-163°C.

Examples 370, 612, 629, 688-690, and 727-729 were prepared following substantially the same procedure, substituting where appropriate for 4-trifluoromethylphenyl *iso*cyanate a compound selected from 4-trifluoromethoxyphenyl *iso*cyanate, or
4-tetrafluoroethoxyphenyl *iso*cyanate, substituting where appropriate for 4′-propoxyacetophenone a compound selected from
4′-chloroacetophenone, 4′-difluoromethoxyacetophenone,
4′-difluoromethoxy-3′-methoxyacetophenone, or
4′-difluoromethoxy-3′-methylacetophenone and substituting where appropriate for methylamine a compound selected from ethylamine, propylamine, or butylamine.

### Example 586: N-(4-trifluoromethylphenyl)-3-(4-propoxyphenyl)-4-(N-propylamino)-4,5,-dihydro-1H-pyrazole-1-carboxamide

To 22 g (35 mmole) of N-(4-trifluoromethylphenyl)-3-(4-propoxyphenyl)-4-(N-propyl-N-(2,2,2-trichloroethoxycarbonyl)-amino)-4,5,-dihydro-1H-pyrazole-1-carboxamide (Example 585) was added 100 ml of tetrahydrofuran, 100 ml of methanol, 4.6 g (70 mmole) of zinc dust, and finally 6.4 g (106 mmole) of acetic acid. After stirring for 2 hours, the starting material had been consumed as shown by TLC. The mixture was filtered from unreacted zinc, concentrated in vacuo, dissolved in 200 ml of diethyl ether, washed twice with 100 ml of water, washed once with 100 ml of dilute aqueous sodium hydroxide and washed once with brine. The resulting ether solution was dried over anhydrous magnesium sulfate, concentrated in vacuo and chromatographed over silica gel using hexanes, diethyl ether, and ethyl acetate yielding 1 g of N-(4-trifluoromethylphenyl)-3-(4-propoxyphenyl)-4-(N-propyl-N-(2,2-dichloroethoxycarbonyl)-amino)-4,5,-dihydro-1H-pyrazole-1-carboxamide and 12 g of the desired compound, mp 130-133°C.

Examples 380, 478, 512, 527, 540, 613, 630, 635, 647, 661-662, 691-693, and 730-731 were prepared following substantially the same procedure, substituting where appropriate for 4-trifluoromethylphenyl *iso*cyanate a compound selected from 4-trifluoromethoxyphenyl *iso*cyanate, or
4-tetrafluoroethoxyphenyl *iso*cyanate, substituting where appropriate for 4′-propoxyacetophenone a compound selected from
4′-chloroacetophenone, 4′-butoxyacetophenone,
4′-difluoromethoxyacetophenone,
4′-difluoromethoxy-3′-methoxyacetophenone, or
4′-difluoromethoxy-3′-methylacetophenone and substituting where appropriate for propylamine a compound selected from ethylamine, methylamine, or butylamine.

Examples 419 and 434 were prepared by substantially following the same procedure and starting with Examples 349 and 568 respectively.

Examples 351, 448 and 461 were prepared following substantially the same procedure as for Example 419 and substituting where appropriate for 4-trifluoromethylphenyl *iso*cyanate a compound selected from 4-trifluoromettioxyphenyl *iso*cyanate or 4-tetrafluoroethoxyphenyl *iso*cyanate and substituting where appropriate for *n*-propylamine a compound selected from methylamine, ethylamine, or *n*-butylamine.

### Example 587: N-(4-trifluoromethylphenyl)-3-(4-propoxyphenyl)-4-(N-formyl-N-propylamino)-4,5,-dihydro-1H-pyrazole-1-carboxamide

To 3.0 g (6.7 mmole) of N-(4-trifluoromethylphenyl)-3-(4-propoxyphenyl)-4-(N-propylamino)-4,5,-dihydro-1H-pyrazole-1-carboxamide (Example 586) dissolved in 25 ml of ethyl acetate and cooled to 0°C, was added 2.4 g (30 mmole) of pyridine and 4.0 g (28 mmole) of formic acetic anhydride mixture (Example 621 a). After 30 minutes, the reaction mixture was washed with water and brine, concentrated in vacuo, and chromatographed over silica gel using hexanes and ethyl acetate to yield 2.5 g of the desired compound, mp 161-163°C.

Examples 520, 532, 563, 614, 631, 636, 648, 663, 694, 701, and 708, 732 were prepared following substantially the same procedure, substituting where appropriate for 4-trifluoromethylphenyl *iso*cyanate a compound selected from 4-trifluoromethoxyphenyl *iso*cyanate, or
4-tetrafluoroethoxyphenyl *iso*cyanate, substituting where appropriate for 4′-propoxyacetophenone a compound selected from
4′-chloroacetophenone, 4′-butoxyacetophenone,
4′-difluoromethoxyacetophenone,
4′-difluoromethoxy-3′-methoxyacetophenone, or
4′-difluoromethoxy-3′-methylacetophenone and substituting where appropriate for propylamine a compound selected from ethylamine, methylamine, or butylamine.

Example 423 was prepared by substantially following the same procedure and starting with Example 419.

Examples 355, 384, 451 and 465 were prepared following substantially the same procedure as for Example 423, substituting where appropriate for 4-trifluoromethylphenyl *iso*cyanate a compound selected from 4-trifluoromethoxyphenyl *iso*cyanate or 4-tetrafluoroethoxyphenyl *iso*cyanate and substituting where appropriate for *n*-propylamine a compound selected from methylamine, ethylamine, or *n*-butylamine.

### Example 590: N-(4-trifluoromethylphenyl)-3-(4-propoxyphenyl)-4-(N-propionyl-N-propylamino)-4,5,-dihydro-1H-pyrazole-1-carboxamide

To 2 g (4.5 mmole) of N-(4-trifluoromethylphenyl)-3-(4-propoxyphenyl)-4-(N-propylamino)-4,5,-dihydro-1H-pyrazole-1-carboxamide Example (586) dissolved in 25 ml of ethyl acetate and cooled to -5°C, was added 0.8 g (9 mmole) of pyridine and, finally, 0.8 g (9 mmole) of propionyl chloride was added. After 30 minutes, the reaction mixture was washed with water and brine, concentrated in vacuo, and chromatographed over silica gel using hexanes and ethyl acetate to yield 0.5 g of the desired compound, mp 152-154°C.

Examples 356-361, 366-369, 375-378, 385-390, 424-429, 430, 436-437, 452-456, 466-470, 474-476, 480-482, 516-517, 521-522, 528-529, 533-534, 564-567, 588-589, 591, 615-618, 637-640, 649-652, 664-667, 695-698, 702-705, 709-712, and 733-736 were prepared following substantially the same procedure, substituting where appropriate for 4-trifluoromethylphenyl *iso*cyanate a compound selected from 4-trifluoromethoxyphenyl *iso*cyanate, or 4-tetrafluoroethoxyphenyl *iso*cyanate, substituting where appropriate for 4′-propoxyacetophenone a compound selected from
4′-chloroacetophenone, 4′-butoxyacetophenone,
4′-difluoromethoxyacetophenone, or
4′-difluoromethoxy-3′-methylacetophenone, substituting where appropriate for propylamine a compound selected from ethylamine, methylamine, or butylamine, and substituting where appropriate for propionyl chloride a compound selected from propionic anhydride, acetic anhydride, acetyl chloride, butyryl chloride, *iso*butyryl chloride, trifluoroacetic anhydride, methane sulfonyl chloride, valeryl chloride, 2-methylbutyryl chloride, 3-methylbutyryl chloride, or hexanoyl chloride.

### Example 592: N-(4-trifluoromethylphenyl)-3-(4-propoxyphenyl)-4-(N-methoxycarbonyl-N-propylamino)-4,5,-dihydro-1H-pyrazole-1-carboxamide

To 1.5 g (3.3 mmole) of N-(4-trifluoromethylphenyl)-3-(4-propoxyphenyl)-4-(N-propylamino)-4,5,-dihydro-1H-pyrazole-1-carboxamide Example (586) dissolved in 25 ml of ethyl acetate and cooled to -5°C, was added 1.2 g (15 mmole) of pyridine and, finally, 1.3 g (13 mmole) of methyl chloroformate in 10 ml of ethyl acetate was added over 30 minutes. After an additional 30 minutes, the reaction mixture was washed with water and brine, concentrated in vacuo, and chromatographed over silica gel using hexanes and ethyl acetate to yield 0.7 g of the desired compound, mp 142-145°C.

Examples 352-354, 381-383, 420-422, 435, 462-464, 473, 479, 518-519, 530-531, 561-562, 593, 619-620, 632-633, 641-642, 653-654, 668-669, 699-700, 706-707, 713-714, and 738-739 were prepared following substantially the same procedure, substituting where appropriate for
4-trifluoromethylphenyl *iso*cyanate a compound selected from
4-trifluoromethoxyphenyl *iso*cyanate, or 4-tetrafluoroethoxyphenyl *iso*cyanate, substituting where appropriate for 4′-propoxyacetophenone a compound from 4′-chloroacetophenone, 4′-butoxyacetophenone,
4′-difluoromethoxyacetophenone,
4′-difluoromethoxy-3′-methoxyacetophenone, or
4′-difluoromethoxy-3′-methylacetophenone, substituting where appropriate for propylamine a compound selected from ethylamine, methylamine, or butylamine and substituting where appropriate for methyl chloroformate a compound selected from ethyl chloroformate, propyl chloroformate, or *iso*propyl chloroformate.

### Example 608: N-(4-trifluoromethylphenyl)-3-(4-propoxyphenyl)-4-(pyrid-2-one-1-yl)-4,5,-dihydro-1H-pyrazole-1-carboxamide

### a. 2-(pyrid-2-on-1-yl)-4′-propoxyacetophenone

To 50 g (≤ 200 mmole) of 2-bromo-4′-propoxyacetophenone (Example 585b) was added 150 g of tetrahydrofuran, 19.5 g (205 mmole) of 2-hydroxypyridine, and 30 g (233 mmole) of di*iso*propylethylamine. The reaction mixture was refluxed for 4 hours, concentrated in vacuo, dissolved in 1000 ml of warm ethyl acetate, washed with 300 ml of warm water, washed with brine, dried over anhydrous magnesium sulfate, and concentrated in vacuo. Trituration with diethyl ether and hexanes gave 33.9 g of the desired compound, mp 120-123°C.

### b. 2-(pyrid-2-on-1-yl)-1-(4-propoxyphenyl)-prop-2-enone

To 13.4 g (50 mmole) of 2-(pyrid-2-on-1-yl)-4′-propoxyacetophenone (Example 608a) in 25 ml of dioxane was added 5.0 g (61 mmole) of 37% formalin, 1.0 g (17 mmole) of acetic acid, and 1.0 g (12 mmole) of piperidine. The mixture was refluxed for 4 hours, concentrated in vacuo, dissolved in 200 ml of diethyl ether, washed with water, washed with brine, dried over anhydrous magnesium sulfate, and concentrated in vacuo. Trituration with diethyl ether and hexanes gave 13 g of the desired compound, mp 37-40°C.

### c. N-(4-trifluoromethylphenyl)-3-(4-propoxyphenyl)-4-(pyrid-2-one-1-yl)-4,5,-dihydro-1H-pyrazole-1-carboxamide

By substantially following the proceedures of Example 585e and Example 585f, using 19 g (67 mmole) of 2-(pyrid-2-on-1-yl)-1-(4-propoxyphenyl)-prop-2-enone (Example 608b) one obtains 3.1 g of the desired compound, mp 183-185°C.

Examples 609-611 and 678-680 were prepared following substantially the same procedure, substituting where appropriate for
4-trifluoromethylphenyl *iso*cyanate a compound selected from
4-trifluoromethoxyphenyl *iso*cyanate, or 4-tetrafluoroethoxyphenyl *iso*cyanate and substituting where appropriate 4-hydroxypyridine for 2-hydroxypyridine.

### Example 621: N-(4-trifluoromethylphenyl)-3-(4-propoxyphenyl)-4-(N-formyl-N-(2-methoxyethyl)-amino)-4,5,-dihydro-1H-pyrazole-1-carboxamide

### a. formic acetic anhydride mixture

To 33 ml (350 mmole) of acetic anhydride cooled to 0°C, was added 16 ml (420 mmole) of formic acid while maintaining the internal temperature below +10°C. The mixture was placed in a preheated water bath and maintained at 50°C for 16 minutes. The mixture was then rapidly cooled to 0°C and used as is. It contains approximately 7 mmole of formic acetic anhydride per gram.

### b. 2-(N-formyl-N-(2-methoxyethyl)-amino)-4′-propoxyacetophenone

By substantially following the proceedure of Example 585c using 37 g (≤ 140 mmole) of 2-bromo-4′-propoxyacetophenone (Example 585b), 16 g (210 mmole) of 2-methoxyethylamine, 22 g (170 mmole) of di*iso*propylethylamine, and 50 g of formic acetic anhydride mixture (≤ 350 mmole) 18.5 g of the desired compound, an oil, was obtained.

### c. 2-(N-formyl-N-(2-methoxyethyl)-amino)-1-(4-propoxyphenyl)-prop-2-enone

By substantially following the proceedures of Example 585d using 18 g (60 mmole) of 2-(N-formyl-N-(2-methoxyethyl)-amino)-4′-propoxyacetophenone (Example 621b) 15.2 g of the desired compound, an oil, was obtained.

### d. 3-(4-propoxyphenyl)-4-(N-formyl-N-(2-methoxyethyl)-amino)-4,5-dihydro-1H-pyrazole

By substantially following the proceedures of Example 585e using 14.7 g (50 mmole) of 2-(N-formyl-N-(2-methoxyethyl)-amino)-1-(4-propoxyphenyl)-prop-2-enone (Example 621c) a diethyl ether solution of the desired compound, which was not further characterized, was obtained.

### e. N-(4-trifluoromethylphenyl)-3-(4-propoxyphenyl)-4-(N-formyl-N-(2-methoxyethyl)-amino)-4,5,-dihydro-1H-pyrazole-1-carboxamide

By substantially following the proceedures of Example 585f using one quarter of the solution prepared in Example 621d (≤ 12 mmole) the desired compound, mp 159-160°C, was obtained.

Examples 483-485, 577-584, 594-601, 622-628, 643-646, 655-658, 670-673, and 684-687 were prepared following substantially the same procedure, substituting where appropriate for 4-trifluoromethylphenyl *iso*cyanate a compound selected 4-trifluoromethoxyphenyl *iso*cyanate,
4-chlorophenyl *iso*cyanate, or 4-tetrafluoroethoxyphenyl *iso*cyanate, substituting where appropriate for 4′-propoxyacetophenone a compound selected from 4′-chloroacetophenone,
4′-butoxyacetophenone, 4′-difluoromethoxyacetophenone,
4′-difluoromethoxy-3′-methoxyacetophenone, or
4′-difluoromethoxy-3′-methylacetophenone, substituting where appropriate for 2-methoxyethylamine a compound selected from methylamine, ethylamine, propylamine, *iso*propylamine, *iso*butylamine, allylamine, propargylamine, or 2-cyanoethylamine and substituting where appropriate methyl chloroformate for formic acetic anhydride mixture.

In addition, other examples of this invention can be prepared following substantially the same procedure and substituting for formic acetic anhydride mixture a compound selected from methyl chloroformate, ethyl chloroformate, propyl chloroformate, *iso*propyl chloroformate, 2,2,2-trichloroethyl chloroformate, di-*t*-butyl dicarbonate, acetic anhydride, acetyl chloride, propionyl chloride, propionic anhydride, butyryl chloride, *iso*butyryl chloride, trifluoroacetic anhydride, methane sulfonyl chloride, valeryl chloride, 2-methylbutyryl chloride, 3-methylbutyryl chloride, or hexanoyl chloride.

### Example 681: N-(4-trifluoromethylphenyl)-3-(4-propoxyphenyl)-4-(1,2,4-triazol-1-yl)-4,5,-dihydro-1H-pyrazole-1-carboxamide

### a. 2-(1,2,4-triazol-1-yl)-4′-propoxyacetophenone

By substantially following the proceedure of Example 608a using 29 g of 2-bromo-4′-propoxyacetophenone (Example 585b), 10 g of 1,2,4-triazole, 15 g (116 mmole) of di*iso*propylethylamine, and 100 ml of tetrahydrofuran, 8.4 g of the desired compound, mp 97-99°C, was obtained.

### b. 2-(1,2,4-triazol-1-yl)-1-(4-propoxyphenyl)-prop-2-enone

To 7.5 g (30 mmole) of 2-(1,2,4-triazol-1-yl)-4′-propoxyacetophenone (Example 681a) in 30 ml of ethanol was added 3.0 g (37 mmole) of 37% formalin, 0.6 g (10 mmole) of acetic acid, and 0.6 g (7 mmole) of piperidine. The mixture was refluxed for 30 minutes, concentrated in vacuo, dissolved in 100 ml of diethyl ether, washed with water, washed with brine, dried over anhydrous magnesium sulfate, and concentrated in vacuo. Trituration with diethyl ether and hexanes gave 7 g of the desired compound, an oil.

### c. N-(4-trifluoromethylphenyl)-3-(4-propoxyphenyl)-4-(1,2,4-triazol-1-yl)-4,5,-dihydro-1H-pyrazole-1-carboxamide

By substantially following the proceedures of Example 585e and Example 585f, using 7 g (67 mmole) of 2-(1,2,4-triazol-1-yl)-1-(4-propoxyphenyl)-prop-2-enone (Example 681b) 1.3 g of the desired compound, mp 225-228°C, was obtained.

Examples 682 and 683 were made by substantially the same proceedure, substituting where appropriate for
4-trifluoromethylphenyl *iso*cyanate a compound selected from
4-trifluoromethoxyphenyl *iso*cyanate or 4-tetrafluoroethoxyphenyl *iso*cyanate.

On the basis of their strong initial pesticidal activity and excellent residual pesticidal activity, compounds according to the invention may be used in low dosages in controlling pests. The amount of dosage depends on a variety of factors, for example, the substance used, the kind of pest, the formulation used, the state of the crop infested with the pest and the prevailing weather conditions. In general, for the control of pests in agriculture and horticulture, a dosage corresponding to from about 0.1 grams to about 1000 grams of the active substance per hectare may be used and from about 5 grams to about 200 grams per hectare of the active substance is preferred. The exact amount of dosage for a given situation can be routinely determined and depends on a variety of factors, for example, the substance used, the kind of pest, the formulation used, the state of the crop infested with the insect and the prevailing weather conditions.

The term "pesticidal" as employed in the specification and claims of this application is to be construed as any means which adversely affects the existence or growth of the target pest. Such means can compromise a complete killing action, eradication, arresting in growth, inhibition, reducing in number of any combination thereof. The term "control" as employed in the specification and claims of this application is to be construed as meaning "pesticidal" and protecting plants from pest damage. By "pesticidally effective amount" is meant that dosage of active substance sufficient to exert the desired pest "control".

Representative pests which can be controlled by the compounds of the present invention include:
American Cockroach (*Periplaneta americana*)
Bean Leaf Beetle (*Cerotoma trifurcata*)
Bean Leaf Roller (*Urbanus proteus*)
Black Carpenter Ant (*Camponotus pennsylvanicus*)
Black Cutworm (*Agrotis ipsilon*)
Boll Weevil (*Anthonomus grandis grandis*)
Colorado Potato Beetle (*Leptinotarsa decemlineata*)
Fall Armyworm (*Spodoptera frugiperda*)
German Cockroach (*Blattella germanica*)
Green June Beetle (*Cotinis nitida*)
House Cricket (*Acheta domesticus*)
Housefly (*Musca domestica*)
Mexican Bean Beetle (*Epilachna varivestis*)
Potato Leaf Hopper (*Empoasca fabae*)
Red Harvester Ant (*Pogonomyrmex barbatus*)
Red Imported Fire Ant (*Solenopsis invicta*)
Redlegged Grasshopper (*Melanopus femurrubrum*)
Southern Armyworm (*Spodoptera eridania*)
Southern Corn Rootworm (*Diabrotica undecimpunctata howardi*)
Tobacco Budworm (*Heliothis virescens*)

The compounds of the present invention can be used in the form of compositions or formulations. Examples of the preparation of compositions and formulations can be found in the American Chemical Society publication "Pesticidal Formulation Research," (1969), Advances in Chemistry Series No. 86, written by Wade Van Valkenburg; and the Marcel Dekker, Inc. publication "Pesticide Formulations", (1973) edited by Wade Van Valkenburg. In these compositions and formulations, the active substance is mixed with conventional inert agronomically acceptable (i.e., plant compatible and/or pesticidally inert) pesticide diluents or extenders such as solid carrier material or liquid carrier material, of the type usable in conventional pesticide compositions or formulations. By "agronomically acceptable carrier is meant any substance which can be used to dissolve, disperse or diffuse the active ingredient in the composition without impairing the active ingredients effectiveness and which by itself has no significant detrimental effect on the soil, equipment, desirable plants, or agronomic enviornment. If desired, adjuvants such as surfactants, stabilizers, antifoam agents and antidrift agents may also be combined.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles. Wettable powders, pastes, flowables and emulsifiable concentrates are concentrated preparations which are diluted with water before or during use. Baits are preparations generally comprising a food or other substance attractive to insects, that includes at least one compound of the instant invention. The invert emulsions are mainly used for air application, where large areas are treated with a comparatively small amount of preparation and may be prepared in the spraying apparatus shortly before, or even during, the spraying operation by emulsifying water in an oil solution or an oil dispersion of the active substance.

Compositions and formulations are prepared in a known manner, for instance by extending the active compounds with conventional pesticide dispersible liquid diluent carriers and/or dispersible solid carriers optionally with the use of carrier vehicle assistants such as conventional pesticide surface-active agents, including emulsifying agents and/or dispersing agents, for example, when water is used as diluent, organic solvents may be added as auxiliary solvents.

The active compounds of the present invention may be employed alone or in the form of mixtures with one another and/or with such solid and/or liquid dispersible carrier vehicles and/or with other known compatible active agents, especially plant protection agents, such as other insecticides, arthropodicides, nematicides, fungicides, bactericides, rodenticides, herbicides, fertilizers, growth-regulating agents, synergists.

In the compositions of the invention, the active compound is present in an amount substantially between about 0.0001-99% by weight. For compositions suitable for storage or transportation, the amount of active ingredient is preferably between about 0.5-90% by weight, and more preferably between about 1-75% by weight of the mixture. Compositions suitable for direct application or field application generally contain the active compound in an amount substantially between about 0.0001-95%, preferably between about 0.001-90% by weight, and more preferably between about 0.01-75% by weight of the mixture.

The active compounds can be applied as insecticide sprays by methods commonly employed, such as conventional high-gallonage hydraulic sprays, low gallonage sprays, ultra-low-volume sprays, airblast spray, aerial sprays, and dusts.

The present invention also contemplates methods of killing, combatting or controlling pests which compromises contacting pests with a combative or toxic amount (i.e. a pesticidally effective amount) of at least one active compound of the invention alone or together with a carrier vehicle (composition or formulation) as noted above. The term "contacting" as employed in the specification and claims means applying to at least one of (a) such pests and (b) the corresponding habit at thereof (i.e., the locus to be protected, for example, to a growing crop or to an area where a crop is to be grown) the active compound of this invention alone or as a constituent of a composition or formulation.

In addition to the aforementioned ingredients the preparations according to the invention may also contain other substances commonly used in preparations of this kind. For example, a lubricant, such as calcium stearate or magnesium stearate, may be added to a wettable powder or to a mixture to be granulated. Furthermore there may, for example, be added "adhesives" such as polyvinylalcohol-cellulose derivatives or other colloidal materials, such as casein, to improve the adherence of the pesticide to the surface to be protected.

Compositions and formulations according to the present invention may also include known pesticidal compounds. This expands the spectrum of activity of the preparation and may give rise to synergism.

The following known insecticidal, fungicidal and acaricidal compounds are suitable for use in such a combined preparation.
Insecticides such as:
acephate, acethion, acetoxon, aldicarb, aldoxycarb, aldrin, allethrin, allyxycarb, alpha-cypermethrin, amidithion, amitraz, amlure, anethol, azethion, azinphos-ethyl, azinphos-methyl, azocyclotin, bacillus thuringiensis, BCPE, bendiocarb, bensultap, benzoximate, benzyl acetate, benzyl benzoate, BHC, bifenthrin, binapacryl, bomyl, BPMC, bromophos, bromophos-ethyl, bromopropylate, bufencarb, buprofezin, butacarb, butocarboxim, butonate, butoxycarboxim, calcium arsenate, carbaryl, carbofuran, carbophenothion, carbosulfan, cartap, chlordane, chlordecone, chlordimeform, chlorfenethol, chlorfenson, chlorfensulphide, chlorfenvinphos, chlormephos, chlorobenzilate, chloropropylate, chlorphoxim, chlorpyrifos, chlorpyrifos methyl, chlorthiophos, clofentezine, CPCBS, CPMC, crotoxyphos, crufomate, cryolite, cufraneb, cyanofenphos, cyanophos, cyanthoate, cyfluthrin, cyhexatin, cypermethrin, cyphenothrin, cyromazine, DAEP, DDT, DDVP, deltamethrin, demeton, demeton-S-methyl, demeton-O-methyl, demeton-S, demeton-S-methyl sulfoxid, demephion-O, demephion-S, dialifor, diazinon, dicapthon, dichlofenthion, dicofol, dicrotophos, dieldrin, dienochlor, diflubenzuron, dihydrorotenone, dimefox, dimetan, dimethoate, dimethrin, dinex, dinitrophenol, dinobuton, dinocap, dioxabenzofos, dioxacarb, dioxathion, disparlure, disulfoton, DMCP, DNOC, d-trans allethrin, endosulfan, endothion, endrin, entice, EPBP, EPN, esfenvalerate, ethiofencarb, ethion, ethoate-methyl, ethoprop, etrimfos, fenamiphos, fenazaflor, fenbutatin-oxide, fenitrothion, fenoxycarb, fenpropathrin, fenson, fensulfothion, fenthion, fenvalerate, flubenzimine, flucythrinate, fluenethyl, flufenoxuron, fluvalinate, fonofos, formetanate hydrochloride, formothion, fosmethilan, fosthietan, furathiocarb, furethrin, grandlure, heptachlor, HETP, hexythiazox, hydramethylnon, hydroprene, IPSP, isazophos, isobenzan, isofenphos, isoprocarb, isoprothiolane, isothioate, isoxathion, jodfenphos, kinoprene, lead arsenate, leptophos, lethane, lindane, lythidathion, malathion, mazidox, mecarbam, mecarphon, menazon, mephosfolan, methamidophos, methidathion, methiocarb, methomyl, methoprene, methoxychlor, methyl parathion, methyl phencapton, mevinphos, mexacarbate, MIPC, mirex, monocrotophos, MTMC, naled, nicotine, nonachlor, omethoate, ovex, oxamyl, oxydeprofs, oxydisulfoton, oxythioquinox, paraoxon, parathion, paris green, permethrin, perthane, phencapton, phenthoate, phorate, phosalone, phosfolan, phosmet, phosnichlor, phosphamidon, phoxim, pirimicarb, pirimiphos-ethyl, pirimiphos-methyl, plifenate, profenofos, promecarb, propargite, propetamphos, propoxur, prothidathion, prothiophos, prothoate, PTMD, pyridaben, pyridaphenthion, quinalphos, resmethrin, ronnell, rotenone, ryania, s-bioallethrin, salithion, schradan, sodium fluosilicate, sophamide, sulfotepp, sulprofos, tefluthrin, temephos, TEPP, terbufos, tetrachlorvinphos, tetradifon, tetramethrin, tetrasul, thallium sulfate, thiocarboxime, thiocyclam-hydrogenoxalate, thiometon; tolclofos-methyl, toxaphene, triazophos, trichlorfon, trichloronate, triflumuron, trimethacarb, vamidothion, xylylcarb.

Fungicides which can be combined with the insecticides of this invention include:
(a) dithiocarbamate and derivatives such as ferbam, ziram, maneb, mancozeb, zineb, propineb, metham, thiram, the complex of zineb and polyethylene thiuram disulfide, dazomet, and mixtures of these with copper salts;
(b) nitrophenol derivatives such as dinocap, binapacryl, and 2-*sec*-butyl-4,6-dinitrophenyl *iso*propyl carbonate;
(c) heterocyclic structures such as captan, folpet, glyodine, anilazine, ditalimfos, 4-butyl-1,2,4-triazole, 5-amino-1-[bis(dimethylamino)phosphinyl]-3-phenyl-1,2,4-triazole, etradiazole, dithianon, thioquinox, benomyl, thiabendazole, 4-(2-chlorophenylhydrazono)-3-methyl-5-isoxazolone, vinclozolin, iprodione, procymidone, triadimenol, triadimefon, bitertanol, prochloraz, fenasimol, bis-(p-chmlorophenyl)-3-pyridinemethanol, bis-(p-chlorophenyl)-5-pyrimidinemethanol, triarimol,flutriafol, flusilazole, propiconazole, ectaconazole, myclobutanil, alpha-[2-(4-chlorophenyl)ethyl]-alpha-phenyl-1H-1,2,4-triazole-1-propanenitrile, hexaconazole, cyproconazole, terbuconazole, diniconazole, fluoroimide, pyridine-2-thiol-1-oxide, 8-hydroxyquinoline sulfate and metal salts thereof, 2,3-dihydro-5-carboxanilido-6-methyl-1,4-oxathiin-4,4-dioxide,2,3-dihydro-5-carboxanilido-6-methyl-1,4-oxathiin, cis-N-[(1,1,2,2-tetrachloroethyl)thiol]-4-cyclohexene-1,2-dicarboximide, cycloheximide, dehydroacetic acid, captafol, ethirimol, quinomethionate, D,L-methyl-N-(2,6-dimethylphenyl)-N-(2′-methoxyacetyl)alanine methyl ester, D,L-methyl-N-(2,6-dimethylphenyl)-N-chloroacetyl-D,L-2-aminobutyrolactone, D,L-N-(2,6-dimethylphenyl)-N-(phenylacetyl)alanine methyl ester, 5-methyl-5-vinyl-3-(3,5-dichlorophenyl)-2,4-dioxo-1,3-oxazolidine, 3-(3,5-dichlorophenyl)-5-methyl-5-(methoxymethyl)-1,3-oxazolidi-2,4-dione, 2,4-dione, 3-(3,5-dichlorophenyl)-1-isopropylcarbamoylhydantoin, 2-cyano-[N-(ethylaminocarbonyl)-2-methoximino]acetamide, fenpropimorph, fenpropidine, 2,6-dimethyl-N-tridecylmorpholine, dodemorph, and triforine;
(d) miscellaneous halogenated fungicides such as chloranil, dichlone, chloroneb, tricamba, TCPN, dichloran, 2-chloro-1-nitropropane, polychloronitrobenzenes such as pentachloronitrobenzene (PCNB), and tetrafluorodichloroacetone;
(e) fungicidal antibiotics such as griseofulvin, kasugamycin, polyoxin, validamycin, and streptomycin;
(f) copper-based fungicides such as copper hydroxide, cuprous oxide, basic cupric chloride, basic copper carbonate, copper terephthalate, copper naphthenate and Bordeaux mixture; and
(g) miscellaneous fungicides such as dodine, phenylmercuric acetate, N-ethylmercuri-1,2,3,6-tetrahydro-3,6-endomethano-3,4,5,6,7,7-hexachlorophthalimide, phenylmercuric monoethanol ammonium lactate, p-dimethylaminobenzene sodium sulfonate, methyl isothiocyanate, 1-thiocyano-2,4-dinitrobenzene, 1-phenylthiosemicarbazide, nickel-containing compounds, calcium cyanamide, lime sulfur, thiophanate-methyl, flutolanil, edinophos, isoprothiolane, propenazole, and tricyclazole.

It has been found by biological evaluation that compounds according to the present invention have pesticidal activity and are capable of controlling larvae and adult forms of pests, especially insects from the orders *Lepidoptera* and *Coleoptera*. One skilled in the art will know how to determine the activity of a given compound against a given insect and the dosage required to obtain general or selective pesticidal effects. In addition, compounds of the present invention were found active against pyrethroid resistant pests such as the Colorado potato beetle and housefly.

In evaluating the pesticidal activity of the compounds of this invention, the following test procedures were employed.

Evaluations were made on the following insects:

| Common Name | Latin Name |
|---|---|
| Mexican Bean Beetle (MBB) | *Epilachna varivestis* |
| Southern Armyworm (SAW) | *Spodoptera eridania* |
| Boll Weevil (BW) | *Anthonomus grandis grandis* |

A test solution containing 600 parts per million (ppm) was made by dissolving the test compound in a solvent (acetone: methanol, 1:1), adding a surfactant and then water to give an acetone:methanol:water system of 5:5:90. A 1:1 mixture of an alkylarylpolyetheralcohol (sold under the trademark Triton® X-155) and a modified phthalic glycerol alkyl resin (sold under the trademark Triton® B-1956) was utilized at the equivalent of 1 ounce per 100 gal. of test solution as a surfactant.

For the bean beetle and armyworm tests, individual bean (*Phaseolus limensis* var Woods' Prolific) leaves are placed on moistened pieces of filter paper in Petri dishes. The leaves are then sprayed with test solution using a rotating turntable and allowed to dry. The dishes are infested with 10 third instar larvae of Southern armyworm or Mexican bean beetle. The dishes are then covered.

For the boll weevil test ten adult weevils are placed in a 0.5 pint glass Mason jar containing a small cube of apple. The weevils are confined to the jars by fiberglass screen mesh secured by a screw-type rim cap. The jars are then sprayed with the test solution using a rotating turntable, directing the spray through the mesh into the jar.

The percent mortality for the bean beetle, armyworm and boll weevil evaluations are determined 96 hours after treatment. Evaluations are based on a scale of 0-l00 percent in which 0 equals no activity and l00 equals total kill.

The rotating turntable consists of a fixed continuously operated spray nozzle under which targets are rotated at a fixed speed and distance. If the target is a Petri dish (such as for the armyworm), the distance from the nozzle is l5 inches. If the target is a Mason jar, the distance between the screened lid and the nozzle is 6 inches (l0 inches from the base of the jar to the nozzle). The nozzle is located 8 inches from the rotating shaft. The targets on individual platforms revolve around the shaft at l revolution per 20 seconds but only a brief portion of this time occurs in the spray path. Targets pass only once under the nozzle and then are removed to drying hoods.

The nozzle used is a l/4 JCO Spraying Systems (Wheaton, Illinois) air atomizing nozzle equipped with a No. 2850 fluid cap and No. 70 air cap. At the l0 psig air pressure used with liquid siphon feed 0.5 GPH (gallons per hour) are delivered in a round spray pattern with a 21° spray angle. Targets are misted with spray droplets to the point that the droplets coalesce to form a uniform thin film insufficient to drown test organisms.

All treatments are maintained at 75-80°F (24-27°C) under continuous fluorescent light in a well-ventilated room. The results of the initial pesticidal evaluations are given in Table III.

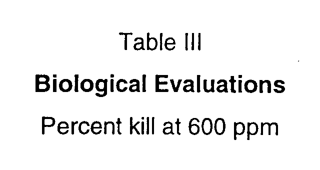

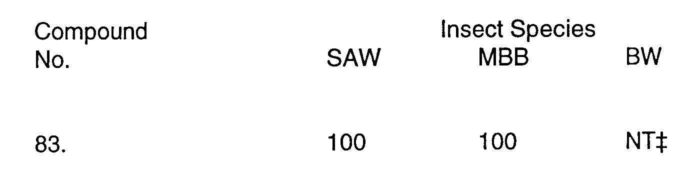

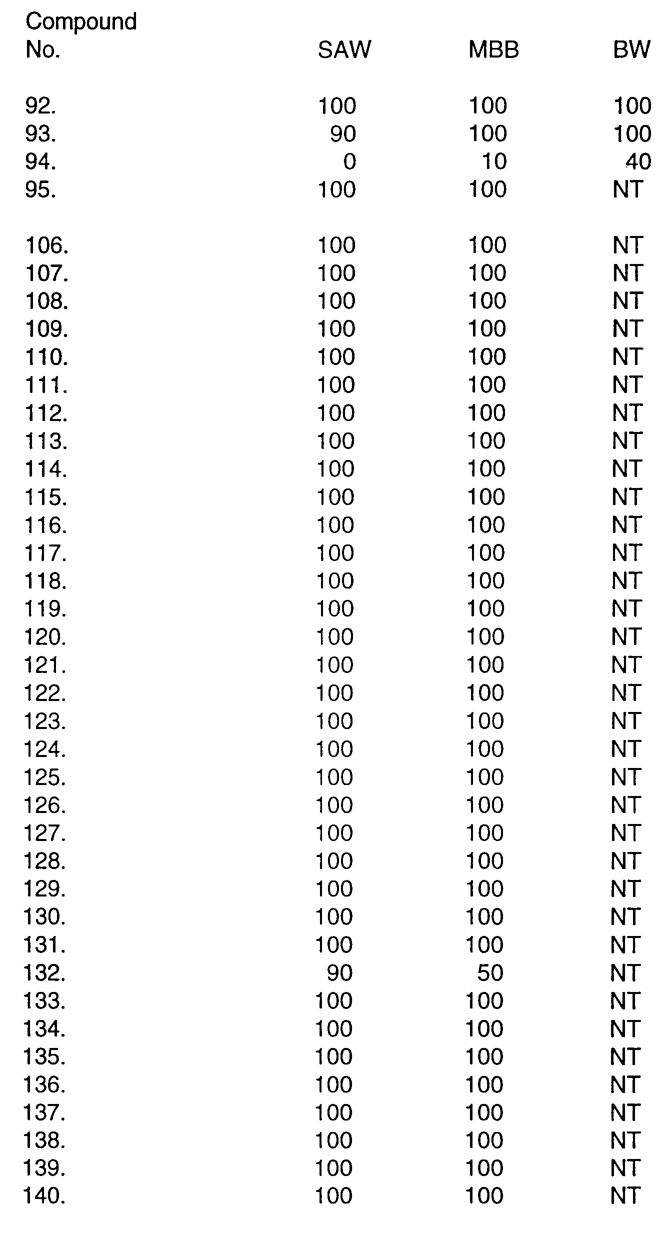

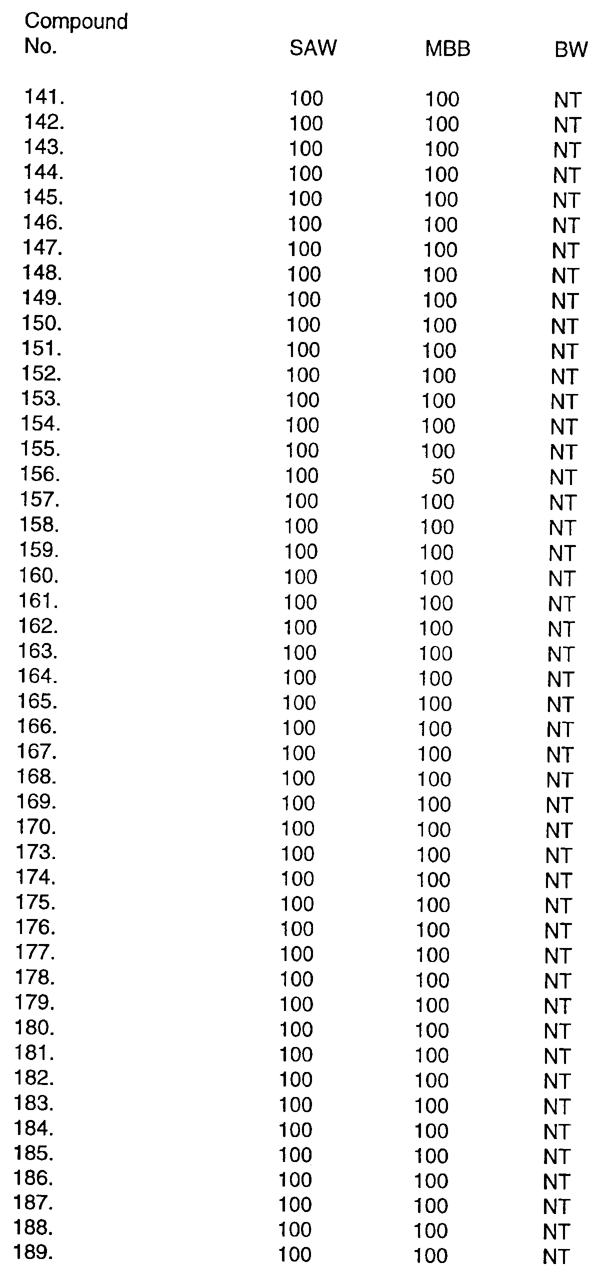

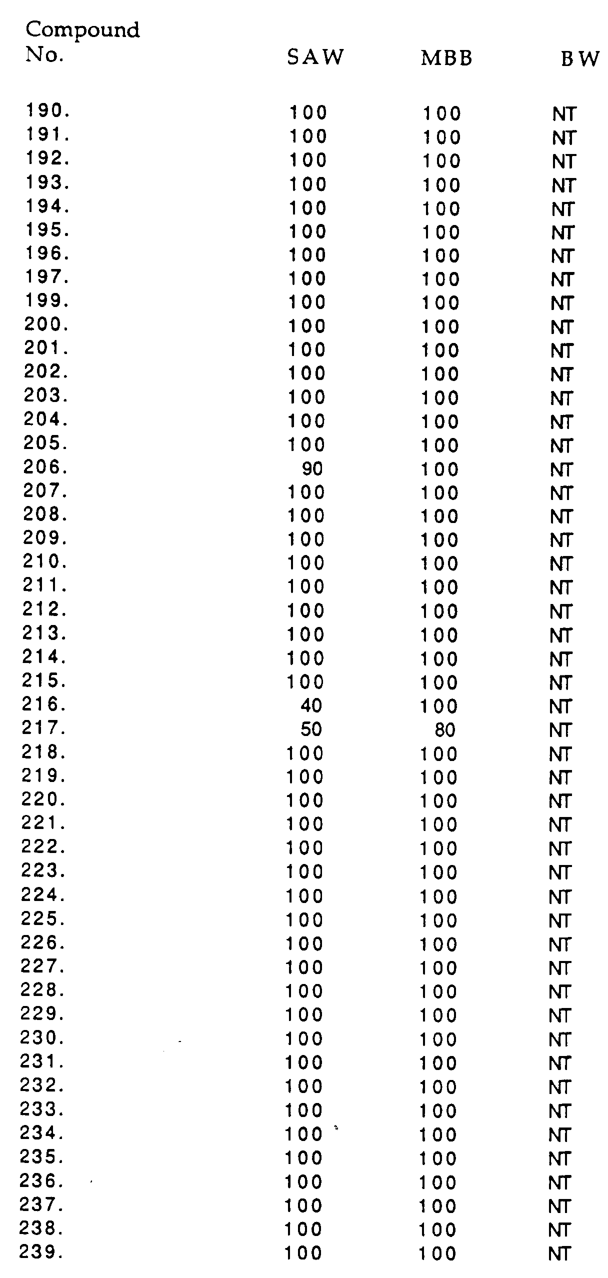

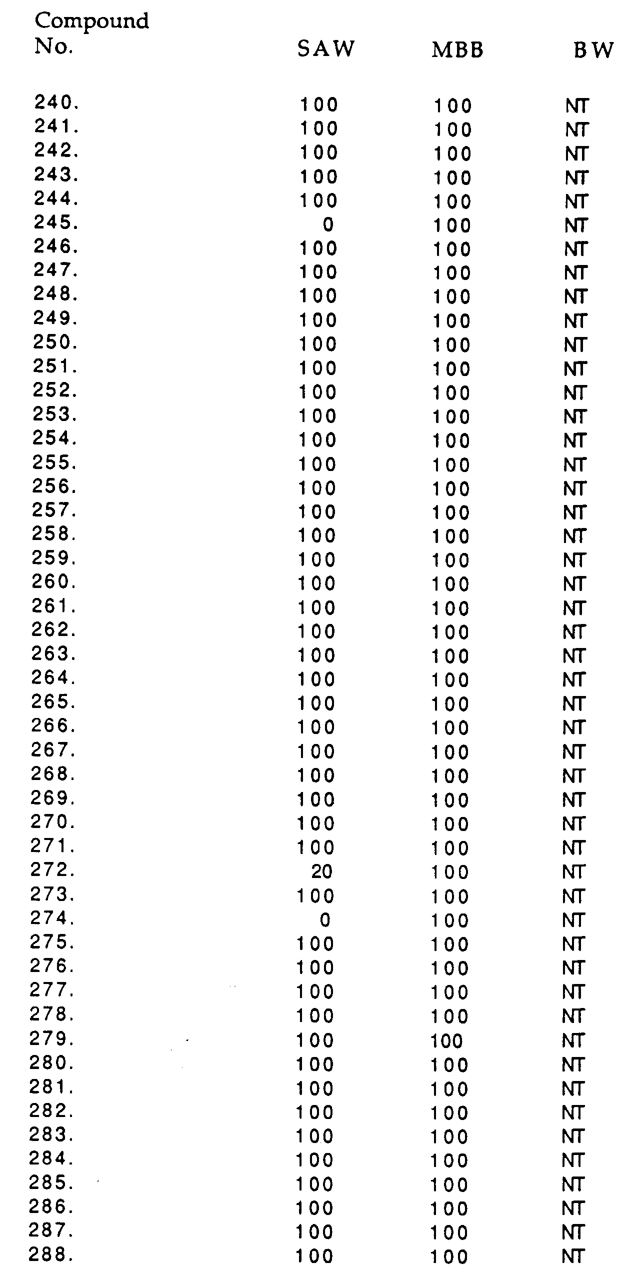

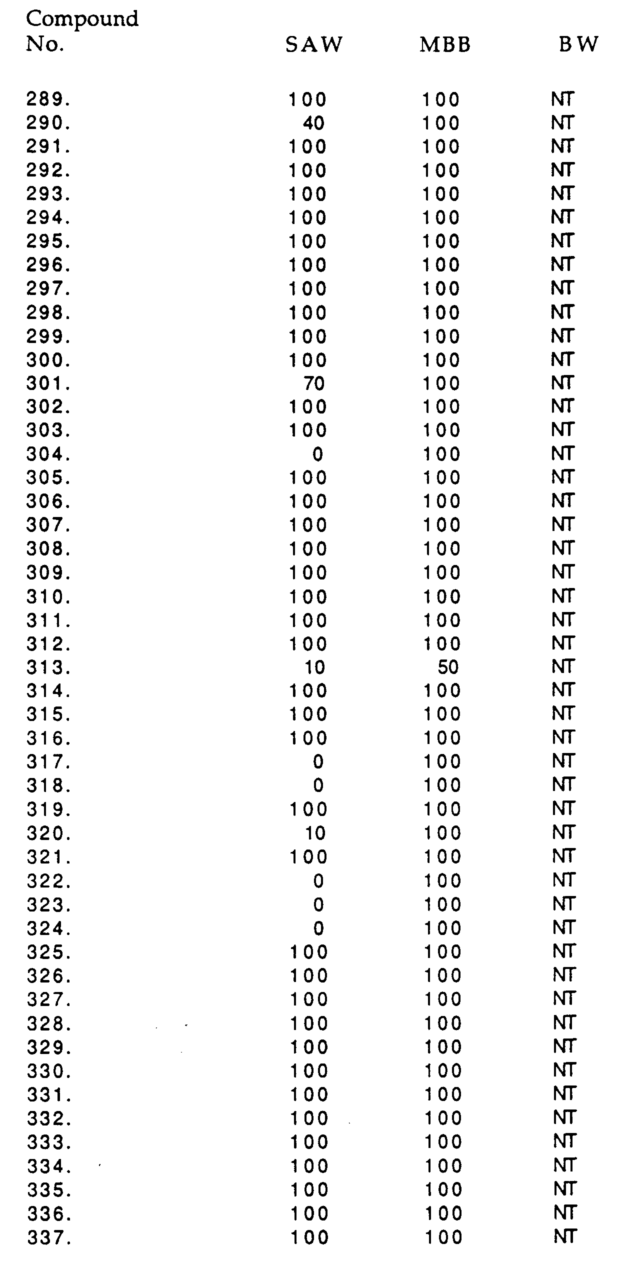

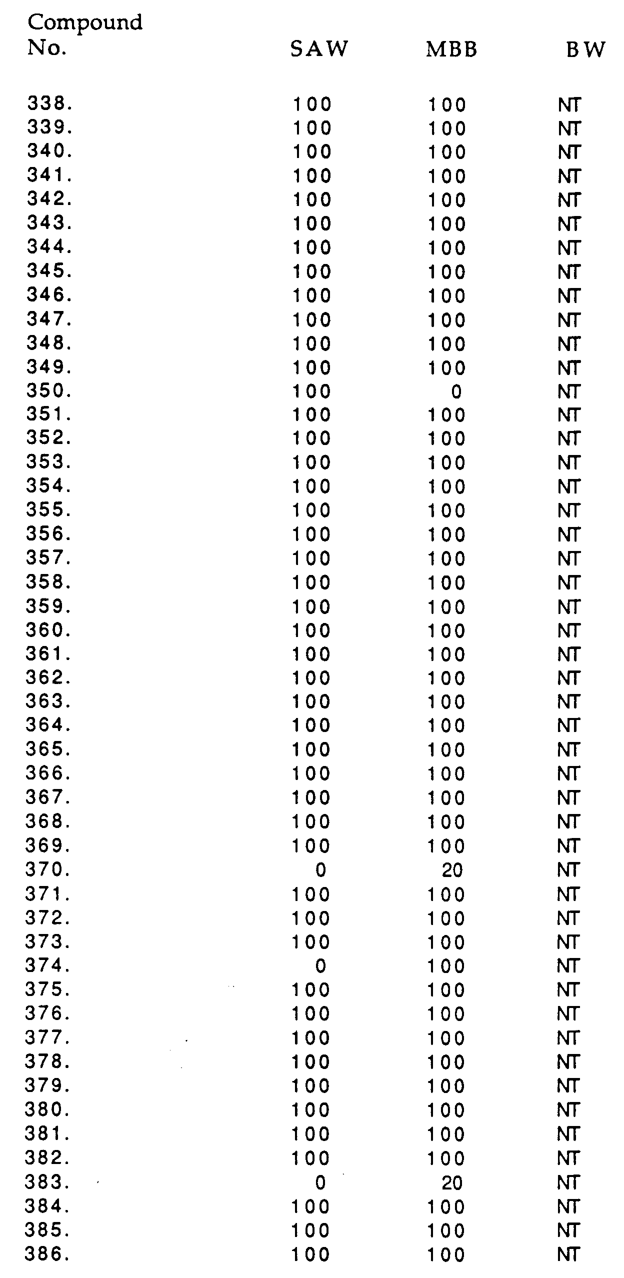

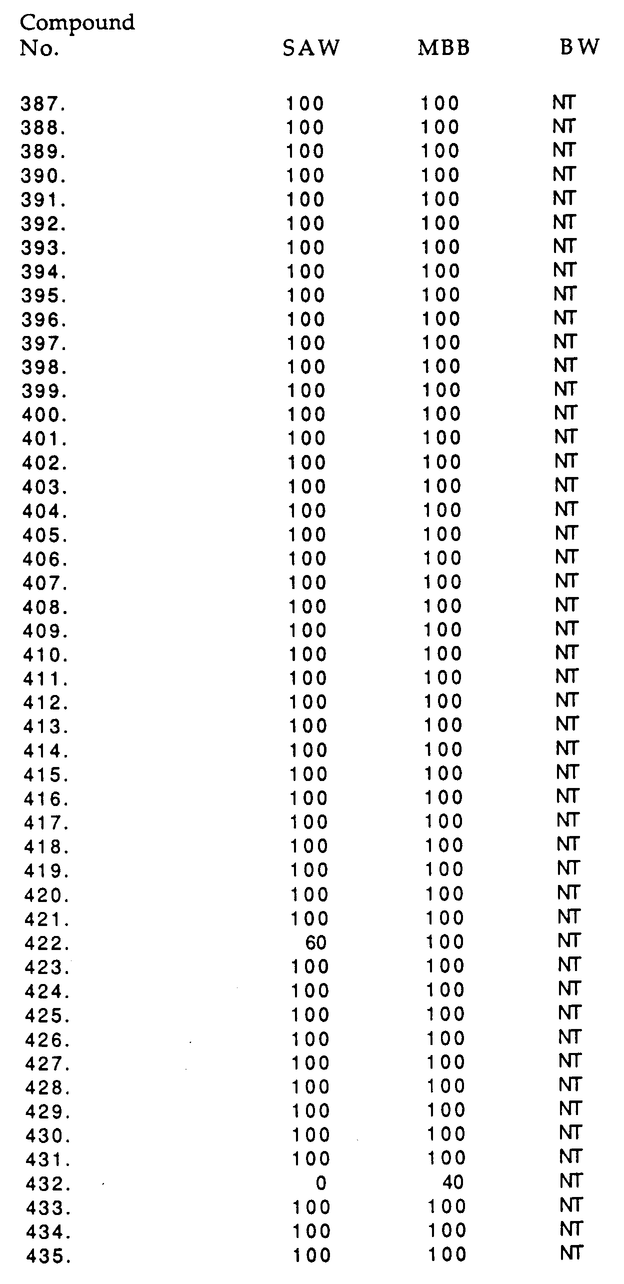

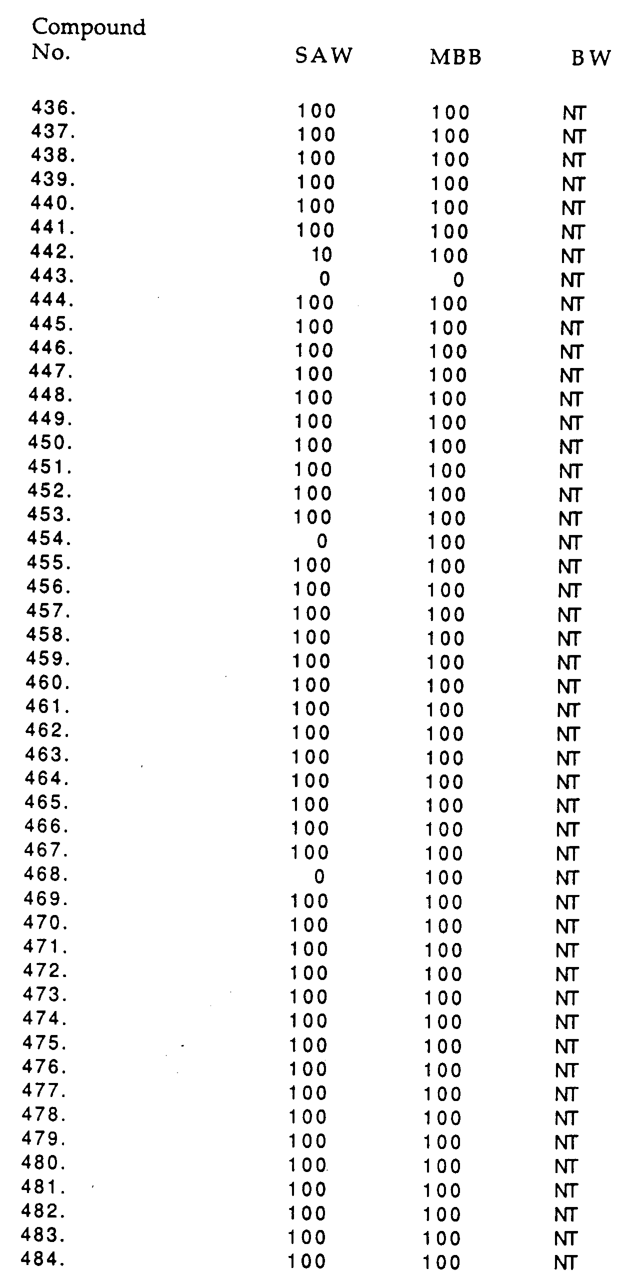

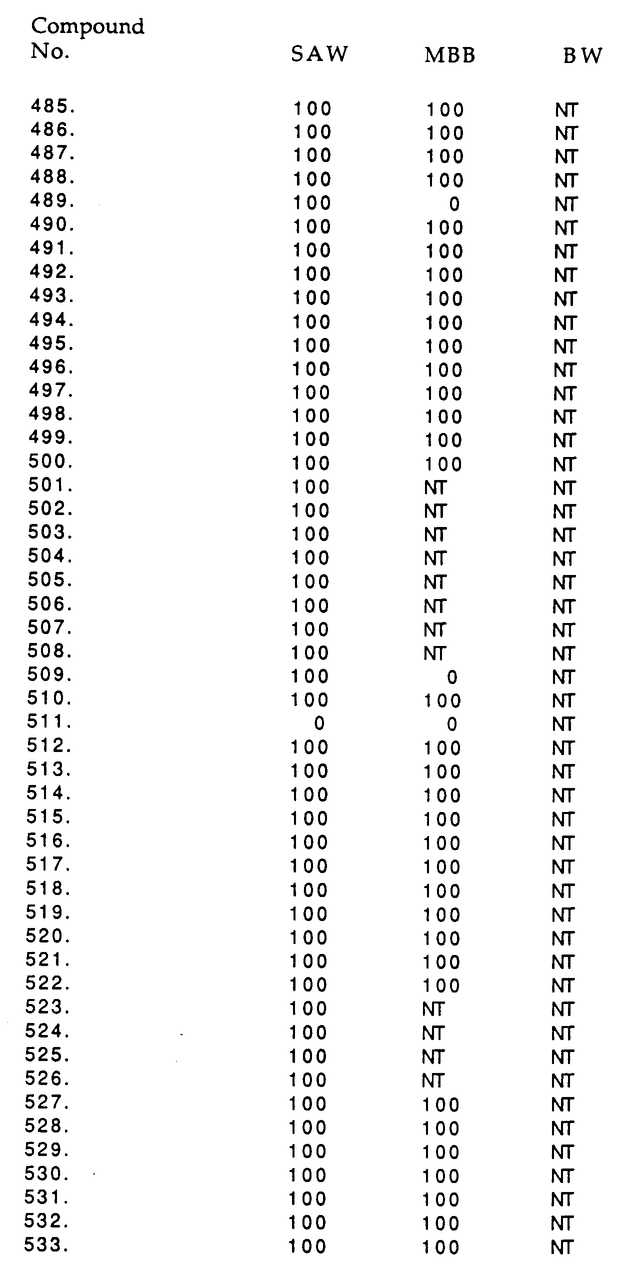

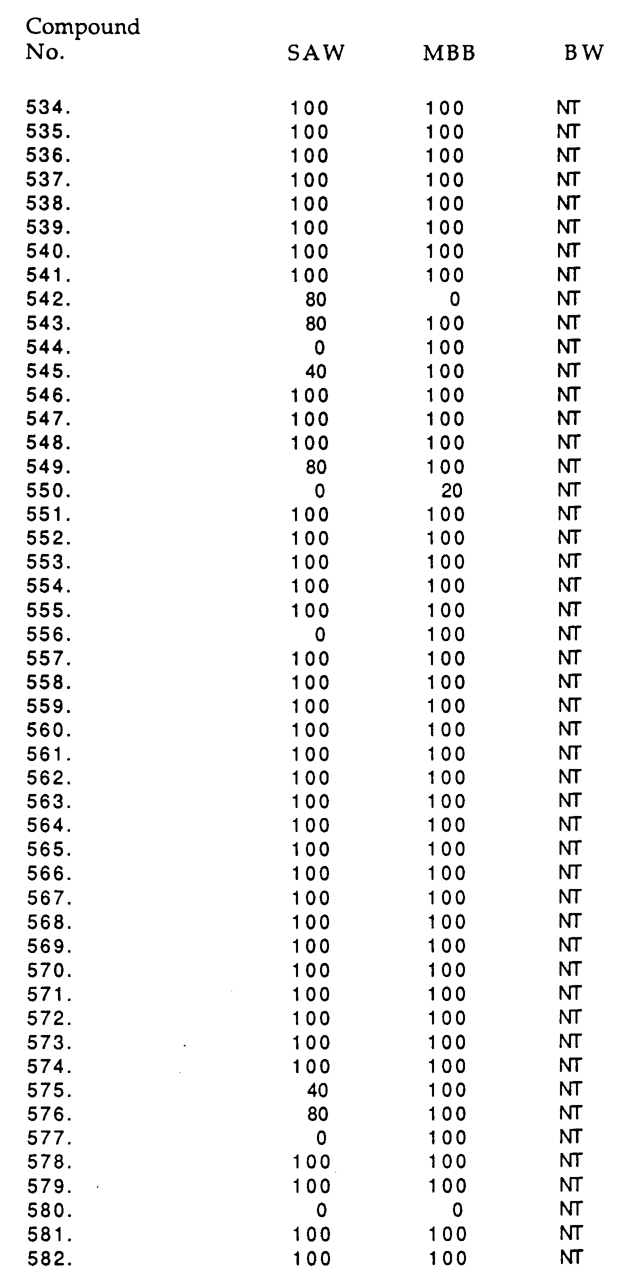

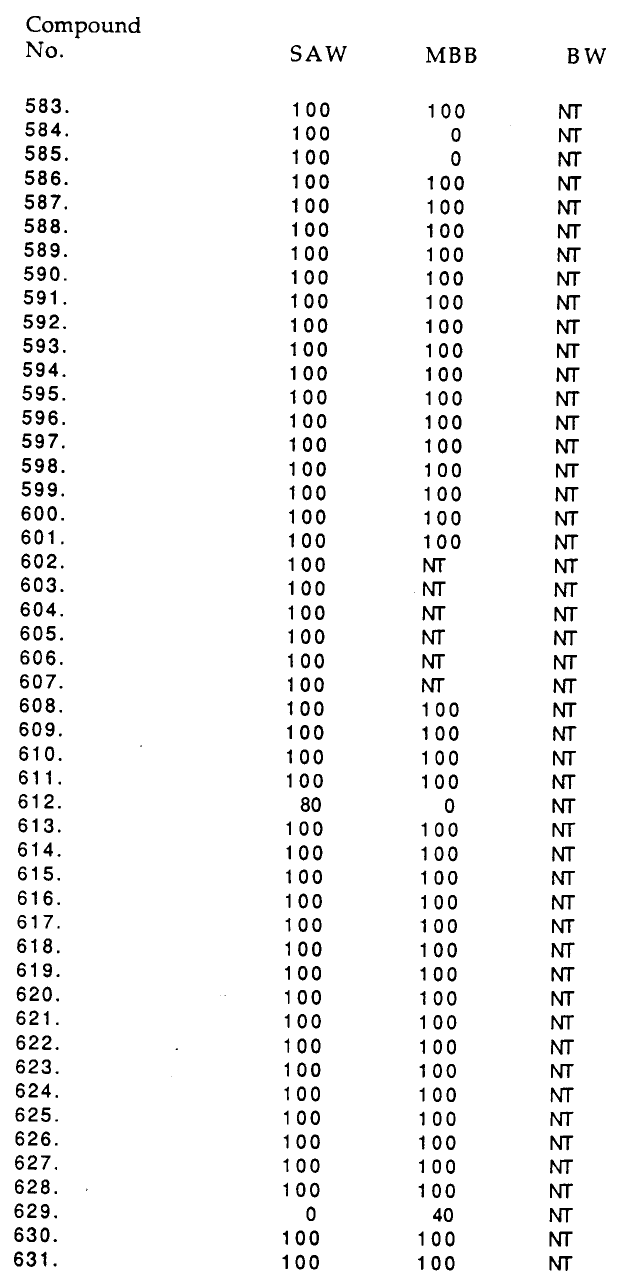

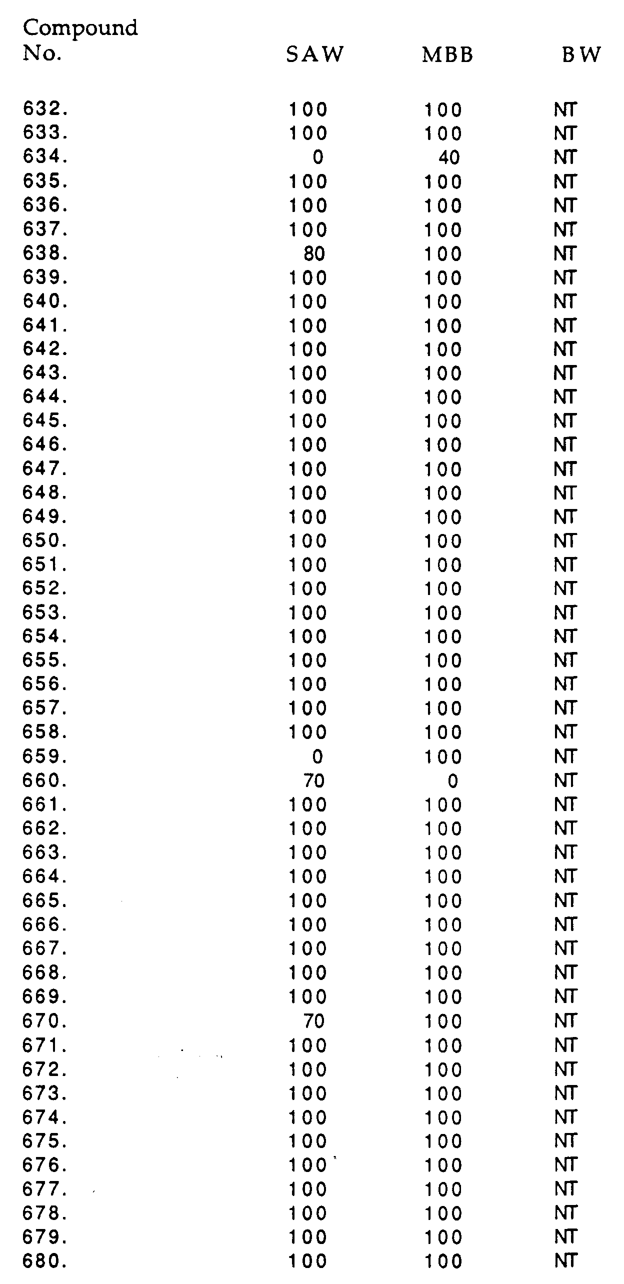

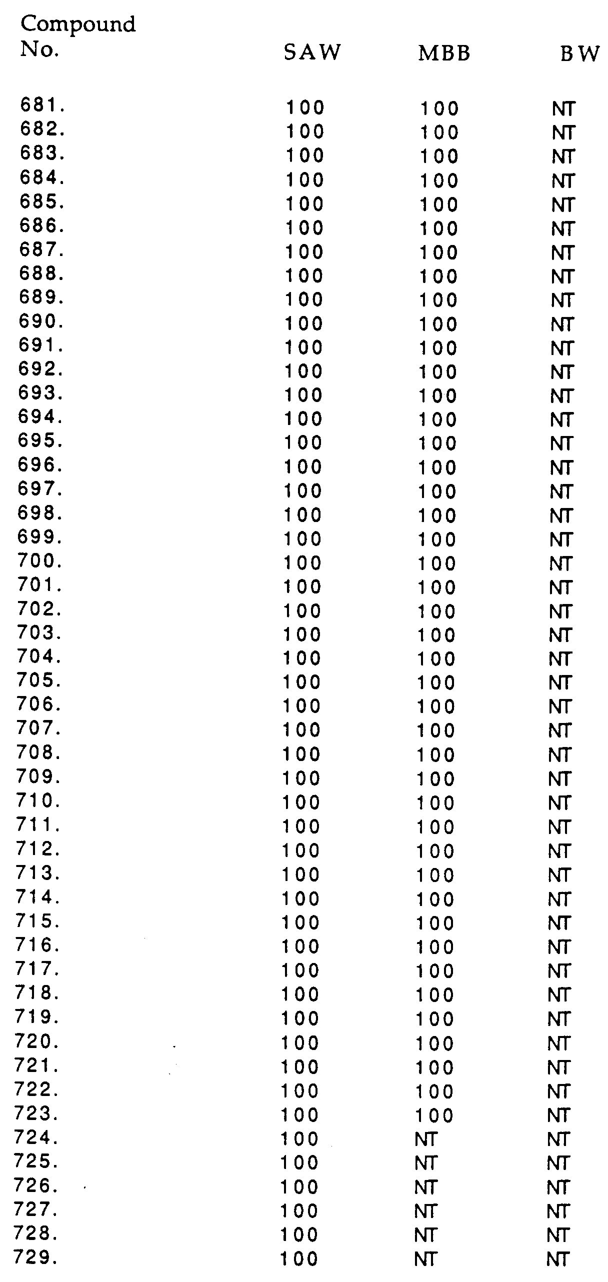

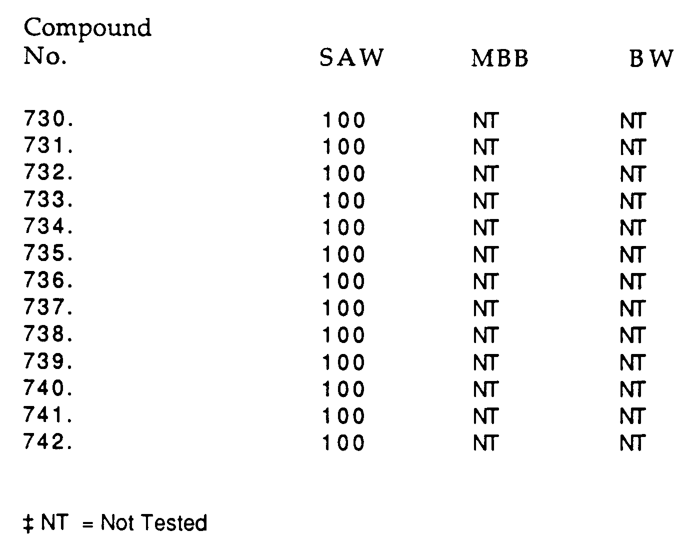

## Claims

1. A compound of the formula 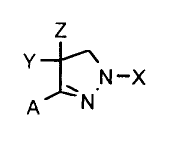 wherein
X is 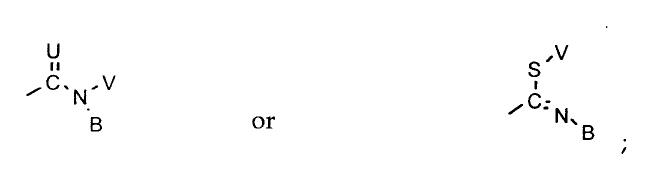
A and B are pyridyl, furyl, thiazolyl or naphthyl, each optionally and independently substituted by one or two of nitro, (C₁-C₆)alkyl, halo(C₁-C₆)alkyl or halo; or
phenyl optionally substituted with one to three of (C₁-C₆)alkyl; halo(C₁-C₆)alkyl; halo; (C₁-C₆)alkoxy; halo(C₁-C₆)alkoxy; (C₃-C₆)alkenyloxy; (C₃-C₆)alkynyloxy; (C₁-C₆)alkoxy(C₁-C₆)alkoxy; phenyl(C₁-C₆)alkoxy; phenyloxy; pyridyloxy; mono(C₁-C₆)alkylaminocarbonyloxy; di(C₁-C₆)alkylaminocarbonyloxy; (C₁-C₆)alkanoyloxy; (C₁-C₆)alkoxycarbonyloxy; (C₁-C₆)alkylsulfonyloxy; (C₁-C₆)alkylthio; halo(C₁-C₆)alkylthio; (C₁-C₆)alkoxy(C₁-C₆)alkyl; (C₁-C₆)alkanoyl; (C₁-C₆)alkoxycarbonyl; nitro; (C₁-C₆)alkylsulfonyl;halo(C₁-C₆)alkylsulfonyl; phenyl; hydroxy; cyano; isocyano; amino; mono(C₁-C₆)alkylamino; di(C₁-C₆)alkylamino; formylamino; (C₁-C₆)alkanoylamino; halo(C₁-C₆)alkanoylamino; phenylcarbonylamino; mono(C₁-C₆)alkylaminocarbonylamino; or di(C₁-C₆)alkylaminocarbonylamino;
U is oxygen or sulfur;
V is hydrogen, (C₁-C₆)alkyl, (C₁-C₆)alkoxy(C₁-C₆)alkyl, formyl, (C₁-C₆)alkylcarbonyl, (C₁-C₆)alkylaminocarbonyl, (C₁-C₆)alkoxycarbonyl, (C₃-C₆)alkenyloxycarbonyl, phenyloxycarbonyl, (C₁-C₆)alkoxycarbonylcarbonyl, cyano(C₁-C₆)alkylthio, (C₁-C₆)alkylthio, phenylthio, (C₁-C₆)alkoxycarbonyl(C₁-C₆)alkylthio or (C₁-C₆)alkoxycarbonylthio; with the proviso that V is not hydrogen if attached to S;
Y is -NR¹R²;
wherein R¹ and R² are each independently hydrogen, cyano, (C₁-C₆)alkyl, halo(C₁-C₆)alkyl, cyano(C₁-C₆)alkyl, phenyl(C₁-C₆)alkyl, (C₃-C₆)alkenyl, halo(C₃-C₆)alkenyl, (C₃-C₆)alkynyl, phenyl, halophenyl, formyl, (C₁-C₆)alkylcarbonyl, halo(C₁-C₆)alkylcarbonyl, (C₂-C₆)alkenylcarbonyl, halo(C₂-C₆)alkenylcarbonyl, (C₁-C₆)alkoxy(C₁-C₆)alkylcarbonyl, phenylcarbonyl, phenyl(C₂-C₆)alkenylcarbonyl, carboxy, (C₁-C₆)alkoxycarbonyl, halo(C₁-C₆)alkoxycarbonyl, cyano(C₁-C₆)alkoxycarbonyl, (C₂-C₆)alkenyloxycarbonyl, (C₃-C₆)alkynyloxycarbonyl, (C₁-C₆)alkanoyl(C₁-C₆)alkoxycarbonyl, (C₁-C₆)alkoxycarbonyl(C₁-C₆)alkoxycarbonyl, carboxy(C₁-C₆)alkoxycarbonyl, phenyloxycarbonyl, phenyl(C₁-C₆)alkoxycarbonyl, ((C₁-C₆)alkylthio)carbonyl, N-(C₁-C₆)alkylaminocarbonyl, N,N-di(C₁-C₆)alkylaminocarbonyl, N-phenyl-N-(C₁-C₆)alkylaminocarbonyl, N-(phenylcarbonyl)aminocarbonyl, di(C₁-C₆)alkylphosphoryl, (C₁-C₆)alkylsulfonyl, (C₂-C₆)alkenylsulfonyl, N,N-di(C₁-C₆)alkylaminosulfonyl, phenylsulfonyl, pyridyl or pyrazinyl; or
R¹ and R² together with the nitrogen to which they are attached form a pyrid-2-one-1-yl, pyrid-4-one-1-yl, triazolyl, 2-oxazolidonyl, isomorpholin-2-yl, pyrrolidinonyl, piperidonyl or succinimidyl ring; and
Z is hydrogen; and
agronomically acceptable salts thereof.

2. A compound as claimed in claim 1, wherein
X is 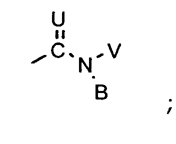 and
V is hydrogen.

3. A compound as claimed in claim 2 having the formula 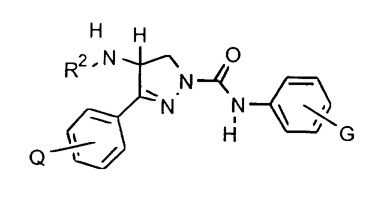 wherein
Q is hydrogen, halo, halo(C₁-C₆)alkoxy or (C₁-C₆)alkoxy;
G is halo, halo(C₁-C₆)alkyl or halo(C₁-C₆)alkoxy; and
R² is hydrogen, (C₁-C₆)alkyl, phenyl(C₁-C₆)alkyl, cyano, (C₁-C₆)alkoxycarbonyl, halo(C₁-C₆)alkoxycarbonyl, ((C₁-C₆)alkylthio)carbonyl, halo(C₁-C₆)alkoxycarbonyl, cyano(C₁-C₆)alkoxycarbonyl, (C₁-C₆)alkoxy(C₁-C₆)alkoxycarbonyl, (C₁-C₆)alkoxycarbonyl(C₁-C₆)alkoxycarbonyl, phenoxycarbonyl, phenyl(C₁-C₆)alkoxycarbonyl, (C₃-C₆)alkenyloxycarbonyl, (C₃-C₆)alkynyloxycarbonyl, formyl, (C₁-C₆)alkylcarbonyl, halo(C₁-C₆)alkylcarbonyl, carboxy(C₁-C₆)alkylcarbonyl, phenylcarbonyl, phenyl(C₁-C₆)alkylcarbonyl, phenyl(C₂-C₆)alkenylcarbonyl, mono(C₁-C₆)alkylaminocarbonyl, di(C₁-C₆)alkylaminocarbonyl, phenylaminocarbonyl, (C₁-C₆)alkoxycarbonyl(C₁-C₆)alkylaminocarbonyl, phenyl((C₁-C₆)alkyl)aminocarbonyl, di(C₁-C₆)alkylphosphoryl, di(C₁-C₆)alkylthiophosphoryl, (C₁-C₆)alkylsulfonyl, (C₂-C₆)alkenylsulfonyl, halo(C₁-C₆)alkylsulfonyl, phenylsulfonyl, di(C₁-C₆)alkylaminosulfonyl, 2-pyridyl or 2-pyrazinyl.

4. A compound as claimed in claim 3, wherein
Q is hydrogen, G is 4-trifluoromethyl and R² is methoxycarbonyl; or
Q is 4-chloro, G is 4-trifluoromethyl and R² is methoxycarbonyl; or
Q is 4-chloro, G is 4-trifluoromethoxy and R² is methoxycarbonyl; or
Q is 4-chloro, 4-n-butyloxy, 4-n-propyloxy or 4-difluoromethoxy, G is 4-trifluoromethyl, 4-trifluoromethoxy or 4-(1,1,2,2-tetrafluoro ethoxy) and R² is n-propyl.

5. A compound as claimed in claim 1, wherein
V is (C₁-C₆)alkyl, (C₁-C₆)alkoxy(C₁-C₆)alkyl, formyl, (C₁-C₆)alkylcarbonyl, (C₁-C₆)alkylaminocarbonyl, (C₁-C₆)alkoxycarbonyl, (C₃-C₆)alkenyloxycarbonyl, phenyloxycarbonyl, (C₁-C₆)alkoxycarbonyl-carbonyl, cyano(C₁-C₆)alkylthio, (C₁-C₆)alkylthio, phenylthio, (C₁-C₆)alkoxycarbonyl(C₁-C₆)alkylthio or (C₁-C₆)alkoxycarbonylthio.

6. A compound as claimed in claim 5 having the formula 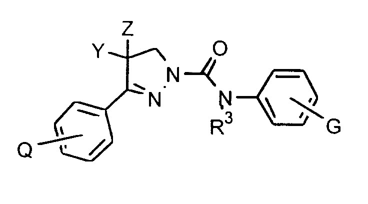 wherein
Q is halo; preferably 4-halo;
G is halo or halo(C₁-C₆)alkyl, preferably 4-halo or 4-halo
(C₁-C₆)alkyl;
R¹ is hydrogen or (C₁-C₆)alkyl;
R² is (C₁-C₆)alkoxycarbonyl;
R³ is (C₁-C₆)alkyl; and
Z is hydrogen.

7. A compound as claimed in claim 5, wherein
Q is 4-chloro, G is 4-chloro, R¹ is methyl, R² is methoxycarbonyl, R³ is methyl and Z is hydrogen; or
Q is 4-chloro, G is 4-trifluoromethyl, R¹ is hydrogen, R² is methoxycarbonyl, R³ is methyl and Z is hydrogen; or
Q is 4-chloro, G is 4-trifluoromethyl, R¹ is methyl, R² is methoxycarbonyl, R³ is methyl and Z is hydrogen.

8. A compound as claimed in claim 1, wherein X is 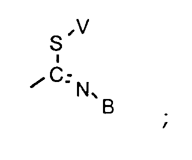
A is 4-chlorophenyl or 4-n-propyloxyphenyl;
B is 4-trifluoromethylphenyl;
Y is N-methyl-N-methoxycarbonylamino or N-propyl-N-formylamino;
Z is hydrogen; and
V is methyl, ethyl, n-propyl, isopropyl, propenyl, methoxyethyl or benzyl.

9. A compound as claimed in claim 1, wherein X is 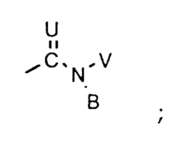
U is sulfur, and preferably A is 4-chlorophenyl or 4-n-propyloxyphenyl, B is 4-chlorophenyl or 4-trifluoromethylphenyl, Y is N-methyl-N-methoxycarbonylamino, V is hydrogen and Z is hydrogen.

10. A compound as claimed in claim 2 having the formula 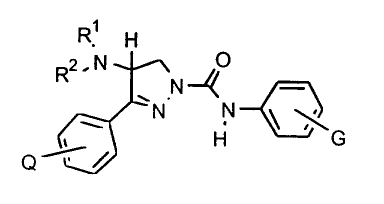 wherein
Q is hydrogen, halo, (C₁-C₆)alkyl, (C₁-C₆)alkoxy (C₁-C₆)alkyl, halo(C₁-C₆)alkyloxy or (C₁-C₆)alkoxy;
G is halo, cyano, halo(C₁-C₆)alkyl, (C₁-C₆)alkoxycarbonyl or halo(C₁-C₆)alkoxy;
R¹ is (C₁-C₆)alkyl, (C₃-C₆)alkenyl, (C₃-C₆)alkynyl, (C₁-C₆)alkoxy (C₁-C₆)alkyl, cyano (C₁-C₆)alkyl, (C₁-C₆)alkoxycarbonyl (C₁-C₆)alkyl, phenyl(C₁-C₆)alkyl, phenyl or halophenyl;
R² is cyano, (C₁-C₆)alkyl, (C₁-C₆)alkylsulfonyl, (C₁-C₆)alkoxycarbonyl, ((C₁-C₆)alkylthio)carbonyl, halo(C₁-C₆)alkoxycarbonyl, formyl, (C₁-C₆)alkylcarbonyl, halo(C₁-C₆)alkylcarbonyl, (C₁-C₆)alkoxy(C₁-C₆)alkylcarbonyl, (C₂-C₆)alkenyl carbonyl, halo(C₂-C₆)alkenylcarbonyl, di(C₁-C₆)alkylaminocarbonyl, phenylcarbonyl, di(C₁-C₆)alkylphosphoryl or di(C₁-C₆)alkylthiophosphoryl; or
R¹ and R² together with the nitrogen to which they are attached form pyrid-2-one-1-yl, pyrid-4-one-1-yl, triazolyl, 2-oxasolidonyl, isomorpholin-2-onyl, pyrrolidinonyl, piperidonyl or succinimidyl.

11. A compound as claimed in claim 10, wherein
Q is 4-chloro, G is 4-trifluoromethyl, R¹ is methyl and R² is formyl, methylcarbonyl, ethylcarbonyl, dimethylaminocarbonyl, methoxycarbonyl, ethoxycarbonyl or isopropyloxycarbonyl; or
Q is 4-chloro, G is 4-trifluoromethyl, R² is methoxycarbonyl and R¹ is ethyl, propyl or propargyl; or
Q is 4-chloro, G is 4-trifluoromethoxy, R² is methoxycarbonyl and R¹ is methyl, ethyl, propyl, allyl or propargyl; or
Q is 4-chloro, G is 4-difluoromethoxy, R¹ is methyl and R² is methoxycarbonyl; or
Q is 4-chloro, G is 4(1,1,2,2-tetrafluoroethoxy), R² is methoxycarbonyl and R¹ is methyl, ethyl or propargyl; or
Q is 4-chloro, G is 4-(1,1,2,3,3,3-hexafluoropropyloxy), R¹ is methyl and R² is methoxycarbonyl; or
Q is 4-ethoxy, G is 4-trifluoromethoxy, R¹ is methyl and R² is methoxycarbonyl; or
Q is 4-n-propyloxy, G is 4-trifluoromethoxy, R¹ is methyl and R² is methoxycarbonyl; or
Q is 4-n-butyloxy, G is 4-trifluoromethoxy, R¹ is methyl and R² is methoxycarbonyl; or
Q is 4-difluoromethoxy, R¹ is methyl, R² is methoxycarbonyl and G is 4-chloro, 4-trifluoromethyl, 4-difluoromethoxy, 4-trifiuoromethoxy, 4-(1,1,2,2-tetrafluoroethoxy), 4-(1,1,2,3,3,3-hexafluoropropyloxy), or 4-isopropyloxycarbonyl; or
Q is hydrogen, R¹ is methyl, R² is methoxycarbonyl and G is 4-trifluoromethyl or 4-trifluoromethoxy.

12. A pesticidal composition, which comprises an agriculturally acceptable carrier and a pesticidally effective amount of a compound according to any preceding claim.

13. A composition as claimed in claim 12, wherein the compound is present in an amount of from 0.0001 to 99 percent, preferably 0.001 to 90 percent, more preferably 0.01 to 75 percent by weight of the composition.

14. A method of controlling pests, preferably insects of the orders Lepidoptera and Coleoptera, which comprises contacting the insects with a pesticidally effective amount of a compound according to any of claims 1 to 11, or a composition according to claim 12 or 13, the compound itself preferably being applied at a rate of from 0.1 to 1000g, preferably 5 to 200g, per hectare.

15. A process for preparing compounds as defined in any one of Claims 1 to 11 having the formula 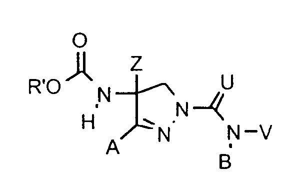
wherein R^{'} is alkyl,
by the steps of:
saponifying a compound of the structure 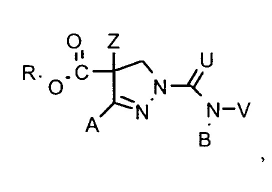 wherein R is alkyl,
obtaining the corresponding carboxylic acid having the formula 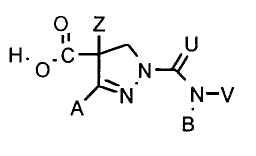
reacting the carboxylic acid to obtain the corresponding carboxylic acid halide;
reacting the carboxylic acid halide with azide anion to obtain the corresponding azidocarbonyl compound;
heating the azidocarbonyl compound to obtain the corresponding isocyanato compound; and
reacting the isocyanato compound with an alcohol to obtain said carboalkoxyamino compound.

16. A process as claimed in claim 15, which further comprises decarboxylating the compound of formula 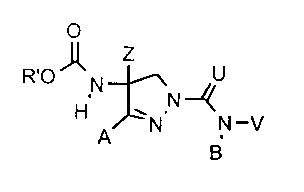 wherein R' is t-butyl to obtain an amino compound having the formula 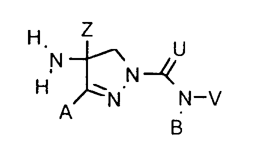 and then optionally alkylating said amino compound to form a compound having the formula 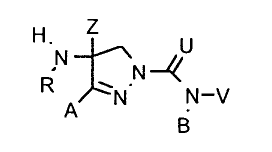 wherein R is alkyl,
and then optionally reacting that compound with an acylating agent to obtain a compound having the formula 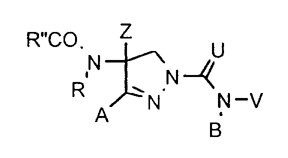 wherein R'' is hydrogen, (C₁-C₆)alkyl, or (C₁-C₆)alkoxy.

17. A process for the preparing compounds as claimed in claim 1 having the formula 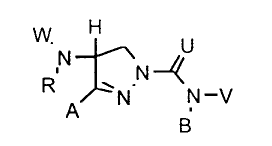 wherein W is (C₁-C₆)alkoxycarbonyl, which process comprises
halogenating a methylketone of the formula 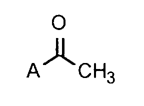 to obtain a halomethylketone;
reacting the halomethylketone with a mono-or di-alkylamine to obtain the corresponding mono- or di-alkylated ketone having the formula 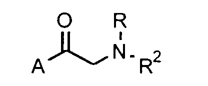
wherein R is (C₁-C₆)alkyl and R² is hydrogen or methyl;
acylating the alkylaminomethyl ketone to obtain an acylated compound having the formula 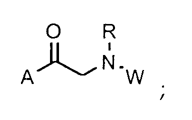
reacting the acylated compound with formaldehyde to obtain the corresponding prop-2-enone having the formula 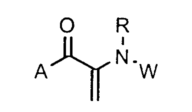 wherein W is (C₁-C₆)alkoxycarbonyl;
reacting the prop-2-enone with hydrazine to obtain the corresponding dihydropyrazole having the formula 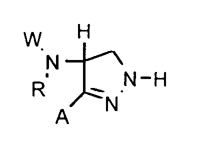 and reacting the dihydropyrazole with an isocyanate to obtain a compound having the formula

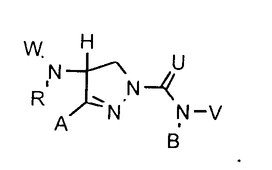

## Patentansprüche

1. Verbindung der Formel 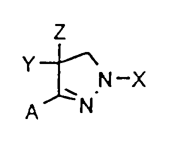 wobei X 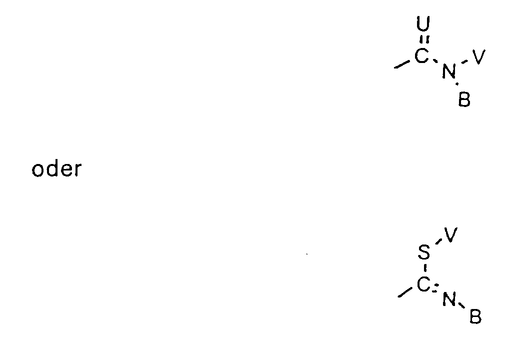
A und B Pyridyl, Furyl, Thiazolyl oder Naphthyl sind, die jeweils gegebenfalls und unabhängig voneinander durch eine oder zwei Gruppen, ausgewählt aus Nitro(C₁-C₆)alkyl, Halogen(C₁-C₆)alkyl oder Halogen, substituiert sind, oder
Phenyl ist, gegebenfalls substituiert mit einer bis drei Gruppen, ausgewählt aus (C₁-C₆)Alkyl, Halogen(C₁-C₆)alkyl, Halogen, (C₁-C₆)Alkoxy, Halogen(C₁-C₆)alkoxy, (C₃-C₆)Alkenyloxy, (C₃-C₆)Alkynyloxy, (C₁-C₆)Alkoxy(C₁-C₆)alkoxy, Phenyl(C₁-C₆)alkoxy, Phenyloxy, Pyridyloxy, Mono(C₁-C₆)alkylaminocarbonyloxy, Di(C₁-C₆)alkylaminocarbonyloxy; (C₁-C₆)Alkanoyloxy, (C₁-C₆)Alkoxycarbonyloxy, (C₁-C₆)Alkylsulfonyloxy, (C₁-C₆)Alkylthio, Halogen(C₁-C₆)alkylthio, (C₁-C₆)Alkoxy(C₁-C₆)alkyl, (C₁-C₆)Alkanoyl, (C₁-C₆)Alkoxycarbonyl, Nitro, (C₁-C₆)Alkylsulfonyl,Halogen(C₁-C₆)alkylsulfonyl, Phenyl, Hydroxy, Cyano, Isocyano, Amino, Mono(C₁-C₆)alkylamino, Di(C₁-C₆)alkylamino, Formylamino, (C₁-C₆)Alkanoylamino; Halogen(C₁-C₆)alkanoylamino, Phenylcarbonylamino, Mono(C₁-C₆)alkylaminocarbonylamino oder Di(C₁-C₆)alkylaminocarbonylamino,
U Sauerstoff oder Schwefel ist,
V Wasserstoff, (C₁-C₆)Alkyl, (C₁-C₆)Alkoxy(C₁-C₆)alkyl, Formyl, (C₁-C₆)Alkylcarbonyl, (C₁-C₆)Alkylaminocarbonyl, (C₁-C₆)Alkoxycarbonyl, (C₃-C₆)Alkenyloxycarbonyl,Phenyloxycarbonyl,(C₁-C₆)Alkoxycarbonylcarbonyl, Cyano(C₁-C₆)alkylthio, (C₁-C₆)Alkylthio, Phenylthio, (C₁-C₆)Alkoxycarbonyl(C₁-C₆)alkylthio oder (C₁-C₆)Alkoxycarbonylthio ist, mit der Maßgabe, daß V nicht Wasserstoff ist, falls es mit S verbunden ist,
Y -NR¹R² ist,
wobei R¹ und R² jeweils unabhängig voneinander Wasserstoff, Cyan, (C₁-C₆)Alkyl, Halogen(C₁-C₆)alkyl, Cyano(C₁-C₆)alkyl, Phenyl(C₁-C₆)alkyl, (C₃-C₆)Alkenyl, Halogen(C₃-C₆)alkenyl, (C₃-C₆)Alkynyl, Phenyl, Halogenphenyl, Formyl, (C₁-C₆)Alkylcarbonyl, Halogen(C₁-C₆)alkylcarbonyl, (C₂-C₆)Alkenylcarbonyl, Halogen(C₂-C₆)alkenylcarbonyl, (C₁-C₆)Alkoxy(C₁-C₆)alkylcarbonyl, Phenylcarbonyl, Phenyl(C₂-C₆)alkenylcarbonyl, Carboxy, (C₁-C₆)Alkoxycarbonyl, Halogen(C₁-C₆)alkoxycarbonyl, Cyano(C₁-C₆)alkoxycarbonyl, (C₂-C₆)Alkenyloxycarbonyl, (C₃-C₆)Alkynyloxycarbonyl, (C₁-C₆)Alkanoyl(C₁-C₆)alkoxycarbonyl, (C₁-C₆)Alkoxycarbonyl(C₁-C₆)alkoxycarbonyl, Carboxy(C₁-C₆)alkoxycarbonyl, Phenyloxycarbonyl, Phenyl(C₁-C₆)alkoxycarbonyl, ((C₁-C₆)Alkylthio)carbonyl, N-(C₁-C₆)Alkylaminocarbonyl, N,N-Di(C₁-C₆)alkylaminocarbonyl, N-Phenyl-N-(C₁-C₆)alkylaminocarbonyl, N-(Phenylcarbonyl)aminocarbonyl, Di(C₁-C₆)alkylphosphoryl, (C₁-C₆)Alkylsulfonyl, (C₂-C₆)Alkenylsulfonyl, N,N-Di(C₁-C₆)alkylaminosulfonyl, Phenylsulfonyl, Pyridyl oder Pyrazinyl sind, oder
R¹ und R² zusammen mit dem Stickstoff, mit welchem sie verbunden sind, einen Pyrid-2-on-1-yl-, Pyrid-4-on-1-yl-, Triazolyl-, 2-Oxazolidonyl-, Isomorpholin-2-yl-, Pyrrolidinonyl-, Piperidonyl- oder einen Succinimidylring bilden, und Z Wasserstoff ist, und agronomisch verträgliche Salze davon.

2. Verbindung nach Anspruch 1, wobei X 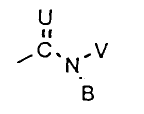 ist und V Wasserstoff ist.

3. Verbindung nach Anspruch 2 mit der Formel 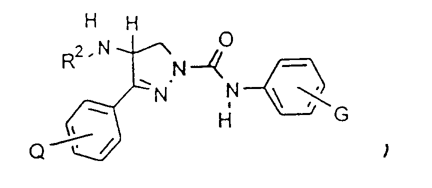 wobei
Q Wasserstoff, Halogen, Halogen(C₁-C₆)alkoxy oder (C₁-C₆)Alkoxy ist,
G Halogen, Halogen(C₁-C₆)alkyl oder Halogen(C₁-C₆)alkoxy ist, und
R² Wasserstoff, (C₁-C₆)Alkyl, Phenyl(C₁-C₆)alkyl, Cyan, (C₁-C₆)Alkoxycarbonyl, Halogen(C₁-C₆)alkoxycarbonyl, ((C₁-C₆)Alkylthio)carbonyl, Halogen(C₁-C₆)alkoxycarbonyl, Cyano(C₁-C₆)alkoxycarbonyl, (C₁-C₆)Alkoxy(C₁-C₆)alkoxycarbonyl, (C₁-C₆)Alkoxycarbonyl(C₁-C₆)alkoxycarbonyl, Phenoxycarbonyl, Phenyl(C₁-C₆)alkoxycarbonyl, (C₃-C₆)Alkenyloxycarbonyl, (C₃-C₆)Alkynyloxycarbonyl, Formyl, (C₁-C₆)alkylcarbonyl, Halogen-(C₁-C₆)alkylcarbonyl, Carboxy(C₁-C₆)alkylcarbonyl, Phenylcarbonyl, Phenyl(C₁-C₆)alkylcarbonyl, Phenyl(C₂-C₆)alkenylcarbonyl, Mono(C₁-C₆)alkylaminocarbonyl, Di(C₁-C₆)alkylaminocarbonyl, Phenylaminocarbonyl, (C₁-C₆)Alkoxycarbonyl(C₁-C₆)alkylaminocarbonyl` Phenyl((C₁-C₆)alkyl)aminocarbonyl, Di(C₁-C₆)alkylphosphoryl, Di(C₁-C₆)alkylthiophosphoryl, (C₁-C₆)Alkylsulfonyl, (C₂-C₆)Alkenylsulfonyl, Halogen(C₁-C₆)alkylsulfonyl, Phenylsulfonyl, Di(C₁-C₆)alkylaminosulfonyl, 2-Pyridyl oder 2-Pyrazinyl ist.

4. Verbindung nach Anspruch 3, wobei
Q Wasserstoff ist, G 4-Trifluormethyl ist und R² Methoxycarbonyl ist, oder
Q 4-Chlor ist, G 4-Trifluormethyl ist und R² Methoxycarbonyl ist, oder
Q 4-Chlor ist, G 4-Trifluormethoxy ist und R² Methoxycarbonyl ist, oder
Q 4-Chlor, 4-n-Butyloxy, 4-n-Propyloxy oder 4-Difluormethoxy ist, G 4-Trifluormethyl, 4-Trifluormethoxy oder 4-(1,1,2,2-Tetrafluorethoxy) ist, und R² n-Propyl ist.

5. Verbindung nach Anspruch 1, wobei V (C₁-C₆)Alkyl, (C₁-C₆)Alkoxy(C₁-C₆)alkyl, Formyl, (C₁-C₆)Alkylcarbonyl, (C₁-C₆)Alkylaminocarbonyl, (C₁-C₆)Alkoxycarbonyl, (C₃-C₆)Alkenyloxycarbonyl, Phenyloxycarbonyl, (C₁-C₆)Alkoxycarbonyl-carbonyl, Cyano(C₁-C₆)alkylthio, (C₁-C₆)Alkylthio, Phenylthio, (C₁-C₆)Alkoxycarbonyl(C₁-C₆)alkylthio oder (C₁-C₆)Alkoxycarbonylthio ist.

6. Verbindung nach Anspruch 5 mit der Formel 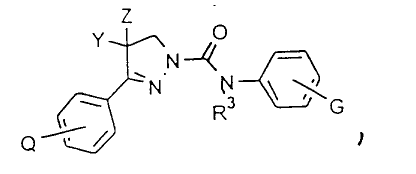 wobei Q Halogen, vorzugsweise 4-Halogen, ist, G Halogen oder Halogen(C₁-C₆)alkyl, vorzugsweise 4-Halogen oder 4-Halogen(C₁-C₆)alkyl, ist, R¹ Wasserstoff oder (C₁-C₆)Alkyl ist, R² (C₁-C₆)Alkoxycarbonyl ist, R³ (C₁-C₆)Alkyl ist, und Z Wasserstoff ist.

7. Verbindung nach Anspruch 5, wobei
Q 4-Chlor ist, G 4-Chlor ist, R¹ Methyl ist, R² Methoxycarbonyl ist, R³ Methyl ist und Z Wasserstoff ist, oder
Q 4-Chlor ist, G 4-Trifluormethyl ist, R¹ Wasserstoff ist, R² Methoxycarbonyl ist, R³ Methyl ist und Z Wasserstoff ist, oder
Q 4-Chlor ist, G 4-Trifluormethyl ist, R¹ Methyl ist, R² Methoxycarbonyl ist, R³ Methyl ist, und Z Wasserstoff ist.

8. Verbindung nach Anspruch 1, wobei X 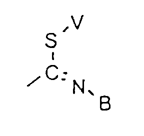 ist,
A 4-Chlorphenyl oder 4-n-Propyloxyphenyl ist, B 4-Trifluormethylphenyl ist,
Y N-Methyl-N-methoxycarbonylamino oder N-Propyl-N-formylamino ist, Z Wasserstoff ist, und V Methyl, Ethyl, n-Propyl, Isopropyl, Propenyl, Methoxyethyl oder Benzyl ist.

9. Verbindung nach Anspruch 1, wobei X 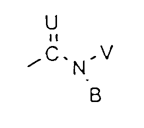 ist,
U Schwefel ist und vorzugsweise A 4-Chlorphenyl oder 4-n-Propyloxyphenyl ist, B 4-Chlorphenyl oder 4-Trifluormethylphenyl ist, Y N-Methyl-N-methoxycarbonylamino ist, V Wasserstoff ist, und Z Wasserstoff ist.

10. Verbindung nach Anspruch 2 mit der Formel 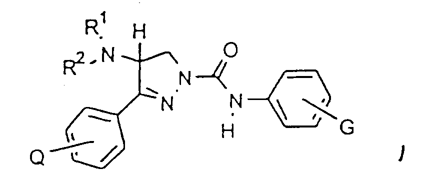 wobei
Q Wasserstoff, Halogen, (C₁-C₆)Alkyl, (C₁-C₆)Alkoxy(C₁-C₆)alkyl, Halogen(C₁-C₆)alkyloxy oder (C₁-C₆)Alkoxy ist,
G Halogen, Cyan, Halogen(C₁-C₆)alkyl, (C₁-C₆)Alkoxycarbonyl oder Halogen(C₁-C₆)alkoxy ist,
R¹ (C₁-C₆)Alkyl, (C₃-C₆)Alkenyl, (C₃-C₆)Alkynyl, (C₁-C₆)Alkoxy(C₁-C₆)alkyl, Cyan(C₁-C₆)alkyl, (C₁-C₆)Alkoxycarbonyl(C₁-C₆)alkyl, Phenyl(C₁-C₆)alkyl, Phenyl oder Halogenphenyl ist,
R² Cyan, (C₁-C₆)Alkyl, (C₁-C₆)Alkylsulfonyl, (C₁-C₆)Alkoxycarbonyl, ((C₁-C₆)alkylthio)carbonyl, Halogen(C₁-C₆)alkoxycarbonyl, Formyl, (C₁-C₆)Alkylcarbonyl, Halogen(C₁-C₆)alkylcarbonyl, (C₁-C₆)Alkoxy(C₁-C₆)alkylcarbonyl, (C₂-C₆)Alkenylcarbonyl, Halogen (C₂-C₆)alkenylcarbonyl, Di(C₁-C₆)alkylaminocarbonyl, Phenylcarbonyl, Di(C₁-C₆)alkylphosphoryloder Di(C₁-C₆)alkylthiophosphoryl ist oder
R¹ und R² zusammen mit dem Stickstoff, mit dem sie verbunden sind, einen Pyrid-2-on-1-yl-, Pyrid-4-on-1-yl-, Triazolyl-, 2-Oxazolidonyl-, Isomorpholin-2-onyl-, Pyrrolidinonyl-, Piperidonyl- or Succinimidyl-Ring bilden.

11. Verbindung nach Anspruch 10, wobei
Q 4-Chlor ist, G 4-Trifluomethyl ist, R¹ Methyl ist und R² Formyl, Methylcarbonyl, Ethylcarbonyl, Dimethylaminocarbonyl, Methoxycarbonyl, Ethoxycarbonyl oder Isopropyloxycarbonyl ist, oder
Q 4-Chlor ist, G 4-Trifluormethyl ist, R² Methoxycarbonyl ist und R¹ Ethyl, Propyl oder Propargyl ist, oder
Q 4-Chlor ist, G 4-Trifluormethoxy ist, R² Methoxycarbonyl ist und R¹ Methyl, Ethyl, Propyl, Allyl oder Propargyl ist, oder
Q 4-Chlor ist, G 4-Difluormethoxy ist, R¹ Methyl ist und R² Methoxycarbonyl ist, oder
Q is 4-Chlor ist, G 4-(1,1,2,2-Tetrafluorethoxy) ist, R² Methoxycarbonyl ist und R¹ Methyl, Ethyl oder Propargyl ist, oder
Q 4-Chlor, G 4-(1,1,2,3,3,3-Hexafluorpropyloxy), R¹ Methyl ist und R² Methoxycarbonyl ist, oder
Q 4-Ethoxy ist, G 4-Trifluormethoxy, R¹ Methyl ist und R² Methoxycarbonyl ist, oder
Q 4-n-Propyloxy ist, G 4-Trifluormethoxy ist, R¹ Methyl ist und R² Methoxycarbonyl ist, oder
Q 4-n-Butyloxy ist, G 4-Trifluormethoxy ist, R¹ Methyl ist und R² Methoxycarbonyl ist, oder
Q 4-Difluormethoxy ist, R¹ Methyl ist, R² Methoxycarbonyl ist und G 4-Chlor, 4-Trifluormethyl, 4-Difluormethoxy, 4-Trifluormethoxy, 4-(1,1,2,2-Tetrafluorethoxy), 4-(1,1,2,3,3,3-Hexafluorpropyloxy) oder 4-Isopropyloxycarbonyl ist, oder
Q Wasserstoff ist, R¹ Methyl ist, R² Methoxycarbonyl ist und G 4-Trifluormethyl oder 4-Trifluormethoxy ist.

12. Pestizide Zusammensetzung, die einen landwirtschaftlich vertäglichen Träger und eine pestizid wirksame Menge einer Verbindung nach einem der vorhergehenden Ansprüche umfaßt.

13. Zusammensetzung nach Anspruch 12, wobei die Verbindung in einer Menge von 0,0001 bis 99 Gew.-%, vorzugsweise von 0,001 bis 90 Gew.-%, mehr bevorzugt von 0,01 bis 75 Gew.-% der Zusammensetzung vorliegt.

14. Verfahren zur Schädlingsbekämpfung, vorzugsweise von Insekten der Ordnungen Lepidoptera und Coleoptera, welches das Inkontaktbringen der Insekten mit einer pestizid wirksamen Menge einer Verbindung nach einem der Ansprüche 1 bis 11 oder einer Zusammensetzung nach Anspruch 12 oder 13 Umfaßt, wobei die Verbindung selbst vorzugsweise in einer Menge von 0,1 bis 1000 g, vorzugsweise von 5 bis 200 g pro Hektar angewandt wird.

15. Verfahren zur Herstellung von wie in einem der Ansprüche 1 bis 11 definierten Verbindungen mit der Formel 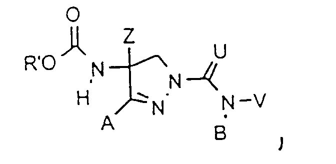
wobei R' Alkyl ist, mit den Schritten:
Verseifen einer Verbindung der Struktur 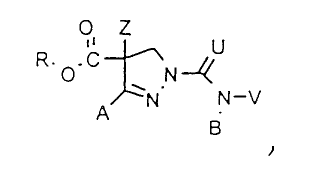
wobei R Alkyl ist,
Erhalten der korrespondierenden Carbonsäure mit der Formel 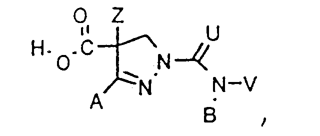
Umsetzen der Carbonsäure, um das korrespondierende Carbonsäurehalogenid zu erhalten,
Umsetzen des Carbonsäurehalogenids mit einem Azidanion, um die korrespondierende Azidocarbonylverbindung zu erhalten,
Erhitzen der Azidocarbonylverbindung, um die korrespondierende Isocyanatverbindung zu erhalten, und
Umsetzen der Isocyanatverbindung mit einem Alkohol, um die Carboalkoxyaminoverbindung zu erhalten.

16. Verfahren nach Anspruch 15, das weiter umfaßt:
Das Decarboxylieren der Verbindung der Formel 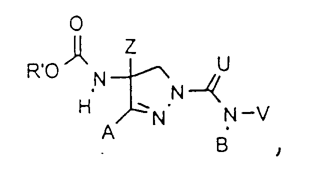 wobei R' t-Butyl ist, um eine Aminoverbindung mit der Formel 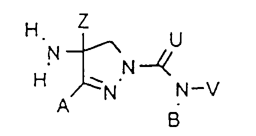
zu erhalten, und danach gegebenenfalls das Alkylieren der Aminoverbindung zur Herstellung einer Verbindung mit der Formel 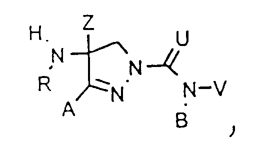
wobei R Alkyl ist, und danach gegebenenfalls das Umsetzen dieser Verbindung mit einem Acylierungsmittel, um eine Verbindung mit der Formel 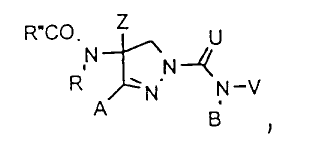
wobei R'' Wasserstoff, (C₁-C₆)Alkyl, oder (C₁-C₆)Alkoxy ist, zu erhalten.

17. Verfahren zur Herstellung von Verbindungen nach Anspruch 1 mit der Formel 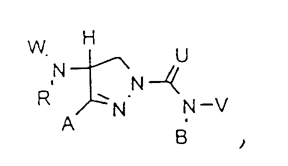 wobei W (C₁-C₆)Alkoxycarbonyl ist, wobei das Verfahren umfaßt:
Das Halogenieren eines Methylketons der Formel 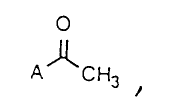 um ein Halogenmethylketon zu erhalten,
das Umsetzen des Halogenmethylketons mit einem Mono- oder Dialkylamin, um das korrespondierende mono- oder di-alkylierte Keton mit der Formel 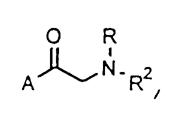 wobei R (C₁-C₆)Alkyl ist und R² Wasserstoff oder Methyl ist, zu erhalten,
das Acylieren des Alkylaminomethylketons, um eine acylierte Verbindung mit der Formel 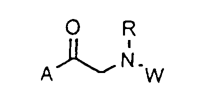 zu erhalten,
das Umsetzen der acylierten Verbindung mit Formaldehyd, um das korrespondierende Prop-2-enon mit der Formel 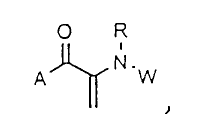 wobei W (C₁-C₆)Alkoxycarbonyl ist, zu erhalten,
das Umsetzen des Prop-2-enons mit Hydrazin, um das korrespondierende Dihydropyrazol mit der Formel

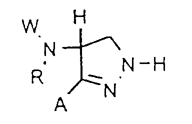

## Revendications

1. Composé de formule 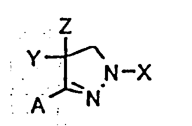 dans laquelle
X est 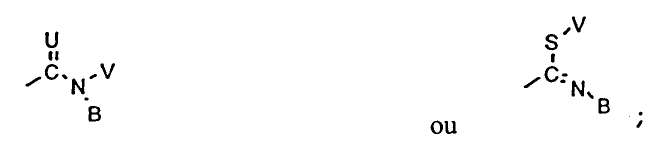
A et B sont des groupes pyridyle, furyle, thiazolyle ou naphtyle, chacun étant éventuellement et indépendamment substitué par un ou deux groupes choisis parmi les groupes nitro, alkyle en C₁-C₆, halogénoalkyle en C₁-C₆ ou halogéno; ou
un groupe phényle éventuellement substitué par un à trois groupes choisis parmi les groupes allq'le en C₁-C₆; halogénoalkyle en C₁-C₆; halogéno; alcoxy en C₁-C₆; halogénoalcoxy en C₁-C₆; alcényloxy en C₃-C₆; alcynyloxy en C₃-C₆; alcoxy(en C₁-C₆)alcoxy(en C₁-C₆); phényl(alcoxy en C₁-C₆); phényloxy; pyridyloxy; monoalkyl(en C₁-C₆)aminocarbonyloxy; dialkyl(en C₁-C₆)aminocarbonyloxy; alcanoyloxy en C₁-C₆; alcoxy(en C₁-C₆)carbonyloxy; alkyl(en C₁-C₆)sulfonyloxy; alkylthio en C₁-C₆; halogénoalkylthio en C₁-C₆; alcoxy(en C₁-C₆)(alkyle en C₁-C₆); alcanoyle en C₁-C₆; alcoxy(en C₁-C₆)carbonyle; nitro; alkylsulfonyle en C₁-C₆; halogénoalkylsulfonyle en C₁-C₆; phényle; hydroxy; cyano; isocyano; amino; monoalkyl(en C₁-C₆)amino; dialkyl(en C₁-C₆)amino; formylamino; alcanoylamino en C₁-C₆; halogénoalcanoylamino en C₁-C₆; phénylcarbonylamino; monoalkyl(en C₁-C₆)aminocarbonylamino; ou dialkyl(en C₁-C₆)aminocarbonylamino;
U est un atome d'oxygène ou de soufre,
V est un atome d'hydrogène ou un groupe alkyle en C₁-C₆, alcoxy(en C₁-C₆)(alkyle en C₁-C₆), formyle, alkyl(en C₁-C₆)carbonyle, alkyl(en C₁-C₆)aminocarbonyle, alcoxy(en C₁-C₆)carbonyle, alcényl(en C₃-C₆)-alcényloxycarbonyle, phényloxycarbonyle, alcoxy(en C₁-C₆)carbonylcarbonyle, cyano(alkylthio en C₁-C₆), alkylthio en C₁-C₆, phénylthio, alcoxy(en C₁-C₆)carbonyl(alkylthio en C₁-C₆) ou alcoxy(en C₁-C₆)carbonylthio; à condition que V ne soit pas un atome d'hydrogène s'il est fixé à S;
Y est -NR¹R²;
où
R¹ et R² sont chacun indépendamment un atome d'hydrogène ou un groupe cyano, alkyle en C₁-C₆, halogénoalkyle en C₁-C₆, cyano(alkyle en C₁-C₆), phényl(alkyle en C₁-C₆), alcényle en C₃-C₆, halogénoalcényle en C₃-C₆, alcynyle en C₃-C₆, phényle, halogénophényle, formyle, alkyl(en C₁-C₆)carbonyle, halogénoalkyl(en C₁-C₆)carbonyle, alcényl(en C₂-C₆)-carbonyle, halogénoalcényl(en C₂-C₆)carbonyle, alcoxy(en C₁-C₆)alkyl(en C₁-C₆)carbonyle, phénylcarbonyle, phénylalcényl(en C₂-C₆)carbonyle, carboxy, alcoxy(en C₁-C₆)carbonyle, halogénoalcoxy(en C₁-C₆)carbonyle, cyano(alcoxy en C₁-C₆)carbonyle, alcényl(en C₂-C₆)oxycarbonyle, alcynyl(en C₃-C₆)oxycarbonyle, alcanoyl(en C₁-C₆)alcoxy(en C₁-C₆)carbonyle, alcoxy(en C₁-C₆)carbonylalcoxy(en C₁-C₆)carbonyle, carboxyalcoxy(en C₁-C₆)carbonyle, phényloxycarbonyle, phénylalcoxy(en C₁-C₆)carbonyle, alkylthio(en C₁-C₆)carbonyle, N-alkyl(en C₁-C₆)aminocarbonyle, N,N-dialkyl(en C₁-C₆)aminocarbonyle, N-phényl-N-alkyl(en C₁-C₆)aminocarbonyle, N-(phénylcarbonyl)aminocarbonyle, dialkyl(en C₁-C₆)-phosphoryle, alkylsulfonyle en C₁-C₆, alcénylsulfonyle en C₂-C₆, N,N-dialkyl(en C₁-C₆)aminosulfonyle, phénylsulfonyle, pyridyle ou pyrazinyle; ou
R¹ et R², conjointement avec l'atome d'azote auquel ils sont fixés, forment un noyau pyrid-2-one-1-yle, pyrid-4-one-1-yle, triazolyle, 2-oxazolidonyle, isomorpholin-2-yle, pyrrolidinonyle, pipéridonyle ou succinimidyle; et
Z est un atome d'hydrogène; et
ses sels acceptables en agronomie.

2. Composé selon la revendication 1, dans lequel
X est 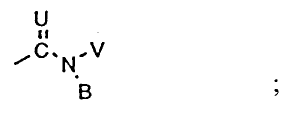 et
V est un atome d'hydrogène.

3. Composé selon la revendication 2, de formule 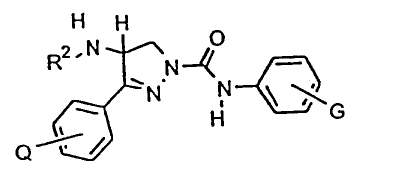 dans laquelle
Q est un atome d'hydrogène ou un groupe halogéno, halogénoalcoxy en C₁-C₆ ou alcoxy en C₁-C₆;
G est un groupe halogéno, halogénoalkyle en C₁-C₆ ou halogénoalcoxy en C₁-C₆; et
R² est un atome d'hydrogène ou un groupe alkyle en C₁-C₆, phényl(alkyle en C₁-C₆), cyano, alcoxy(en C₁-C₆)carbonyle, halogénoalcoxy(en C₁-C₆)carbonyle, alkylthio(en C₁-C₆)carbonyle, halogénoalcoxy(en C₁-C₆)carbonyle, cyanoalcoxy(en C₁-C₆)carbonyle, alcoxy(en C₁-C₆)-alcoxy(en C₁-C₆)carbonyle, alcoxy(en C₁-C₆)carbonylalcoxy(en C₁-C₆)-carbonyle, phénoxycarbonyle, phényl(alcoxy en C₁-C₆)carbonyle, alcényl(en C₃-C₆)oxycarbonyle, alcynyl(en C₃-C₆)oxycarbonyle, formyle, alkyl(en C₁-C₆)carbonyle, halogénoalkyl(en C₁-C₆)carbonyle, carboxyalkyl(en C₁-C₆)carbonyle, phénylcarbonyle, phénylalkyl(en C₁-C₆)carbonyle, phénylalcényl(en C₂-C₆)carbonyle, monoalkyl(en C₁-C₆)aminocarbonyle, dialkyl(en C₁-C₆)aminocarbonyle, phénylaminocarbonyle, alcoxy(en C₁-C₆)-carbonylalkyl(en C₁-C₆)aminocarbonyle, phénylalkyl(en C₁-C₆)aminocarbonyle, dialkyl(en C₁-C₆)phosphoryle, dialkyl(en C₁-C₆)thiophosphoryle, alkylsulfonyle en C₁-C₆, alcénylsulfonyle en C₂-C₆, halogénoalkylsulfonyle en C₁-C₆, phénylsulfonyle, dialkylaminosulfonyle en C₁-C₆, 2-pyridyle ou 2-pyrazinyle.

4. Composé selon la revendication 3, dans lequel
Q est un atome d'hydrogène, G est un groupe 4-trifluorométhyle et R² est un groupe méthoxycarbonyle; ou
Q est un groupe 4-chloro, G est un groupe 4-trifluorométhyle et R² est un groupe méthoxycarbonyle, ou
Q est un groupe 4-chloro, G est un groupe 4-trifluorométhoxy et R² est un groupe méthoxycarbonyle; ou
Q est un groupe 4-chloro, 4-n-butyloxy, 4-n-propyloxy ou 4-difluorométhoxy, G est un groupe 4-trifluorométhyle, 4-trifluorométhoxy ou 4-(1,1,2,2-tétrafluoroéthoxy) et R² est un groupe n-propyle.

5. Composé se!on la revendication 1, dans lequel
V est un groupe alkyle en C₁-C₆, alcoxy(en C₁-C₆)alkyle(en C₁-C₆), formyle, alkyl(en C₁-C₆)carbonyle, alkyl(en C₁-C₆)aminocarbonyle, alcoxy(en C₁-C₆)carbonyle, alcényl(en C₃-C₆)oxycarbonyle, phényloxycarbonyle, alcoxy(en C₁-C₆)carbonyl-carbonyle, cyano(alkylthio en C₁-C₆), alkylthio en C₁-C₆, phénylthio; alcoxy(C₁-C₆)carbonyl(alkylthio en C₁-C₆) ou alcoxy(en C₁-C₆)carbonylthio.

6. Composé selon la revendication 5, de formule 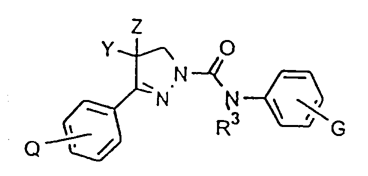 dans laquelle
Q est un groupe halogéno; de préférence 4-halogéno;
G est un groupe halogéno ou halogénoalkyle en C₁-C₆, de préférence
4-halogéno ou 4-halogénoalkyle en C₁-C₆;
R¹ est un atome d'hydrogène ou un groupe alkyle en C₁-C₆;
R² est un groupe alcoxy(en C₁-C₆)carbonyle;
R³ est un groupe alkyle en C₁-C₆; et
Z est un atome d'hydrogène.

7. Composé selon la revendication 5, dans lequel
Q est un groupe 4-chloro, G est un groupe 4-chloro, R¹ est un groupe méthyle, R² est un groupe méthoxycarbonyle, R³ est un groupe méthyle et Z est un atome d'hydrogène; ou
Q est un groupe 4-chloro, G est un groupe 4-trifluorométhyle, R¹ est un atome d'hydrogène, R² est un groupe méthoxycarbonyle, R³ est un groupe méthyle et Z est un atome d'hydrogène; ou
Q est un groupe 4-chloro, G est un groupe 4-trifluorométhyle, R¹ est un groupe méthyle, R² est un groupe méthoxycarbonyle, R³ est un groupe méthyle et Z est un atome d'hydrogène.

8. Composé selon la revendication 1, dans lequel X est 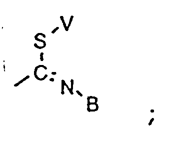
A est un groupe 4-chlorophényle ou 4-n-propyloxyphényle;
B est un groupe 4-trifluorométhylphényle;
Y est un groupe N-méthyl-N-méthoxycarbonylamino ou N-propyl-N-formylamino;
Z est un atome d'hydrogène; et
V est un groupe méthyle, éthyle, n-propyle, isopropyle, propényle, méthoxyéthyle ou benzyle.

9. Composé selon la revendication 1, dans lequel X est 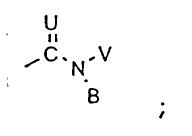
U est un atome de soufre, et de préférence A est un groupe 4-chlorophényle ou 4-n-propyloxyphényle, B est un groupe 4-chlorophényle ou 4-trifluorométhylphényle, Y est un groupe N-méthyl-N-méthoxycarbonylamino, V est un atome d'hydrogène et Z est un atome d'hydrogène.

10. Composé selon la revendication 2, de formule 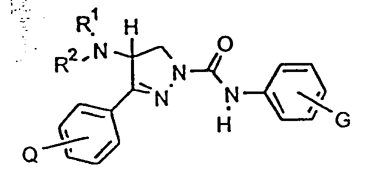 dans laquelle
Q est un atome d'hydrogène ou un groupe halogéno, alkyle en C₁-C₆, alcoxy(en C₁-C₆)alkyle(en C₁-C₆), halogénoalkyloxy en C₁-C₆ ou alcoxy en C₁-C₆;
G est un groupe halogéno, cyano, halogénoalkyle en C₁-C₆, alcoxy(en C₁-C₆)carbonyle ou halogénoalcoxy en C₁-C₆;
R¹ est un groupe alkyle en C₁-C₆, alcényle en C₃-C₆, alcynyle en C₃-C₆, alcoxy(en C₁-C₆)alkyle(en C₁-C₆), cyano(alkyle en C₁-C₆), alcoxy(en C₁-C₆)carbonyl(alkyle en C₁-C₆), phényl(alkyle en C₁-C₆), phényle ou halogénophényle;
R² est un groupe cyano, alkyle en C₁-C₆, alkylsulfonyle en C₁-C₆, alcoxy(en C₁-C₆)carbonyle, alkylthio(en C₁-C₆)carbonyle, halogénoalcoxy(en C₁-C₆)carbonyle, formyle, alkyl(en C₁-C₆)carbonyle, halogénoalkyl(en C₁-C₆)carbonyle, alcoxy(en C₁-C₆)alkyl(en C₁-C₆)carbonyle, alcényl(en C₂-C₆)carbonyle, halogénoalcényl(en C₂-C₆)carbonyle, dialkyl(en C₁-C₆)-aminocarbonyle, phénylcarbonyle, dialkyl(en C₁-C₆)phosphoryle ou dialkyl(en C₁-C₆)thiophosphoryle; ou
R¹ et R², conjointement avec l'atome d'azote auquel ils sont fixés, forment un noyau pyrid-2-one-1-yle, pyrid-4-one-1-yle, triazolyle, 2-oxazolidonyle, isomorpholin-2-onyle, pyrrolidinonyle, pipéridonyle ou succinimidyle.

11. Composé selon la revendication 10, dans lequel
Q est un groupe 4-chloro, G est un groupe 4-trifluorométhyle, R¹ est un groupe méthyle et R² est un groupe formyle, méthylcarbonyle, éthylcarbonyle, diméthylaminocarbonyle, méthoxycarbonyle, éthoxycarbonyle ou isopropyloxycarbonyle; ou
Q est un groupe 4-chloro, G est un groupe 4-trifluorométhyle, R² est un groupe méthoxycarbonyle et R¹ est un groupe éthyle, propyle ou propargyle; ou
Q est un groupe 4-chloro, G est un groupe 4-trifluorométhoxy, R² est un groupe méthoxycarbonyle et R¹ est un groupe méthyle, éthyle, propyle, allyle ou propargyle; ou
Q est un groupe 4-chloro, G est un groupe 4-difluorométhoxy, R¹ est un groupe méthyle et R² est un groupe méthoxycarbonyle; ou
Q est un groupe 4-chloro, G est un groupe 4-(1,1,2,2-tétrafluoroéthoxy), R² est un groupe méthoxycarbonyle et R¹ est un groupe méthyle, éthyle ou propargyle; ou
Q est un groupe 4-chloro, G est un groupe 4-(1,1,2,3,3,3-hexafluoropropyloxy), R¹ est un groupe méthyle et R² est un groupe méthoxycarbonyle; ou
Q est un groupe 4-éthoxy, G est un groupe 4-trifluorométhoxy, R¹ est un groupe méthyle et R² est un groupe méthoxycarbonyle; ou
Q est un groupe 4-n-propyloxy, G est un groupe 4-trifluorométhoxy, R¹ est un groupe méthyle et R² est un groupe méthoxycarbonyle, ou
Q est un groupe 4-n-butyloxy, G est un groupe 4-trifluorométhoxy, R¹ est un groupe méthyle et R² est un groupe méthoxycarbonyle; ou
Q est un groupe 4-difluorométhoxy, R¹ est un groupe méthyle, R² est un groupe méthoxycarbonyle et G est un groupe 4-chloro, 4-trifluorométhyle, 4-difluorométhoxy, 4-trifluorométhoxy, 4-(1,1,2,2-tétrafluoroéthoxy), 4-(1,1,2,3,3,3-hexafluoropropyloxy), ou 4-isopropyloxycarbonyle; ou
Q est un atome d'hydrogène, R¹ est un groupe méthyle, R² est un groupe méthoxycarbonyle et G est un groupe 4-trifluorométhyle ou 4-trifluorométhoxy.

12. Composition pesticide, qui comprend un véhicule acceptable en agriculture et une quantité efficace en tant que pesticide d'un composé selon l'une quelconque des revendications précédentes.

13. Composition selon la revendication 12, dans laquelle le composé est présent en quantité de 0,0001 à 99 pour cent, de préférence de 0,001 à 90 pour cent, mieux encore de 0,01 à 75, pour cent en poids de la composition.

14. Procédé pour lutter contre les nuisibles, de préférence les insectes des ordres des lépidoptères et des coléoptères, qui comprend la mise en contact des insectes avec une quantité efficace comme pesticide d'un composé selon l'une quelconque des revendications 1 à 11, ou d'une composition selon la revendication 12 ou 13, le composé proprement dit étant de préférence appliqué à un taux de 0,1 à 1 000 g, de préférence de 5 à 200 g, par hectare.

15. Procédé pour préparer les composés tels que définis dans l'une quelconque des revendications 1 à 11, de formule 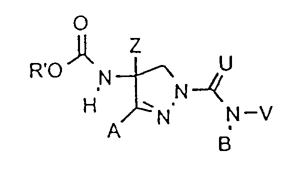
dans laquelle R' est un groupe alkyle, par les étapes consistant à :
saponifier un composé de structure 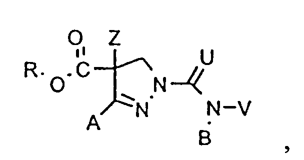 dans laquelle R est un groupe alkyle, obtenir l'acide carboxylique correspondant de formule 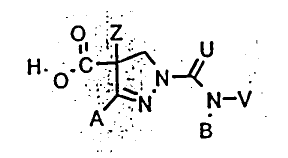
faire réagir l'acide carboxylique pour obtenir l'halogénure d'acide carboxylique correspondant;
faire réagir l'halogénure d'acide carboxylique avec un anion azoture pour obtenir le composé azidocarbonyle correspondant;
chauffer le composé azidocarbonyle pour obtenir le composé isocyanato correspondant; et
faire réagir le composé isocyanato avec un alcool pour obtenir ledit composé carboalcoxyamino.

16. Procédé selon la revendication 15, comprenant en outre la décarboxylation du composé de formule 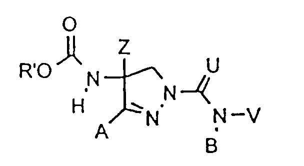 dans laquelle R' est un groupe t-butyle, pour obtenir un composé amino de formule 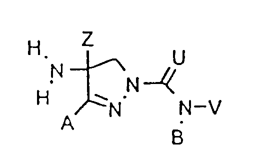 puis éventuellement l'alkylation dudit composé amino pour former un composé de formule 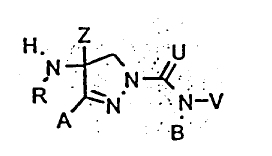 dans laquelle R est un groupe alkyle,
puis éventuellement la réaction de ce composé avec un agent d'acylation pour donner un composé de formule 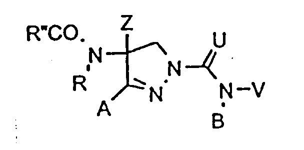 dans laquelle R'' est un atome d'hydrogène ou un groupe alkyle en C₁-C₆ ou alcoxy en C₁-C₆.

17. Procédé de préparation de composés selon la revendication 1, de formule 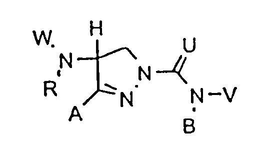 dans laquelle W est un groupe alcoxy(en C₁-C₆)carbonyle, ce procédé comprenant les étapes consistant à
halogéner une méthylcétone de formule 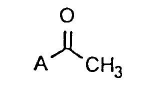 pour obtenir une halogénométhylcétone;
faire réagir l'halogénométhylcétone avec une mono- ou di-alkylamine pour obtenir la cétone mono- ou di-alkylée correspondante, de formule 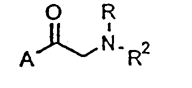 dans laquelle R est un groupe alkyle en C₁-C₆et R² est un atome d'hydrogène ou un groupe méthyle;
acyler l'alkylaminométhylcétone pour obtenir un composé acylé de formule 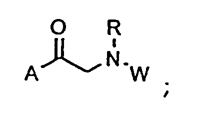
faire réagir le composé acylé avec du formaldéhyde pour obtenir la prop-2-énone correspondante de formule 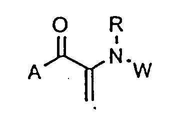 dans laquelle W est un groupe alcoxy(en C₁-C₆)carbonyle;
faire réagir la prop-2-énone avec de l'hydrazine pour obtenir le dihydropyrazole correspondant de formule 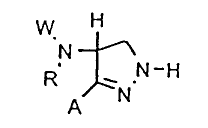
et faire réagir le dihydropyrazole avec un isocyanate pour obtenir un composé de formule
